(19) Europäisches Patentamt
European Patent Office
Office européen des brevets

(11) EP 0 723 398 B1

(12) EUROPEAN PATENT SPECIFICATION

(45) Date of publication and mention of the grant of the patent:
**23.03.2005 Bulletin 2005/12**

(21) Application number: **94930729.2**

(22) Date of filing: **13.10.1994**

(51) Int Cl.[7]: **A01N 43/36**, A61K 31/40, A61K 38/08, A61K 38/16, A61K 38/41, C07D 487/22, C12N 9/02

(86) International application number:
**PCT/US1994/011558**

(87) International publication number:
**WO 1995/010185 (20.04.1995 Gazette 1995/17)**

(54) **SUPEROXIDE DISMUTASE MIMETICS**
SUPEROXIDDISMUTASE MEMETIKA
SUPEROXYDE-DISMUTASE MIMETIQUES

(84) Designated Contracting States:
**AT BE CH DE DK ES FR GB GR IE IT LI LU MC NL PT SE**

(30) Priority: **15.10.1993 US 136207**

(43) Date of publication of application:
**31.07.1996 Bulletin 1996/31**

(60) Divisional application:
**04010434.1 / 1 442 747**

(73) Proprietors:
- **DUKE UNIVERSITY**
  **Durham, North Carolina 27708-0083 (US)**
- **UNIVERSITY OF ALABAMA, BIRMINGHAM RESEARCH FOUNDATION**
  **Birmingham, AL 35294-0111 (US)**

(72) Inventors:
- **CRAPO, James, D.**
  **Durham, NC 27705 (US)**
- **FRIDOVICH, Irwin**
  **Durham, NC 27707 (US)**
- **OURY, Tim**
  **Durham, NC 27704 (US)**
- **DAY, Brian J., 3611 University Drive**
  **Durham, NC 27707 (US)**
- **FOLZ, Rodney, J.**
  **Durham, NC 27706 (US)**
- **FREEMAN, Bruce, A.**
  **Birmingham, AL 35223 (US)**

(74) Representative: **Crisp, David Norman et al**
**D Young & Co**
**120 Holborn**
**London EC1N 2DY (GB)**

(56) References cited:
**EP-A- 0 414 915**
**EP-A- 0 524 161**
**WO-A-91/04315**
**WO-A-92/07935**
**US-A- 5 227 405**

- **INOUE M. ET AL: "Expression of hybrid Cu/Zn-type superoxide dismutase which has high affinity for heparin-like proteoglycans on vascular endothelial cells" J. BIOL. CHEM, vol. 266, no. 25, 5 September 1991, pages 16409-16414, XP002074664**
- **DAY BJ ET AL: "Manganic porphyrins possess catalase activity and protect endothelial cells against hydrogen peroxide-mediated injury." ARCH BIOCHEM BIOPHYS, NOV 15 1997, 347 (2) P256-62, XP002074503 UNITED STATES**
- **TSAN M.-F.: "Superoxide dismutase and pulmonary oxygen toxicity" PROC. SOC. EXP. BIOL. MED., 1993, 203/3 (286-290), XP002074505 USA**
- **LEE J ET AL: "Rapid decomposition of peroxynitrite by manganese porphyrin -antioxidant redox couples" BIOORGANIC & MEDICINAL CHEMISTRY LETTERS, vol. 7, no. 22, 18 November 1997, page 2913-2918 XP004136556**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 723 398 B1

- JOURNAL OF INORGANIC BIOCHEMISTRY, Vol. 15, issued 1981, PASTERNACK et al., "Catalysis of the Disproportionation of Superoxide by Metalloporphyrins. III", pages 261-267.
- AGENTS AND ACTIONS, Vol. 15, No. 5/6, issued 1984, OBERLY et al., "Anticancer Activity of Metal Compounds with Superoxide Dismutase Activity", pages 535-538.
- PHARMACOLOGY AND THERAPEUTICS, Vol. 39, issued 1988, KUMAR et al., "Radioprotection by Antioxidant Enzymes and Enzyme Mimetics", pages 301-309.
- JOURNAL OF BIOLOGICAL CHEMISTRY, Vol. 268, No. 31, issued 05 November 1993, WEISS et al., "Evaluation of Activity of Putative Superoxide Dismutase Mimics", pages 23049-23054.
- PROCEEDINGS OF THE NATIONAL ACADEMY OF SCIENCES USA, Vol. 89, issued July 1992, PARGE et al., "Atomic Structures of Wild-Type and Thermostable Mutant Recombinant Human Cu,Zn Superoxide Dismutase", pages 6109-6113.
- INORGANIC REACTION MECHANISMS, Vol. 7, issued 1981, LAPPIN, "Bioinorganic Studies", pages 334-343.

## Description

### TECHNICAL FIELD

The present invention relates, in general, to a method of modulating physiological and pathological processes and, in particular, to a method of modulating intra- and extracellular levels of superoxide radicals and thereby processes in which such radicals are a participant.

### BACKGROUND

Oxygen free radicals are produced as part of the normal metabolism of all cells but also are an important component of the pathogenesis of many disease processes. For example, oxygen free radicals are critical elements of the pathogenesis of diseases of the lung, the central nervous system and skeletal muscle. Oxygen free radicals also play a role in modulating the effects of nitric oxide (NO·). In this context, they contribute to the pathogenesis of vascular disorders, inflammatory diseases and the aging process.

A critical balance of defensive enzymes against oxygen radicals is required to maintain normal cell and organ function. Superoxide dismutases (SODs), a family of metalloenzymes which catalyze the intra- and extracellular conversion of $O_2^-$ into $H_2O_2$ plus $O_2$, and represent the first line of defense against the detrimental effects of superoxide radicals. Mammals produce three distinct SODs. One is a dimeric copper- and zinc-containing enzyme (CuZn SOD) found in the cytosol of all cells. A second is a tetrameric manganese-containing SOD (Mn SOD) found within mitochondria, and the third is a tetrameric, glycosylated, copper- and zinc-containing enzyme (EC-SOD) found in the extracellular fluids and bound to the extracellular matrix. Several other important antioxidant enzymes are known to exist within cells, including catalase and glutathione peroxidase. While extracellular fluids and the extracellular matrix contain only small amounts of these enzymes, other extracellular antioxidants are known to exist, including radical scavengers and inhibitors of lipid peroxidation, such as ascorbic acid, uric acid, and $\alpha$-tocopherol (Halliwell et al, Arch. Biochem. Biophys. 280:1 (1990)). The relative lack of extracellular antioxidant enzymes may reflect the possible function of extracellular reactive oxygen species as bioeffector molecules (Halliwell et al, Arch. Biochem. Biophys. 280:1 (1990)). The relative deficiency of such enzymes may also result in greater susceptibility to extracellular oxidant stresses.

The enzyme EC-SOD, in many extracellular locations, exists only at low concentrations. While its physiologic role *in vivo* is yet to be defined, in many extracellular locations, EC-SOD is not thought to function as a bulk scavenger of $O_2^-$. As indicated above, EC-SOD is a tetrameric Cu/Zn-containing glycoprotein with a subunit molecular weight of 30,000 (Marklund, Proc. Natl. Acad. Sci. USA 79:7634 (1982); Tibell et al, Proc. Natl. Acad. Sci. USA 84:6634 (1987); see also USP 5,130,245 and WO 91/04315). Biochemical data suggest that EC-SOD binds to heparan sulfate proteoglycans on endothelial cells, where it has been speculated to serve as a "protective coat" (Marklund, J. Clin. Invest. 74:1398 (1984); Karlsson et al, Biochem. J. 255:223 (1988)). Endothelial cells secrete both $O_2^-$ (Halliwell, Free Radical Res. Commun. 5:315 (1989)) and endothelium-derived relaxing factor, putatively identified as nitric oxide (NO·) (Noak and Murphy, *in* Oxidative Stress Oxidants and Antioxidants, eds Sies, H. (Academic, San Diego), pp. 445-489 (1991)). NO· functions as a vasoregulator and as a regulator of neurotransmission (Schuman and Madison, Science 254:1503 (1991)). NO· can, however, be toxic to neurons in some situations (Dawson et al, Proc. Natl. Acad. Sci. USA 88:6368 (1991)). $O_2^-$ is known to inactivate NO·-induced vasorelaxation (Gryglewski et al, Nature 320:454 (1986); Rubanyi and Vanhoutte, Am. J. Physiol. 250:H822 (1986); Rubanyi and Vanhoutte, Am. J. Physiol. 250:H815 (1986); Bull et al, Br. J. Pharmacol. 95:1308 (1988); Nucci et al, Proc. Natl. Acad. Sci. USA 85:2334 (1988)). Thus, a possible function for EC-SOD is to protect NO· released from cells from $O_2^-$-mediated inactivation.

The reaction of $O_2^-$ with NO· is also known to produce a potentially toxic intermediate in the form of the peroxynitrite anion ($ONOO^-$) (Beckman et al, Proc. Natl. Acad. Sci. USA 87:1620 (1990); Mulligan et al, Proc. Natl. Acad. Sci. USA 88:6338 (1991); Hogg et al, Biochem. J. 281:419 (1992); Matheis et al, Am. J. Physiol. 262:H616 (1992)). Thus EC-SOD may also function to prevent the formation of $ONOO^-$.

Surprisingly, it has been found that EC-SOD increases, rather than decreases, central nervous system $O_2$ toxicity and that this effect of EC-SOD occurs through modulation of NO·. This result implicates NO· as an important mediator in $O_2$ toxicity. The invention thus relates to methods of manipulating nitric oxide function that involve the use of extracellular antioxidants. These methods find application in various disease and non-disease states in which oxidative stress plays a role, including inflammation. In a broader sense, the invention relates generally to methods of modulating intra- and extracellular processes in which $O_2^-$ is a participant.

### SUMMARY OF THE INVENTION

The present invention relates to a method of modulating intra- or extracellular levels of superoxide radicals. More particularly, the invention relates to a method of modulating normal or pathological processes involving superoxide

radicals using, for example, low molecular weight mimetics of SOD.

In one aspect, the invention provides use of a mimetic of superoxide dismutase of formula

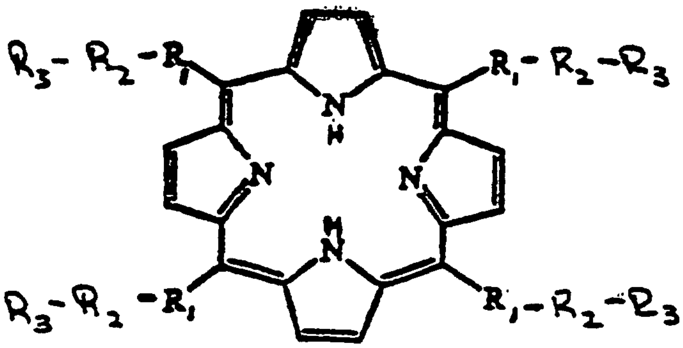

or a pharmaceutically acceptable salt thereof, wherein:

$R_1$ is a bond,

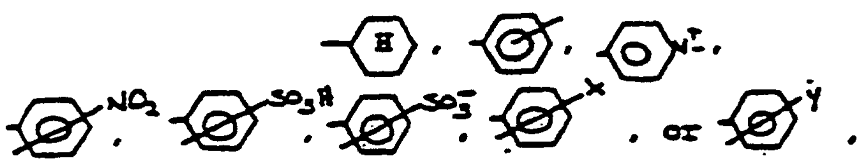

wherein X is a halogen and Y is an alkyl group and wherein

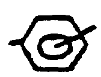

indicates bonding to $R_2$ at any position and

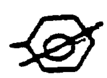

indicates bonding to $R_2$ and the substituent at any position; and

$R_2$ is a bond, $-(CY'_2)_n^-$, $-(CY'_2-CY'=CY')_n^-$, $-(CY'_2-CY'_2-CH=CH)_n^-$, $-(CY'=CY')_n^-$, or

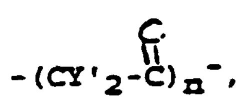

wherein Y' is hydrogen or an alkyl group and wherein n is 1 to 8; and

$R_3$ is -Y", -OH, $-NH_2$, $-N^+(Y")_3$, -COOH, -COO-, $-SO_3H$, $-SO_3-$, $-CH_2-PO_3H_2$ or $-CH_2-PO_3H-$, wherein Y" is an alkyl group,

complexed with manganese;

in the preparation of a medicament for protecting cells from superoxide radical-induced toxicity in any one or more of the following applications

1. the protection against ischemic reperfusion injuries associated with myocardial infarction, stroke, acute head trauma, organ reperfusion following transplantation, bowel ischemia, pulmonary infarction, surgical occlusion of

blood flow, or soft tissue injury;

2. the protection against skeletal reperfusion injuries;
3. the protection against damage to skin and eyes from sunlight;
4. the protection against damage to eyes from glaucoma or macular degeneration;
5. the protection against diseases of the bone;
6. the protection against connective tissue disorders associated with defects in collagen synthesis or degradation;
7. the treatment of diseases of the central nervous system;
8. the treatment of diseases of the musculature;
9. the treatment of neurologic disorders;
10. the treatment of arthritis, systemic hypertension, atherosclerosis, edema, septic shock, pulmonary hypertension, impotence, infertility, endometriosis, premature uterine contractions, memory disorders, microbial infections, and/or gout;
11. the treatment of inflammations; and
12. the treatment of chronic sinusitis, and/or autoimmune diseases.

In another aspect, the invention provides a method of protecting plant cells from superoxide radical-induced toxicity comprising contacting said cells with a non-toxic amount, sufficient to effect said protection, of a mimetic of superoxide dismutase of formula

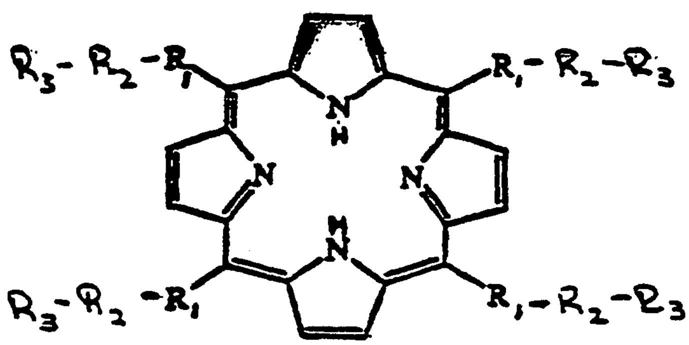

or a pharmaceutically acceptable salt thereof, wherein:

$R_1$ is a bond,

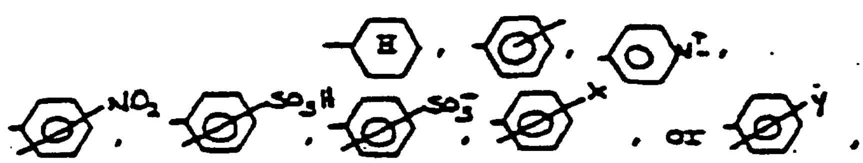

wherein X is a halogen and Y is an alkyl group and wherein

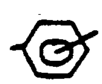

indicates bonding to $R_2$ at any position and

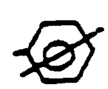

indicates bonding to $R_2$ and the substituent at any position; and

$R_2$ is a bond, $-(CY'_2)_n^-$, $-(CY'_2-CY'=CY')_n^-$, $-(CY'_2-CY'_2-CH=CH)_n^-$, $-(CY'=CY')_n^-$, or

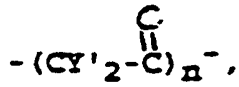

wherein Y' is hydrogen or an alkyl group and wherein n is 1 to 8; and

$R_3$ is -Y", -OH, $-NH_2$, $-N^+(Y")_3$, -COOH, -COO-, $-SO_3H$, $-SO_3$-, $-CH_2-PO_3H_2$ or $-CH_2-PO_3H$-, wherein Y" is an alkyl group,

complexed with manganese.

Objects and advantages of the present invention will be clear from the description that follows.

BRIEF DESCRIPTION OF THE DRAWINGS

Figure 1 shows the EC-SOD expression vector used to construct transgenic mice. Transgenic mice were

Figure 2 shows the Northern analysis of tissues from transgenic mice. Twenty µg of total RNA from the tissues of transgenic mice were denatured with gloxal and electrophoresed through a 1.2% agarose gel and blotted onto nitrocellulose. The filter was probed with the entire human EC-SOD cDNA. The 2.5 Kb band corresponds to mRNA of the human EC-SOD transgene containing the 1 Kb intervening sequence (see Figure 1). The 1.5 Kb band corresponds to the fully processed mRNA of the human EC-SOD transgene.

Figure 3 shows the percent survival of transgenic and nontransgenic mice exposed to 6 ATA oxygen for 25 minutes. Mice were injected with saline or given 20 mg/kg N-ω-nitro-L-arginine (LNNA) i.p. 10 minutes before compression. 400 mg/kg of diethyldithiocarbamate (DDC) in saline was injected i.p. 55 min before compression. *p<0.017 tested by $\chi^2$ with Bonferroni correction, compared to transgenic saline treated mice.

Figure 4 shows time to onset of first seizure in transgenic and nontransgenic mice exposed to 6 ATA oxygen. Mice were injected with saline or given 20 mg/kg N-ω-nitro-L-arginine (LNNA) i.p. 10 minutes before beginning compression. 400 mg/kg diethyldithiocarbamate (DDC) was injected i.p. 55 minutes prior to compression. Results are expressed as mean ± S.D. of time to first seizure with zero time taken once chamber reached 6 ATA. *p<0.05 tested by analysis of variance with the Scheffe F-test compared to nontransgenic saline treated mice.

taken once chamber reached 6 ATA. *p<0.05 tested by analysis of variance with the Scheffe F-test compared to nontransgenic saline treated mice.

Figure 5 shows the effect of diethyldithiocarbamate and β-mercaptoethanol on survival in 6 ATA oxygen for 30 minutes. (C57BL/6 X C3H)F1 mice were injected i.p. with saline, 180 mg/kg β-mercaptoethanol (2-ME), or 400 mg/kg diethyldithiocarbamate (DDC) in saline 55 min. before compression. *p<0.025 tested by $\chi^2$ with Bonferroni correction compared to saline treated mice.

Figure 6 shows the seizure latency in wild-type mice exposed to 6 ATA oxygen after being treated with saline or 20 mg/kg N-ω-nitro-L-arginine (LNNA) or 20 mg/kg N-ω-nitro-L-arginine plus 50 mg/kg L-arginine (LNNA + L-Arg). *p<0.05 tested by analysis of variance with a paired Student's t-test compared to saline treated mice.

Figure 7 shows the percent survival in wild-type mice exposed to 6 ATA oxygen. Mice were given an i.p. injection of normal saline (0.008 cc/g) or 20 mg/kg N-ω-nitro-L-arginine (LNNA) (0.008 cc/g) 15 minutes prior to compression. The mice were exposed to 6 ATA of oxygen for 20 minutes (n=10, saline only), 25 minutes (n=10, both groups), 30 minutes (n=10, saline only), 50 minutes (n=6, LNNA only), 75 minutes (n=12, LNNA only), 90 minutes (n=14, LNNA only), 105 minutes (n=6 LNNA only) and 120 minutes (n=6, LNNA only) and percent survival was measured for each group.

Figure 8 shows the survival dose response curve for N-ω-nitro-L-arginine (LNNA). Wild-type mice were given an i.p. injection of normal saline (0.008 cc/g) or 0, 2, 10, 20, or 30 mg/kg LNNA (0.008 cc/g) 15 minutes prior to

compression and then exposed to 75 minutes at 6 ATA oxygen. Percent survival was calculated for each treatment group.

Figure 9 shows the percent survival in wild-type mice pretreated with saline, 20 mg/kg N-ω-nitro-L-arginine (LNNA), or 20 mg/kg N-ω-nitro-L-argiaine plus 50 mg/kg L-arginine (LNNA + L-Arg) and then exposed to 75 minutes of 6 ATA oxygen. *$p<0.05$ tested with a χ-square test with Bonferroni correction.

Figure 10 shows the percent survival in transgenic and nontransgenic mice exposed to 6 ATA oxygen for 75 minutes. Mice were injected with saline or given 20 mg/kg N-ω-nitro-L-arginine (LNNA) i.p. 10 minutes before compression. *$p<0.05$ tested by $\chi^2$ compared to nontransgenic saline treated mice. † $p<0.05$ tested by $\chi^2$ compared to transgenic saline treated mice.

Figure 11 shows the comparison of edema formation in EC-SOD transgenic mice to edema formation in nontransgenic littermates after cold-induced injury to the right cerebral hemisphere as well as in non-injured mice. Values are presented as mean ± standard error. *$p<0.05$ compared to Edema Index of respective nontransgenic controls using a paired Student's t-test.

Figure 12 shows the effect of augmented levels of EC-SOD on vascular permeability changes after cold-induced brain injury. Vascular permeability is demonstrated as Evan's blue leakage in the injuried right cerebral hemispheres of nontransgenic (control) and EC-SOD transgenic mice.

Figure 13 shows a Western blot analysis of rh-EC-SOD and a human lung homogenate to demonstrate antibody specificity. Proteins were separated on a 10% 0.75 mm SDS-polyacrylamide gel and transferred to nitrocellulose. Proteins were hybridized with the antibody to recombinant human EC-SOD (4.3 μg/ml) and the antibody was detected by hybridization with $^{125}$I-Protein-A followed by autoradiography. The EC-SOD lane contained 0.05 μg of pure recombinant human type C EC-SOD lane protein. The lung lane contained 10 μg of a 20,000 x g supernatant of a human lung homogenate.

Figures 14A-14C show the light microscopic immunohistochemical localization of BC-SOD in human lung. Tissues were labeled using the antibody to recombinant human BC-SOD (5.4 mg/ml; anti-EC-SOD) or the same antibody in which the anti-EC-SOD IgG was absorbed out using purified recombinant EC-SOD attached to CNBr-sepharose (EC-SOD absorbed). Antibody was detected using a biotin/streptavidin-hoseradish peroxidase labeling technique. A, Large elastic pulmonary artery labeled with anti-EC-SOD. Note labeling around smooth muscle cells beneath the endothelium and beneath the elastic layer of the vessel (short arrow), and the lack of labeling for EC-SOC on the surface of endothelial cells (open arrow) and on elastin (long arrow). B, Muscular pulmonary artery labeled with anti-EC-SOD. Note high amount of labeling in the connective tissue matrix surrounding the vessel and lymphatics (long arrow), in the matrix surrounding smooth muscle cells (short arrow), and the lack of labeling on the surface of endothelial cells (open arrow). C, Muscular pulmonary artery labeled with EC-SOD absorbed antisera. The absorption of anti-EC-SOD IgG abolished all labeling in the muscular vessel. (Bars = 50 μm).

Figures 15A-15C show the immunohistochemical localization of EC-SOD in human lung. Tissues were labeled using the antibody to recombinant human EC-SOD (5.4 mg/ml; anti-EC-SOD). Antibody was detected using a biotin/streptavidin-horseradish peroxidase labeling technique. A, Large cartilaginous airway labeled with anti-EC-SOD. Note the intense labeling for EC-SOD in the matrix around smooth muscle cells (short arrow), between the epithelial cells (long arrow), and the lack of labeling on the surface of the epithelial cells (open arrows), and in the matrix of cartilage (asterisk). B, Noncartilaginous airway labeled with anti-EC-SOD. Note the intense labeling for EC-SOD throughout the entire matrix beneath the epithelium (short arrow), and the lack of labeling on the surface of the epithelium (open arrow). C, Lung parenchyma labeled with anti-EC-SOD. EC-SOD labeling is primarily at alveolar septal tips (short arrow), and in the matrix surrounding small vessels (long arrow). No labeling for EC-SOD was seen on the surface of alveolar epithelial cells (open arrow). (Bars = 50 μm).

Figures 16A-16C show the electron microscopic immunolocalization of EC-SOD in vascular connective tissue. Tissues were labeled using the antibody to recombinant human EC-SOD (40 μg/ml; anti-EC-SOD) or the same antibody after the anti-EC-SOD IgG was absorbed out using purified recombinant EC-SOD attached to CNBr-sepharose (EC-SOD absorbed). Antibody was detected using 10 nm protein-A gold. A, Vascular collagen labeled with anti-EC-SOD, B, Vascular elastin labeled with anti-EC-SOD. C, Vascular collagen labeled with EC-SOD absorbed antisera. Note the intense labeling of EC-SOD in association with type I collagen and the lack of labeling in association with elastin (E). In addition, absorption of anti-EC-SOD antibody abolished all labeling for EC-SOD

in association with type I collagen. (Bars = 200 nm).

Figure 17 shows the electron microscopic immunolocalization of EC-SOD around vascular smooth muscle. Tissues were labeled using the antibody to recombinant human EC-SOD (40 µg/ml). Antibody was detected using 10 nm protein-A gold. There is a high degree of labeling in the connective tissue matrix around the vascular smooth muscle cell (S) in association with type I collagen (short arrow), and other unidentified matrix elements (long arrow). (Bars = 200 nm).

Figures 18A-18B show the electron miscropic immunolocalization of EC-SOD on the surface of pulmonary endothelial cells. Tissues were labeled using the antibody to recombinant human EC-SOD (40 µg/ml). Antibody was detected using 10 nm protein-A gold. A, Endothelial cell from a small muscular pulmonary artery, B, Endothelial cell from a pulmonary capillary. No labeling for EC-SOD was on the surface of the endothelial cells (short arrows). EC-SOD is seen in the plasma (P) and is associated with extracellular matrix proteins beneath the endothelium (long arrows). (Bars = 200 nm).

Figure 19 shows the electron microscopic immunolocalization of EC-SOD around bronchial epithelial cells. Tissues were labeled using the antibody to recombinant human EC-SOD (40 µg/ml). Antibody was detected using 10 nm protein-A gold. EC-SOD was found in the junction between the epithelial cells (arrow) and was also seen to some extent inside the cells. (Bars = 200 nm).

Figures 20A-20D show a partial restriction map, sequencing strategy, genomic structure, and protein structure of human EC-SOD Clone #7. Fig. 20A, a partial restriction map of human EC-SOD genomic clone #7 is shown in the 5' to 3' orientation. A 1 kb size marker is indicated. B, BamH I; H, Hind III; P, Pst I; S, Sal I; K, Kpn I; E, EcoR I. In Fig. 20B, the subcloning and sequencing strategy is shown. Various size overlapping restriction fragments were subcloned into the plasmid vector pGEM3Zf(+) for subsequent DNA sequence analysis. All DNA was sequenced on both strands using Sequenase (USB) and double-stranded DNA template, except for ~2 kb of the 3' 7K36 fragment in which only one orientation was sequenced. In Fig. 20C, the exon/intron structure of the human EC-SOD gene is shown. The position of the coding region for preEC-SOD in exon 3 is shown by the dashed lines. In Fig. 20D, the four structural domains of human EC-SOD protein are diagrammed. The signal peptide is indicated by an arrow. This is followed by the mature glycosylated (CHO) amino terminal peptide domain. A third region has very high amino acid sequence homology to human CuZn-SOD. The carboxy terminal domain has multiple charged basic residues (+) which are critical for binding heparin glycosaminoglycan.

Figures 21A-21B show human multiple tissue Northern blots of EC-SOD. Fig. 21A, two µg of poly A(+) mRNA from eight different human tissues were electrophoresed on a denaturing agarose gel, transferred to a charged nylon membrane, and probed with [$^{32}$P]-labeled antisense human EC-SOD cRNA. RNA molecular size markers (kilobases) are shown on the right. Quantitative transfer was monitored by ethidium bromide staining. The results demonstrate a unique 1.4 kb mRNA present in all eight tissues examined. Interestingly skeletal muscle demonstrates a second, larger mRNA of ~4.2 kb, while brain shows a faint approximately 2.2 kb band. In Fig. 21B, bands corresponding to EC-SOD mRNA were quantitated by laser densitometric scanning, normalized to the 1.4 kb brain band, and expressed as relative absorbance units.

Figures 22A-22B show analysis of the transcription initiation site. The 5' rapid amplification of cDNA ends (5' RACE) technique was used to identify the site of transcription initiation for the human EC-SOD gene. In Fig. 22A, a schematic diagram illustrates the annealing sites for the various oligonucleotides. The dark line represents first-strand reverse transcribed cDNA of human heart poly A(+) mRNA which has been primed with EC7 (an EC-SOD gene specific primer) and poly C tailed using terminal deoxynucleotidyl transferase (TdT). HEC1, HEC2, EC4, and EC7 are 5' human EC-SOD gene specific primers. The anchor primer is supplied with the 5' RACE kit (GIBCO BRL) and hybridized to the poly C tail. In Fig. 22B, PCR was used to amplify segments of DNA using [anchor + EC4] or [HEC1 + EC7] as primers and either poly C tailed (+TdT, lanes 1 & 4) or non-poly C tailed (-TdT, lanes 2 & 5) cDNA as template. Lane 3 includes PCR amplified DNA using [HEC1 + EC7] as primers and a full-length human EC-SOD cDNA as template. The resulting amplified DNAs were electrophoresed on a 2% agarose gel, transferred to charged nylon membranes, and probed with [$^{32}$P]-labeled HEC2, a 5' nested gene specific EC-SOD primer. DNA molecular weight markers were run between lanes 2 and 3. The expected size of the PCR amplified region in lanes 3, 4 and 5 is 217 bp. Only a single band is seen in lane 1, with a molecular size of approximately 185 to 200 bp.

Figure 23 shows genomic Southern blot analysis of the human EC-SOD gene. Ten micrograms of human genomic DNA were completely digested with each of the restriction enzymes shown, electrophoresed on a 1% agarose gel

and transferred to charged nylon membranes. The blots were proved with a [$^{32}P$]-labeled EC-SOD partial length cRNA which corresponds to the first approximate 1050 nucleotides and autoradiographed. The specific restriction endonuclease is shown at the top of each lane. DNA molecular size markers (in kilobases) are shown on the right.

Figure 24 shows the nucleotide sequence and deduced amino acid sequence of the human EC-SOD gene. The complete nucleotide sequence of the human gene is shown. The deduced amino acid sequence of the signal peptide and mature protein is indicated using the single letter amino acid code.

Figure 25 shows a Lineweaver-Burk plot demonstrating non-competitive inhibition of xanthine oxidase by MnTBAP.

Figure 26 shows the protection of pulmonary artery endothelial cells from xanthine oxidase-induced injury by MnTBAP. Control □; MnTBAP ▨.

Figure 27 shows the protection of lung epithelial cells from paraquat-induced injury of SOD mimetics.

Figure 28 shows the protection of pulmonary artery endothelial cells from paraquat-induced injury by MnTBAP.

Figure 29 shows the lack of protection of pulmonary artery endothelial cells from paraquat-induced injury by ZnTBAP.

Figure 30 shows the protection of MnTBAP against paraquat-induced lung injury.

DETAILED DESCRIPTION OF THE INVENTION

The present invention relates to protecting against the deleterious effects of superoxide radicals and to preventing and treating disease states that involve or result from oxidant stress. The invention also relates to modulating biological processes involving superoxide radicals.

Mimetics of SOD appropriate for use in the present methods include manganic derivatives of methine substituted porphines, or pharmaceutically acceptable salts thereof. Preferred mimetics are of the formula:

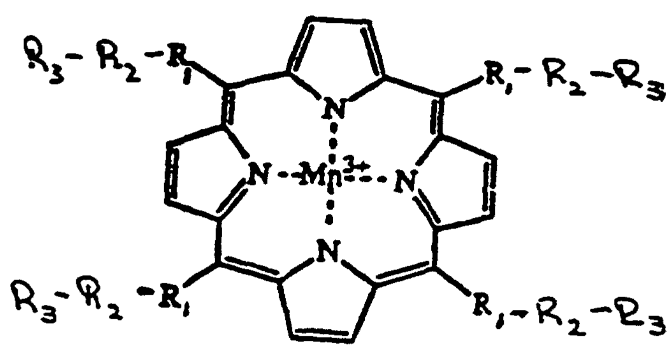

wherein $R_1$, $R_2$ and $R_3$ are as defined above.

In a more specific embodiment,

$R_1$ is a bond,

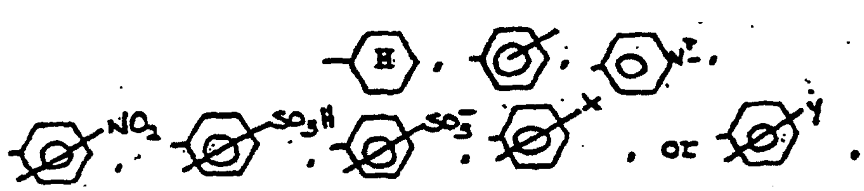

wherein X is Cl or Br and Y is a $C_{1-4}$ alkyl group;

$R_2$ is a bond, $-(CY'_2)_n-$, $-(CY'_2-CY'=CY')_n^-$, $-(CY'_2-CY'_2-CH=CH)_n^-$, $-(CY'=CY')_n^-$, or

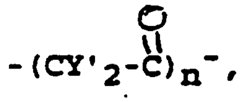

wherein Y' is hydrogen or a $C_{1-4}$ alkyl group and wherein n is 1 to 4; and

$R_3$ is Y", -OH, $-NH_2$, $-N^+(Y")_3$, -COO-, $-COO^-$, $-SO_3-$, $-SO_3^-$, $-C-PO_3H-$ or $-C-PO_3H^-$, wherein Y" is a $C_{1-4}$ alkyl group.

In a further specific embodiment,

$R_1$ is a bond,

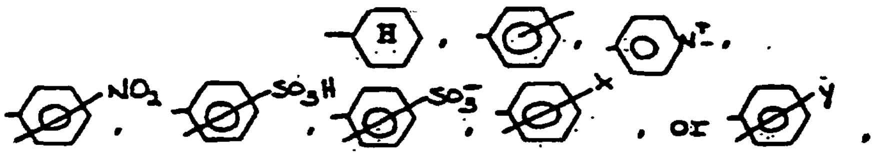

, or

wherein X is Cl or Br and Y is methyl or ethyl, and

$R_2$ is a bond, $-(CY'_2)_n^-$, $-(CY'_2-CY'=CY')_n^-$, $-(CY'_2-CY'_2-CH=CH)_n^-$, $-(CY'=CY')_n^-$, or

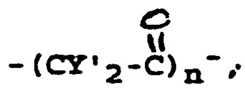

wherein Y' is hydrogen or methyl or ethyl and wherein n is 1 or 2; and

$R_3$ is methyl, ethyl, -OH, $-NH_2$, $-N^+(CH_3)_3$, $-N^+(CH_2CH_3)_3$, -COO-, $-COO^-$, $-SO_3-$, $-SO_3^-$, $-C-PO_3H-$ or $-C-PO_3H^-$.

In another specific embodiment,

$R_1$ is a bond,

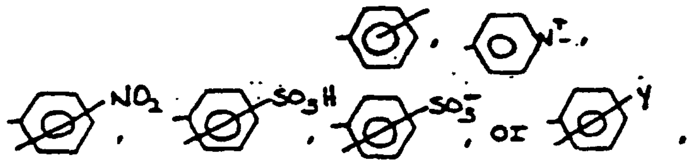

, or

wherein Y is alkyl, preferably, $C_{1-4}$ alkyl, more preferably methyl or ethyl,

$R_2$ is a bond, $-(CY'_2)_n^-$, $-(CY'=CY')_n^-$, or

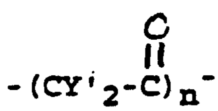

,

wherein Y' is hydrogen or alkyl (preferably $C_{1-4}$ alkyl, more preferably methyl or ethyl) and wherein n is 1 to 4 (preferably 1 or 2); and

$R_3$ is $C_{1-4}$ alkyl (preferably methyl or ethyl), -OH, -NH-, $-N^+(CH_3)_3$, $-N^+(CH_2CH_3)_3$, -COO-, $-COO^-$, $-SO_3-$, $-SO_3^-$, $-C-PO_3H-$ or $-C-PO_3H^-$.

In yet another specific embodiment,

$R_1$ is a bond,

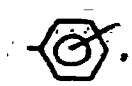 , 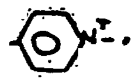 , 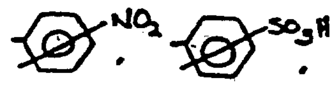 ,

or

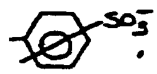 .

$R_2$ is a bond, $-(CY'_2)_n^-$ or $-(CY'=CY')_n^-$, wherein Y' is hydrogen or alkyl (preferably $C_{1-4}$ alkyl, more preferably methyl or ethyl) and wherein n is 1 to 4 (preferably 1 or 2), and

$R_3$ is $C_{1-4}$ alkyl (preferably methyl or ethyl), -OH, -NH-, $-N^+(CH_3)_3$, $-N^+(CH_2CH_3)_3$, -COO-, $-COO^-$, $-SO_3-$, $-SO_3^-$, $-C-PO_3H-$ or $-C-PO_3H^-$.

In addition to the substituents described above, one or more of the pyrrole rings of the porphyrin can be substituted with an electron withdrawing group, for example, a $NO_2$ group, a halogen (eg Cl), or a nitrite.

Specific mimetics suitable for use in the present methods include Mn(III) tetrakis (1-methy-4-pyridyl)porphyrin (MnTMPyP), Mn(III) tetrakis (4-trimethyl-aminophenyl)porphyrin (MnTMAP) and Mn(III) tetrakis (4-benzoic acid)porphyrin (MnTBAP).

Targeted forms of the mimetics can be produced by coupling directly or via a linker to a cell surface or extracellular matrix targeting moiety.

The targeted mimetics can be used to mimic EC-SOD. Since the sequence of human EC-SOD is known (Hjalmarsson et al, Proc. Natl. Acad. Sci. USA 84:6340 (1987)), the C-terminal oligopeptide can prepared and attached to the "mimetic core" (eg, a Mn(III)-porphyrin) via, for example, a free amine or carboxy group with a 1-ethyl-3-(3-dimethylaminopropyl) carbodiimide (EDC) coupling reaction (Yamada et al, Biochemistry 20:4836 (1981); Davis and Preston, Anal. Biochem. 116:402 (1981)) (note the R' group in the structures set forth below). This simple coupling procedure makes it possible to attach a wide variety of different binding domains in order to target the EC-SOD mimetics. Heparin binding affinity of a mimetic can be assessed by passing the mimetic over a heparin-sepharose CL-6B column (Karlsson et al, Biochem. J. 256:29 (1988)).

Candidate targeting moieties suitable for attaching to SOD (eg EC-SOD) mimetics to confer GAG binding properties include the following:

i) the A+ helix of protein C inhibitor (Boissinot et al, Biochem. Biophys. Res. Commun. 190:250 (1993)) - $NH_2$ - His Arg His His Pro Arg Glu Met Lys Lys Arg Val Glu Asp Leu - COOH;

ii) the C-terminal end of human EC-SOD (heparin binding domain) (Karlsson et al, Biochem. J. 255:223 (1988)) - $NH_2$ - Arg Glu His Ser Glu Arg Lys Lys Arg Arg Arg Glu Ser Glu Cys Lys Ala Ala - COOH;

iii) variants of the C-terminal end of human EC-SOD having heparin binding affinity (Sandström et al, J. Biol. Chem. 267:18205 (1992)) -

a. $NH_2$ - Arg Glu His Ser Glu Arg Lys Lys Arg Arg Arg Glu - COOH;
b. $NH_2$ - Arg Glu His Ser Glu Arg Lys Lys Arg Arg Arg Ala - COOH;
c. $NH_2$ - Arg Glu His Ser Glu Arg Lys Lys Arg Arg Arg Ala Ser Glu Cys Lys Ala Ala - COOH;
d. $NH_2$ - Arg Glu His Ser Glu Arg Lys Lys Arg Arg Arg Glu Ser Glu Ala Lys Ala Ala - COOH;
e. $NH_2$ - Arg Glu His Ser Glu Arg Lys Lys Arg Arg Arg Glu Ser Glu Cys Ala Ala Ala - COOH;
f. $NH_2$ - Arg Glu His Ser Glu Arg Lys Lys Arg Arg Arg Ala Ser Ala Cys Lys Ala Ala - COOH;
g. $NH_2$ - Arg Glu His Ser Glu Arg Lys Lys Arg Arg Arg Ala Ser Glu Cys Ala Ala Ala - COOH;
h. $NH_2$ - Arg Glu His Ser Glu Arg Lys Lys Gly Arg Arg Ala Ser Glu Cys Ala Ala Ala - COOH;

iv) any combination of repeating positively changed amino acids (ie poly $(Arg)_n$, poly $(Lys)_n$, poly $(Arg)_n$ $(Lys)_n$ or poly $(Arg\ Lys)_n$ or poly $(Lys\ Arg)_n$, poly $(Lys\ Lys\ Arg\ Arg)_n$, wherein n is, preferably, in the range of 1 to 12, more preferably, 1 to 8, and, most preferably, 1 to 6, with or without a C-terminal Glu or Ala (Sandström et al, J. Biol. Chem. 267:18205 (1992)); and

v) polyethyleneimine, e.g. $(NH-CH_2-CH_2-NH)nH$, wherein n is 1 to 6.

In addition to the foregoing, targeting moieties also include heparin binding proteins generally and sugars, including mannose and oligosaccharides.

Appropriate linkers include

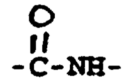 or 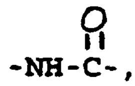,

$-SO_2-NH-$, $-PO_3-NH-$, or

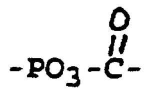.

Specific examples of suitable SOD (eg EC-SOD) mimetics are set forth below:

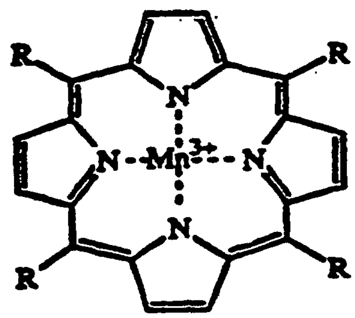

R = 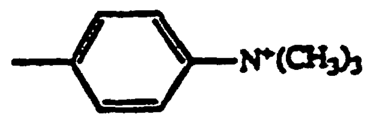

R = 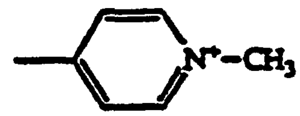

R = 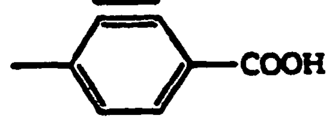

R = 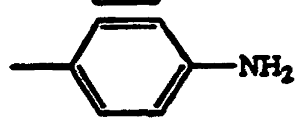

R = 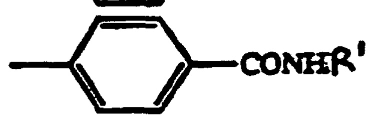

R = 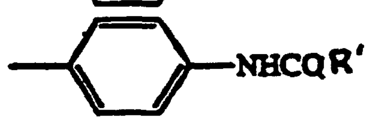

R' = NH2-ArgGluHisSerGluArgLysLysArgArgArg GluSerGluCysLysAlaAla-COOH

5,10,15,20-Tetra Kis [R-group] manganese (III) porphyrin

Mimetics suitable for use in the present invention can be selected by assaying for SOD activity and stability. SOD activity can be monitored in the presence and absence of EDTA using the method of McCord and Fridovich (J. Biol. Chem. 244 (1969)). The efficacy of a mimetic can be determined by measuring the effect of the mimetic on the growth of a SOD null *E. coli* strain versus a wild type strain. Specifically, wild type *E. coli* (AB1157) and SOD null *E. coli*. (JI132) can be grown in M9 medium containing 0.2% casamino acids and 0.2% glucose at pH 7.0 and 37°C; growth can be monitored in terms of turbidity followed at 700 nm spectrophotometrically. Active mimetics can be tested for toxicity in mammalian cell culture by measuring lactate dehydrogenase (LDH) release. Specifically, rat L2 cells (a lung type II like cell; (Kaighn and Douglas, J. Cell Biol. 59:160a (1973)) can be grown in Ham's F-12 medium with 10% fetal calf serum supplement at pH 7.4 and 37°C; cells can be seeded at equal densities in 24 well culture dishes and grown to approximately 90% confluence; SOD mimetics can be added to the cells at log doses (eg micromolar doses in minimal essential medium (MEM)) and incubated for 24 hours. Toxicity can be assessed by morphology and by measuring the release of the cytosolic injury marker, LDH (eg on a thermokinetic plate reader), as described by vassault

(In: Methods of Enzymatic Analysis, Bergmeyer (ed) pp. 118-26 (1983); oxidation of NADH is measured at 340 nm). Efficacy of active mimetics can be assessed by determining their ability to protect mammalian cells against methylviologen (paraquat)-induced toxicity. Specifically, rat L2 cells grown as described above and seeded into 24 well dishes can be pre-incubated with various concentrations of the SOD mimetic and then incubated with a concentration of methylviologen previously shown to produce an $LC_{75}$ in control L2 cells. Efficacy of the mimetic can be correlated with a decrease in the methylviologen-induced LDH release (St. Clair et al, FEBS Lett. 293:199 (1991)). The efficacy of SOD mimetics can be tested *in vivo* with mouse and/or rat models using both aerosol administration and parental injection. For example, male Balb/c mice can be randomized into 4 groups of 8 mice each to form a standard 2X2 contingency statistical model. Animals can be treated with either paraquat (40 mg/kg, ip) or saline and treated with SOD mimetic or vehicle control. Lung injury can be assessed 48 hours after paraquat treatment by analysis of bronchoalveolar lavage fluid (BALF) damage parameters (LDH, protein and % PMN) as previously described (Hampson et al, Tox. Appl. Pharm. 98:206 (1989); Day et al, J. Pharm. Methods 24:1 (1990)). Lungs from 2 mice of each group can be instillation fixed with 4% paraformaldehyde and processed for histopathology at the light microscopic level.

Synthesis of mimetics suitable for use in the present invention can be effected using art-recognized protocols. In the case of Mn(III)-porphyrin mimetics, porphyrin rings with various methine bridge carbon side groups can be purchased commercially and the Mn(III) metal ion inserted into the porphyrin ring by methods including the following: (1) admixture of Mn(II) acetate with porphyrin in the presence of oxygen, under which condition selective stabilization of Mn(III) by the porphyrin causes autooxidation of Mn(II); (2) preparation of $Mn(III)(OH)_3$ by a modification of the Winkler method (Sastry et al, Anal. Chem. 41:857 (1969)) followed by reaction with the porphyrin; (3) stirring $MnO_2$ with the porphyrin in the presence of $NH_2OH$, which serves to reduce the Mn(IV) to Mn(III), which is then trapped by the porphyrin; or (4) a method modified from Pasternack et al (Biochemistry 22:2406 (1983)) which refluxes excess $MnCl_3$ with the porphyrin. Mn(III)-porphyrin complexes can be precipitated from solution with sodium perchlorate, washed and residue perchlorate removed by strong anionic exchange resin. Formation of the Mn(III)-porphyrin can be followed spectrophotometrically by monitoring a characteristic Sorét band at 468 nm. Coupling of a binding domain to the "mimetic core" can be carried out as described above.

The present invention results, at least in part, from the realization that EC-SOD specifically regulates NO· function. In addition, the invention is based on the realization that EC-SOD is synthesized by epithelial cells and is primarily localized in the interstitium, on matrix elements and collagen and around smooth muscle cells (particularly lung airways and vasculature). NO· is an intercellular signal and, as such, NO· must traverse the extracellular matrix to exert its effects. NO·, however, is highly sensitive to inactivation mediated by $O_2^-$ present in the extracellular spaces. EC-SOD is thus an enzyme ideally suited to increase the bioavailability of NO· by preventing its degradation by $O_2$-.

As indicated above, the invention also relates to mimetics of EC-SOD that can be targeted to strategic locations and to the use of such mimetics in manipulating extracellular levels of NO·. The invention, however, is not limited to NO· manipulation as the sole mechanism of action of the mimetics. Rather, the invention relates to oxygen radical scavenging generally.

The present invention relates, in a further specific aspect, to inhibiting production of superoxide radicals. In this aspect, the SOD mimetics are used to inhibit oxidases, such as xanthine oxidase, that are responsible for production of superoxide radicals (see Example VII). The ability of a mimetic to protect mammalian cells from xanthine/xanthine oxidase-induced injury can be assessed, for example, by growing rat L2 cells in 24-well dishes. Cells can be pre-incubated with various concentrations of an SOD mimetic and then xanthine oxidase (XO) can be added to the culture along with xanthine (X). The appropriate amount of XO/X used in the study can be pre-determined for each cell line by performing a dose-response curve for injury. X/XO can be used in an amount that produces approximately an $LC_{75}$ in the culture. Efficacy of the mimetic can be correlated with a decrease in XO/X-induced LDH release. The ability of the mimetics to inhibit the production of such radicals makes possible the use the mimetics as therapeutics for the treatment of gout and reperfusion injuries.

The mimetics can be used to scavenge hydrogen peroxide, as well as superoxide radicals. As scavengers of hydrogen peroxide, the mimetics serve as mimics of either catalase or peroxidase. Appropriate mimetic scavengers can be selected by following absorbance at 240 nm in the presence of hydrogen peroxide (see Beers and Sizer, J. Biol. Chem. 195:133 (1952)). Therapies based on this activity correspond with those noted below.

The mimetics can be used as catalytic scavengers of superoxide radicals to protect against ischemia reperfusion injuries associated with myocardial infarction, stroke, acute head trauma, organ reperfusion following transplantation, bowel ischemia, pulmonary infarction, surgical occlusion of blood flow, and soft tissue injury. The mimetics can further be used to protect against skeletal muscle reperfusion injuries. The mimetics can also be used to protect against damage to the eye due to sunlight (and to the skin) as well as glaucoma, and macular degeneration of the eye. Diseases of the bone are also amenable to treatment with the mimetics. Further, connective tissue disorders associated with defects in collagen synthesis or degradation can be expected to be susceptible to treatment with the present mimetics.

In addition to the above, the mimetics can be used as catalytic scavengers of superoxide radicals to increase the very limited storage viability of transplanted hearts, kidneys, skin and other organs and tissues.

The amount of mimetic to be used in a particular treatment or to be associated with a particular substance can be determined by one skilled in the art.

The availability of the mimetics also makes possible studies of $O_2^-$ mediated processes.

Diagnostic protocols are made possible by the availability of the BC-SOD gene sequence (see Example V and Figure 24). A defect in the BC-SOD gene is more likely to occur than a defect in nitric oxide synthase due to the nature and number of physiological functions served by NO·. Detection of an EC-SOD gene defect would signal the need for measures to be undertaken to elevate levels of functional EC-SOD, or related superoxide scavenging compounds, at strategic locations to correct NO· imbalances and thus disorders involving oxidative stress.

To effect modulation of the efficacy of extracellular NO·, eg, in relaxing smooth muscle, molecules (agents) having EC-SOD activity are administered under conditions such that levels of extracellular $O_2^-$ are altered. Molecules suitable for use include forms of SOD that bind heparin sulfate or other glycosaminoglycans (GAG), for example, by virtue of the fact that they contain positively charged amino acids near their carboxy terminal end.

The general requirements of mimetics are that they: (a) be stable enough to retain the ligated metal (i.e. Mn) in the presence of the multiple chelating agents present in living systems, (b) be active enough that reasonable doses can serve to significantly augment the total SOD activity in the extracellular spaces, (c) be able to adhere to the surfaces of cells or extracellular matrix elements (eg collagen) when protection against extracellular sources of $O_2^-$ is needed, and d) be of low toxicity. Examples of suitable mimetics include nitrogen-containing macro cyclic ligands effective as catalysts for dismutating superoxide, including Mn(III) complexes of porphyrins with bulky cationic substituents on the methine bridge carbons, such as those described above (eg MnTMAP and MnTMPyP). Such complexes are very active and are stable enough to retain full activity in the presence of excess EDTA or in the presence of tissue extracts.

The mimetics described above can be formulated into pharmaceutical compositions suitable for use in the present invention. Such compositions include the active agent together with a pharmaceutically acceptable carrier, excipient or diluent. The composition can be present in dosage unit form for example, tablets, capsules or suppositories. The composition can also be in the form of a sterile solution suitable for injection or inhalation. Compositions can also be in a form suitable for opthalmic use. The invention also includes the use of compositions formulated for topical administration, such compositions taking the form, for example, of a lotion, cream, gel or ointment. The concentration of active agent to be included in the composition can be selected based on the nature of the agent, the dosage regimen and the result sought.

The dosage of the composition to be administered can be determined without undue experimentation and will be dependent upon various factors including the nature of the active agent, the route of administration, the patient, and the result sought to be achieved.

Suitable doses of mimetics will vary, for example, with the mimetic and with the result sought. The results of Faulkner et al (J. Biol. Chem. 269:23471 (1994)) indicate that the *in vivo* oxidoreductase activity of the mimetics is such that a pharmaceutically effective dose will be low enough to avoid problems of toxicity. Doses that can be used include those in the range of 1 to 50 mg/kg.

Further examples of diseases or disorders appropriate for treatment using the present invention include diseases of the central nervous system (including AIDS dementia, stroke, amyotrophic lateral sclerosis (ALS), Parkinson's disease and Huntington's disease) and diseases of the musculature (including diaphramic diseases (eg respiratory fatigue in emphysema, bronchitis and cystic fibrosis), cardiac fatigue of congestive heart failure, muscle weakness syndromes associated with myopathies, ALS and multiple sclerosis). Many neurologic disorders (including stroke, Huntington's disease, Parkinson's disease, ALS, Alzheimer's and AIDS dementia) are associated with an over stimulation of the major subtype of glutamate receptor, the NMDA (or N-methyl-D-aspartate) subtype. On stimulation of the NMDA receptor, excessive neuronal calcium concentrations contribute to a series of membrane and cytoplasmic events leading to production of oxygen free radicals and nitric oxide (NO·). Interactions between oxygen free radicals and NO· have been shown to contribute to neuronal cell death. Well-established neuronal cortical culture models of NMDA-toxicity have been developed and used as the basis for drug development. In these same systems the SOD mimetics of the invention inhibit NMDA induced injury.

The present invention also relates to treating arthritis, systemic hypertension, atherosclerosis, edema, septic shock, pulmonary hypertension, including primary pulmonary hypertension, impotence, infertility, endometriosis, premature uterine contractions, memory disorders, microbial infections and gout.

Therapeutic regimens, including mode of administration, appropriate for effecting treatment of the conditions described above can be readily determined by one skilled in the art.

Inflammations, particularly inflammations of the lung, are amenable to treatment using the present invention (note particularly the inflammatory based disorders of asthma, ARDS including oxygen toxicity, pneumonia (especially AIDS-related pneumonia), cystic fibrosis, chronic sinusitis and autoimmune diseases (such as rheumatoid arthritis)). EC-SOD is localized in the interstitial spaces surrounding airways and vasculature smooth muscle cells. EC-SOD and $O_2^-$ mediate the antiinflammatory - proinflammatory balance in the alveolar septum. NO· released by alveolar septal cells acts to suppress inflammation unless it reacts with $O_2^-$ to form $ONOO^-$. By scavenging $O_2^-$, EC-SOD tips the

balance in the alveolar septum against inflammation. Significant amounts of ONOO- will form only when BC-SOD is deficient or when there is greatly increased $O_2^-$ release. EC-SOD mimetics, such as those described herein, can be used to protect against destruction caused by hyperoxia. Appropriate therapeutic regimens can be readily established by one skilled in the art.

As indicated above, it is expected that defects in the EC-SOD gene, that are manifest in the protein itself, may in fact be the cause of pathologic problems related to NO· function, rather than defects in nitric oxide synthase. Thus, diagnostic protocols suitable for use in identifying EC-SOD gene defects are disclosed herein. This aspect is based on the availability of the EC-SOD gene sequence. Figure 24 presents a gene sequence as well as portions of non-coding regions of that sequence of at least 15 bases, preferably, at least 50 bases, more preferably, at least 100 bases and most preferably, at least 500 bases. Such portions, and the complements thereof can be used as probes or primers in protocols including those described in Example VI.

Screening of subjects for defects in the EC-SOD gene can be done using the approach used to identify mutations on the β-adrenergic receptor gene (Reihaus et al, Am. J. Respir. Cell. Mol. Biol. 8:334 (1993)). That approach is similar to the one used by Rosen et al (Nature 262:59 (1993)) (see Example VI).

The following are predicted sites of important gene mutations:

Positions 1-558: This represents a 5' flanking region which contains transcriptional regulatory elements. Mutations here can be expected to lead to deficient levels of EC-SOD or defective enhancement or reduction in EC-SOD levels under conditions which require manipulating the EC-SOD concentration. The following regions have been identified as putative regulatory regions. Mutations in these regions can be expected to result in deficient levels of EC-SOD:

| | |
|---|---|
| 89 - 95 | metal regulatory response element |
| 121 - 126 | cyclic AMP responsive element |
| 370 - 375 | glucocorticoid response element |
| 238 - 244 | skeletal muscle trans-activating factor response element |
| 251 - 256 | cis responsive element in the induction of the c-fos proto-oncogene |
| 162 - 168 | TPA reponsive element |
| 171 - 179 | SV40 enhancer region |

Positions 560-570: Mutations here can be expected to lead to an inability to splice out intron 1. This would result in no EC-SOD production (or much reduced) due to initiation of translation at multiple cryptic ATG sites located in intron 1 which are upstream of the EC-SOD ATG start codon. For example, base pair 653, 656, 720, 743, 748, etc, would potentially initiate translation.

Positions 564-1135: Intron 1 contains DNA sequence unique to EC-SOD. In addition, there are potential transcription regulatory regions within this DNA stretch which are listed below; mutations in intron 1 would lead to deficient levels of EC-SOD or defective enhancement or reduction in EC-SOD levels under conditions that require manipulating the EC-SOD concentration:

| | |
|---|---|
| 1085 - 1095 | Xenobiotic responsive region |
| 650 - 661 | Antioxidant responsive element |

Positions 71-95: Mutations here can be expected to lead to an inability to splice out intron 1. This would result in no EC-SOD production (or much reduced) due to initiation of translation at multiple cryptic ATG sites located in intron 1 that are upstream of the EC-SOD ATG start codon. For example, base pair 653, 656, 720, 743, 748, etc, would potentially initiate translation.

Positions 1211-1230: Mutations here can be expected to lead to an inability to splice out intron 2. This would result in no EC-SOD production (or much reduced) due to initiation of translation at multiple cryptic ATG sites located in intron 2 that are upstream of the EC-SOD ATG start codon. Examples of upstream ATG translation start sites can be found at 1339 and 1518.

Positions 5055-5080: Mutations here would lead to an inability to splice out intron 2. This would result in no EC-SOD production (or much reduced) due to initiation of translation at multiple cryptic ATG sites located in intron 2 which are upstream of the EC-SOD ATG start codon. Examples of upstream ATG translation start sites can be found at 1339 and 1518.

Positions 5085-5138: Mutations here would (1) interfere with efficiency of translation of EC-SOD resulting in deficient levels of the enzyme (2) interfere with targeting of EC-SOD to the endoplasmic reticulum which is required

for secretion of EC-SOD, (3) interfere with co-translational signal peptide processing (ie, removal of the signal peptide) that may lead to deficient levels due to inability to proteolytically cleave the signal peptide from the mature protein which in turn would result in the protein being trapped in the endoplasmic reticulum, (4) interfere with post-translational processing (specifically glycosylation) which may result in defective levels due to the synthesis of poorly soluble protein.

Positions 5139-5150: Mutations here may interfere with the signal peptidase cleavage site resulting in a mutant EC-SOD which would contain an altered amino terminus leading to defective EC-SOD function of deficient levels.

Positions 5403-5405: Mutations here can be expected to result in loss of glycosylation which may result in defective levels due to the synthesis of poorly soluble protein.

Positions 5424-5720: Mutations here can be expected to result in defective EC-SOD activity. This region is critical for binding to the substrate and catalyzing the dismutation of superoxide anion radical. In addition, this region would also affect any other activities of this enzyme including the reduction of other species such as nitric oxide, etc.

Positions 5721-5804: Mutations in this region would cause defects in binding of EC-SOD to target tissues such as type I collagen in the extracellular matrix, and around smooth muscle cells in both vessels and bronchi. Such mutations here are highly likely to cause disease.

Positions 6385-6395: Mutations here can be expected to result in defective polyadenylation that can lead to deficient levels of EC-SOD due to a decreased half-life of EC-SOD mRNA species.

Certain details of the present invention are described in greater detail in the non-limiting Examples that follow.

**Example I**

Preparation and Characterization of Transgenic Mice

Protocols:

i) Construction of Transgenic Mice

*Construction of the human EC-SOD expression vector*: The EC-SOD expression vector (Figure 1) was constructed as follows: The entire human EC-SOD cDNA fragment (Hjalmarrson et al, Proc. Natl. Acad. Sci. USA 84:6340 (1987); Hendrickson et al, Genomics 8:736 (1990)) flanked by EcoRI restriction sites was converted with mung bean nuclease to form blunt-ends, ligated to SalI linkers, digested with SalI, and then inserted into the SalI site of the human β-actin expression vector pHβAPr-1. The EcoRI-HindIII fragment of the resultant plasmid containing the human β-actin promoter (provided by Dr. Larry Kedes of the University of Southern California, Los Angeles, California), intron, and EC-SOD cDNA was isolated. In addition, the HpaI site of SV40 at position 2666 in plasmid pMSG (Pharmacia LKB Biotechnology, Piscataway, New Jersey) was converted to a HindIII site by linker ligation and the HindIII-PstI fragment containing the polyadenylation site of the SV40 early region was isolated. These two DNA fragments were then ligated to an EcoRI plus PstI digested pKS vector (Stratogene, La Jolla, California). The EcoRI-XbaI fragment containing the entire expression construct free of plasmid sequences was isolated and used to establish transgenic mice. All of the recombinant DNA procedures were done according to established methods (Sambrook et al, Molecular Cloning: A Laboratory Manual 3, Cold Spring Harbor; Cold Spring Harbor Laboratory, 1989).

*Development of transgenic mice*: Purified DNA at 2.5 μg/ml in 5 mM Tris-HCl, pH 7.4, 0.1 mM EDTA was injected into the pronuclei of fertilized eggs isolated from mice ((C57BL/6 X C3H)F1 X (C57BL/6 X C3H)F1)((C57BL/6 X C3H)F1 mice were purchased from Charles River). Mouse eggs surviving microinjection were then implanted into the oviducts of pseudopregnant foster mothers (CD1) (CD1 mice were purchased from Charles River) following procedures described by Hogan et al (Hogan et al, Manipulating the Mouse Embryo, Cold Spring Harbor; Cold Spring Harbor Laboratory 1986, 32). Mice carrying the transgene were identified by Southern blot analysis of tail DNA probed with the entire human EC-SOD cDNA. Transgenic founders were found the first litter screened. These mice were bred with (C57BL/6 X C3H)FI to produce offspring for further studies. (In all of the following experiments with the EC-SOD transgenic mice, the nontransgenic mice refer to littermates of the transgenic mice that did not contain the transgene for the human EC-SOD. In experiments in which EC-SOD transgenic mice were not used, wild type (C57BL/6 X C3H) FI were used.)

*Production of homozygous EC-SOD transgenic mice*: Homozygous transgenic mice were produced by an $F_1$ cross of heterozygous transgenic mice. Tail DNA from $F_2$ mice were isolated and treated with RNAase. 10 μg of DNA from each mouse was cut with PstI and then electrophoresed through a 1.2% agarose gel. Southern blot analysis of tail DNA probed with the entire human EC-SOD cDNA was then done. The human EC-SOD cDNA did not cross-react with the mouse EC-SOD gene. Band intensity was compared visually to determine which mice were homozygous, heterozygous, or negative for the human EC-SOD transgene.

ii) Characterization of Transgenic Mice

_Northern analysis_: Transgenic mice and nontransgenic littermates were killed with an overdose of pentobarbital. Tissues were quickly excised and frozen in liquid nitrogen until ready for further processing. Total RNA was then isolated by the CsCl procedure was described (Sambrook et al, Molecular Cloning: A Laboratory Manual. 3. Cold Spring Harbor, Cold Spring Harbor Laboratory, 1989). Twenty µg of total RNA from the tissues of transgenic mice and nontransgenic littermates and an RNA ladder were then denatured with glyoxal, electrophoresed through a 1.2% agarose gel and blotted to nitrocellulose as described (Sambrook et al, Molecular Cloning: A Laboratory Manual. 3. Cold Spring Harbor, Cold Spring Harbor Laboratory, 1989). The blots were then probed with the entire human EC-SOD cDNA.

_Separation of SOD isoenzymes by concanavalin A sepharose chromatography_: Tissues taken from 3 mice were weighed, then combined and homogenized in 10 volumes of ice-cold 50 mM potassium phosphate, pH 7.4, with 0.3 M KBr, 3 mM diethylenetriaminepentaacetic acid, and 0.5 mM phenylmethylsulfonyl fluoride. Separation of EC-SOD from CuZn SOD and Mn SOD was accomplished by passing tissue homogenates over a concanavalin A sepharose column as described (Marklund et al, Clin. Chim. Acta 126:4 (1982)).

_SOD activity_: EC-SOD activity and total SOD activity (CuZn SOD and Mn SOD) remaining after EC-SOD extraction were measured by inhibition of cytochrome C reduction at pH 10, as previously described (Crapo et al, Methods Enzymol. 53:382 (1978)). Total protein was determined by the BCA protein assay (Pierce, Rockford, IL). The SOD activities were then expressed as units/mg total protein.

Results:

i) EC-SOD Transgenic Mice

_Characterization of transgenic mice_: Mice carrying the human EC-SOD transgene were detected by Southern blot analysis. Northern analysis of various tissues from the F1 of one mouse found to carry the transgene is shown in Figure 2. High levels of message for human EC-SOD were detected in the heart, skeletal muscle, and brain of transgenic mice, with little or no message observed in the lung, liver, and spleen. No message was detectable in nontransgenic littermates.

Homozygous mice were generated by breeding two heterozygous F1 mice. Homozygous mice were detected by differential band intensities found using Southern blot analysis of equal amounts of PstI digested DNA from the offspring. EC-SOD activity in the mice was found to increase in response to the total copies of the EC-SOD transgene (Table I).

TABLE I

EC-SOD activity in tissues of nontransgenic, heterozygous transgenic, and homozygous transgenic mice. Tissues from 3 mice were combined for each measurement. Activity is expressed as Units/g tissue wet weight.

| Tissue | Nontransgenic | Heterozygous | Homozygous |
|---|---|---|---|
| Brain | 18 | 38 | 50 |
| Heart | 35 | 69 | 102 |

**Example II**

Central Nervous System Oxygen Toxicity

Protocols:

_Oxygen exposures_: 7-8 week old mice were exposed to hyperbaric oxygen five at a time in a small-animal chamber (Bethlehem, Pennsylvania). After flushing the chamber with pure oxygen, compression to 50 meters (6 ATA) was performed within 5 minutes. The oxygen concentration in the chamber was monitored continuously with a Servomex oxygen analyzer (model 572, Sybron, Norwood, Massachusetts) and maintained at ≥99%. The carbon dioxide concentration was analyzed from intermittent samples of chamber gas with an IR detector (IR Industries, Santa Barbara, California) and was not allowed to rise above 0.1%. The chamber temperature was maintained at 25-26°C, but the compression of oxygen in the chamber raised the temperature to 30-32°C transiently, but an environmental control system restored the normal chamber temperature within 3 minutes. The exposures lasted 25 to 75 minutes and were followed by decompression for 5 minutes. The mice were observed continuously for signs of oxygen toxicity from the beginning of the exposure until 4 hours after removal from the chamber. The time to the first generalized convulsion

(seizure latency) and the time to death were recorded. These exposure conditions are designed to cause CNS oxygen toxicity without appreciable evidence of pulmonary oxygen toxicity.

*Treatment with diethyldithiocarbamate*: One hour prior to exposure to 6 ATA oxygen, mice were given either i.p. injections of either 0.008 cc/g saline or 400 mg/kg diethyldithiocarbamate dissolved in normal saline (0.008 cc/g). The mice were then exposed to 6 ATA oxygen for 25 mintues as described above.

To determine the extent of EC-SOD and CuZn SOD inhibition by diethyldithiocarbamate, mice were given diethyldithiocarbamate and sacrificed one hour later. The brains were removed and assayed for EC-SOD and CuZn SOD activity as described above.

*Treatment with β-mercaptoethanol*: One hour prior to exposure to 6 ATA oxygen, mice were given either i.p. injections of 0.008 cc/g saline or 180 mg/kg β-mercaptoethanol (0.008 cc/g). This dose of β-mercaptoethanol was selected because it contains an equal number of reducing thiols as the dose of diethyldithiocarbamate. The mice were then exposed to 6 ATA oxygen for 30 minutes as described above.

*Treatment with N-ω-nitro-L-arginine, an inhibitor of nitric oxide synthase*: Ten minutes prior to beginning compression, 0.008 cc/g saline or 20 mg/kg (0.008 cc/g) N-ω-nitro-L-arginine dissolved in sterile water was given i.p to the transgenic and nontransgenic mice. Mice were then exposed at 6 ATA oxygen for 25 or 75 minutes as described above.

*Statistical analysis*: A paired Student's t-test was used to compare enzyme activities in transgenic and nontransgenic mice. A $\chi^2$ test with Bonferroni correction was used to assess significance in survival differences to hyperbaric exposures. Analysis of variance with a Scheffe F-test was used to compare differences in seizure latency in the different groups of mice.

Results:

*Hyperbaric oxygen exposures*: To test the effects of increased brain EC-SOD levels on CNS oxygen toxicity, both transgenic and nontransgenic mice (see Example I) were exposed to 6 ATA oxygen for 25 minutes. Transgenic mice were more susceptible (83% mortality) to CNS oxygen toxicity than nontransgenic mice (33% mortality) (Figure 3).

Transgenic and nontransgenic mice were subsequently treated with an inhibitor of CuZn SOD, diethyldithiocarbamate, to confirm that the increased sensitivity of transgenic mice to CNS oxygen toxicity was the result of increased SOD activity. In both transgenic and nontransgenic mice, treatment with 400 mg/kg diethyldithiocarbamate resulted in 80% inhibition of EC-SOD and 60% inhibition of CuZn SOD in the brain. This is consistent with previous findings (Frank et al, Biochem. Pharmacol. 27:251 (1978); Heikkila et al, J. Biol. Chem. 251:2182 (1976)). Treatment with diethyldithiocarbamate conferred increased resistance to CNS oxygen toxicity for both transgenic and nontransgenic mice. Survival increased to 100% in transgenic mice and 93% in nontransgenic mice (Figure 3). The onset of seizures was also delayed four-fold in mice treated with diethyldithiocarbamate (Figure 4) .

To evaluate whether or not diethyldithiocarbamate protects against CNS oxygen toxicity by acting as a reducing agent rather than as an inhibitor of SOD activity, mice were treated with an equimolar amount of reducing thiols in the form of β-mercaptoethanol and exposed to hyperbaric oxygen. Figure 5 shows that β-mercaptoethanol did not protect against CNS oxygen toxicity.

One possibility that might explain why EC-SOD exacerbates CNS oxygen toxicity would be that nitric oxide is a mediator of CNS oxygen toxicity and EC-SOD is protecting nitric oxide from superoxide mediated inactivation. To test the hypothesis that nitric oxide contributes to CNS oxygen toxicity, wild-type (C57BL/6 X C3H)F1 mice were treated with an inhibitor of nitric oxide synthase, N-ω-nitro-L-arginine. Figure 6 shows the effects of N-ω-nitro-L-arginine on seizure latency in mice. Pretreatment with N-ω-nitro-L-arginine resulted in a significant increase in seizure latency (13.50±5.6 min) when compared to saline treated mice (2.75±1 min). Figure 7 shows that nitric oxide synthase inhibition also significantly increased survival after exposure to hyperbaric oxygen. Mice given the inhibitor of nitric oxide synthase displayed 50% mortality after exposure to 90 minutes of 6 ATA oxygen and 100% mortality was not obtained until after 2 hours of this exposure. Saline treated mice, however, had a 50% mortality after only 25 minutes of exposure, with 100% mortality after only 30 minutes at 6 ATA of oxygen. Figure 8 shows that the percent survival in hyperbaric oxygen was dependent on the dose of the inhibitor given. The protection offered by this competitive inhibitor of nitric oxide synthase could be reversed when an excess of L-arginine was given (Figure 6 and Figure 9).

The effects of the nitric oxide synthase inhibitor, N-ω-nitro-L-arginine, upon CNS oxygen toxicity was then studied in the transgenic mice. This treatment dramatically reduced CNS oxygen toxicity in both transgenic and nontransgenic mice. Survival after a 25 minute exposure to 6 ATA oxygen increased to 100% in both groups (Figure 3). Seizure latency was also significantly delayed (Figure 4). The exposure time was then increased to 75 minutes to investigate whether transgenic mice were still more sensitive than nontransgenic mice to hyperbaric oxygen. The results in Figure 10 indicate that treatment with N-ω-nitro-L-arginine abolished the difference in sensitivity that was observed between untreated transgenic and nontransgenic mice during the 25 minute exposure shown in Figure 3.

**Example III**

Cold-Induced Brain Edema

Protocols:

*Injury model*: Young (6-7 week) mice (see Example I) were anesthetized with 60 mg/kg pentobarbital (Nembutal, Abbott Laboratories, Chicago, Illinois). An incision was then made in the scalp and a steel rod 20 cm long, 3 mm in diameter, equilibrated in liquid nitrogen with an 8 cm bath of liquid nitrogen 4 cm from the end of the rod, was placed on the skull over the right cerebral hemisphere for 30 seconds. The skin incision was then sutured.

Two hours after the injury the mouse was given an additional dose of pentobarbital. The chest cavity was opened, the lungs were excised, and the mouse was then perfused with 20 ml saline through the left ventricle of the heart. The brain was then removed and the cerebellum was excised. The right (R) and left (L) cerebral hemispheres were separated and immediately weighed (wet weight, W). Each hemisphere was then dried at 70°C for 2-3 days in a hot air oven until a constant weight was achieved (dry weight, D). An index of edema (I) was then calculated as shown in equation 13.

$$I=(W/D\ R - W/D\ L)/(W/D\ L) \times 100 \qquad [13]$$

This calculation allowed the left hemisphere to serve as an internal control for the injured right hemisphere in each mouse.

*Chemical treatments*: Six groups of experiments were conducted to investigate the importance of extracellular superoxide, iron, and nitric oxide in cold-induced brain edema. In all groups, drugs were dissolved in saline and injected at 0.008 cc/g 15 minutes prior to cold injury. In Group 1, edema formation of EC-SOD transgenic mice was compared with that of nontransgenic littermates. Group 2 compared edema formation between wild-type (C57BL/6 X C3H)F1 mice treated with saline and (C57BL/6 X C3H)F1 mice treated with 0.33 mg/g deferoxamine (0.51 μmoles/g). Group 3 compared (C57BL/6 X C3H)F1 mice treated with saline to (C57BL/6 X C3H)F1 mice treated with 0.51 μmoles/g $Fe^{3+}$-saturated deferoxamine. Group 4 consisted of (C57BL/6 X C3H)F1 mice treated with saline and (C57BL/6 X C3H)F1 mice treated with 0.02 mg/g N-ω-nitro-L-arginine methyl ester. Group 5 consisted of (C57BL/6 X C3H)F1 mice treated with saline and (C57BL/6 X C3H)F1 mice treated with 0.02 mg/g N-ω-nitro-L-arginine methyl ester plus 0.05 mg/g L-arginine. Group 6 compared edema formation between nontransgenic mice, EC-SOD transgenic mice treated with saline, and EC-SOD transgenic mice treated with 0.02 mg/g N-ω-nitro-L-arginine methyl ester.

Iron saturated deferoxamine was made by dissolving equimolar amounts of deferoxamine and then ferric chloride in saline. Saturation of deferoxamine with ferric iron was determined spectrophotometrically be measuring the absorbance at 425 nm ($\varepsilon$=2500 $M^{-1}$ $cm^{-1}$ for $Fe^{3+}$-deferoxamine) (Monzyk and Crumbliss, J. Amer. Chem. Soc. 104: 4921 (1982)).

*Evan's blue treatment*: One hour and 50 minutes after cold injury, 5 ml/kg of 1% Evan's Blue in saline was injected into the femoral artery of transgenic and nontransgenic mice. The mice were sacrificed 10 minutes later. The lungs were then excised and the mice were then perfused with normal saline through the left ventricle until there was no more blue color in the effluent. The brains were then removed and photographed.

*Statistical analysis*: A paired Student's t-test was used to compare significance of edema development compared to nontransgenic mice or saline treated mice for each of the groups examined. Analysis of variance with a Fisher PLSD test was used to compare significance in Group 6. P-value less than 0.05 were considered to be significant.

Results:

*Cold-induced Brain Edema*: When transgenic mice and nontransgenic littermates were subjected to cold-induced injury to the right cerebral hemisphere it was found that the transgenic mice were significantly protected against edema formation compared to nontransgenic littermates (Figure 11). Percent edema was 44% less in transgenic mice then in nontransgenic littermates and Evan's blue dye extravasation was visibly less in transgenic mice compared to nontransgenic littermates (Figure 12).

To test the contribution of iron to edema formation in this model, mice were pretreated with i.p. injections of deferoxamine or saline prior to cold-induced injury. Table II shows that pretreatment with deferoxamine resulted in 43% less edema formation compared to mice only given saline. Mice were then pretreated with i.p. injections of iron-saturated deferoxamine or saline before cold-induced injury to see if the iron chelating properties of this compound were truly necessary for protection against edema formation. Table IV shows that, even when deferoxamine was saturated with iron, it was still capable of protecting against edema formation and resulted in 32-48% less edema than that found

in saline treated controls. The absolute values for the edema index were found to quite variable, however, repeated experiments consistently show the same signficant trends in protection against edema formation in the various treatments examined.

Table II

| Evaluation of the effect of the ferric iron chelator deferoxamine on edema formation after cold-induced brain injury. Wild-type (C57BL/6 X C3H)F1 mice were treated with deferoxamine to determine what effect the iron chelating properties of this compound had on edema formation. Values are presented as mean ± standard error. | | | |
|---|---|---|---|
| | **Treatment** | **n** | **Edema Index** |
| | Saline | 7 | 5.92±0.62 |
| | Deferoxamine | 7 | 3.41±0.49* |
| **Run 1**: | | | |
| | Saline | 6 | 8.24±0.53 |
| | Fe-Deferoxamine | 6 | 5.63±0.63* |
| **Run 2**: | | | |
| | Saline | 5 | 7.65±1.30 |
| | Fe-Deferoxamine | 5 | 3.97±0.59* |

*p<0.05 compared to Edema Index of respective saline treated controls using a paired Student's t-test.

These results indicate that deferoxamine is capable of protecting against edema formation by a mechanism independent of its ability to scavenge iron. Because deferoxamine is capable of scavenging both the peroxynitrite anion (Radi et al, Arch. Biochem. Biophys. 288(2):481 (1991)) as well as the hydroxyl radical (Hoe et al, Chemico-Biological Interactions 41:75 (1982)), it was hypothesized that it is these properties of deferoxamine that enable it to protect against vasogenic edema.

To test this hypothesis, the synthesis of nitric oxide was inhibited with N-ω-nitro-L-arginine methyl ester, a competitive inhibitor of the enzyme nitric oxide synthase, to determine if this would result in protection against edema formation after a cold-induced injury. Table III shows that treatment with N-ω-nitro-L-arginine methyl ester significantly protected mice against edema formation resulting in 37% less edema formation than that occurring in saline treated controls. This protection by N-ω-nitro-L-arginine methyl ester was reversed by simultaneous administration of an excess of L-arginine to the mice (Table III).

Table III

| The effect of inhibition of nitric oxide synthesis on edema formation after cold-induced brain injury. Wild-type (C57BL/6 X C3H)F1 mice were treated with the competitive inhibitor of nitric oxide synthase, N-ω-nitro-L-arginine methyl ester (LNAME) to determine what effect nitric oxide had on vasogenic edema. Mice were also given N-ω-nitro-L-arginine methyl ester plus an excess of L-arginine (LNAME + L-Arg) to see if the effects seen with LNAME alone could be reversed. Values are presented as mean ± standard error. | | |
|---|---|---|
| **Treatment** | **n** | **Edema Index** |
| Saline | 6 | 5.77±0.29 |
| LNAME | 6 | 3.65±0.51* |
| | | |
| Saline | 6 | 6.56±0.21 |
| LNAME + L-Arg | 6 | 6.03±0.71 |

*p<0.05 compared to Edema Index of respective saline treated controls using a paired Student's t-test.

In the final experiments, EC-SOD transgenic mice were treated with either saline or N-ω-nitro-L-arginine methyl ester to determine if there was an additive effect in preventing edema formation in mice which have both increased levels of EC-SOD as well as the inhibitor of nitric oxide synthase. Table IV shows that when EC-SOD transgenic mice were given the inhibitor of nitric oxide synthase, no added protection against edema formation was detected relative to transgenic mice protected only by increased levels of EC-SOD in the brain.

Table IV

| Evaluation of the effect of inhibition of nitric oxide synthesis on edema formation in transgenic mice. Comparison of edema formation in nontransgenic mice to edema formation in transgenic mice with elevated levels of brain EC-SOD activity, and to edema formation in transgenic mice treated with an inhibitor of nitric oxide synthesis (20 mg/kg N-ω-nitro-L-arginine; Transgenic + LNAME) 15 minutes prior to cold-induced injury. Values are presented as mean ± standard error and were compared using analysis of variance with a Fisher PLSD test. No significant difference was seen between transgenic and transgenic + LNAME mice. | | |
|---|---|---|
| **Treatment** | **n** | **Edema Index** |
| Nontransgenic | 6 | 7.91±0.67 |
| Transgenic | 6 | 4.91±0.78* |
| Transgenic + LNAME | 6 | 4.30±0.96* |

*p<0.05 compared to Edema Index of nontransgenic mice.

Example IV

Immunolocalization of EC-SOD

Protocols:

*Human lung*: Five human lung samples were obtained to evaluate the distribution of EC-SOD in human lung tissue. One sample was obtained from a surgical pathology specimen of a right upper lobe resected from a 43 year old white female with a 50 pack year smoking history (equivalent to one pack per day for one year) and a solitary nodule found on chest X-ray. The patient was diagnosed with squamous cell carcinoma. Tissue from a region not involved in the carcinoma from this lobe was used in the studies presented here. A second lung was obtained from a right upper lobe surgical pathology specimen resected from a 51 year old white male with a 60 pack year smoking history found to have an isolated nodule on X-ray. The patient had no other illness and was diagnosed with squamous cell carcinoma. Lung tissue not involved in the carcinoma from this specimen was used for the localization of EC-SOD. A third lung was obtained from a rapid autopsy (tissue obtained 6 hours after death) of a 66 year old white male with dementia, but no history of smoking or lung disease. The fourth lung examined was obtained from excess lung tissue of a lung too large for the recipient of a lung transplant. The lung was donated from a 45 year old white female with no history of smoking or lung disease. The fifth lung examined in these studies was also from excess lung tissue used for lung transplantation from a 39 year old white male with no history of smoking or lung disease. Notably, no differences in labeling patterns were seen between the surgical pathology specimens, the autopsy tissues from donors for lung transplantation.

The tissues were fixed in 2% paraformaldehyde/0.2% gluteraldehyde in 0.01 M phosphate buffered saline (PBS; 1.2 g $NaH_2PO_4$, 8 g NaCl, 0.2 g KCl, in 1 liter pH 7.3) for 1 hour followed by overnight fixation in 4% paraformaldehyde at 4°C and then in O.C.T. compound. The tissues were frozen in liquid nitrogen chilled hexane and stored at -70°C until they were sectioned for light microscopic studies.

For electron microscopic studies, lung tissues were processed as in the light microscopic studies up to the equilibration in sucrose. After equilibration in sucrose, the lung tissues were infiltrated with 10% gelatin at 37°C for 10 minutes. The tissue slices, in gelatin, were then solidified on ice, cut into 2 mm/side cubes, and then cryoprotected in 4% polyvinyl alcohol containing 2 M sucrose overnight. These samples were then mounted onto stubs, flash frozen in liquid nitrogen, and then stored in liquid nitrogen until they were sectioned for electron microscopic studies.

*Characterization of antibody to human recombinant*

*EC-SOD*: Human recombinant EC-SOD (furnished by S.L. Marklund, Umeå, Sweden; Tibell et al Proc. Natl. Acad. Sci. USA 84:6634 (1987)) and the 20,000 x g supernatant of a human lung homogenate were denatured in the presence of β-mercaptoethanol and sodium dodecyl sulfate by boiling for 5 minutes and then electrophoresed through 12% polyacrylamide gel in the presence of sodium dodecyl sulfate. The protein was then electrophoretically transferred to nitrocellulose. The blot was then incubated with 4.3 μg/ml of an IgG purified fraction of rabbit anti-rh-EC-SOD (furnished by S.M. Marklund, Umeå University Hospital, Umeå, Sweden) affinity purified with rh-EC-SOD followed by incubation with $^{125}$I-protein A and autoradiography.

*Absorption of anti-EC-SOD IgG*: CNBr activated sepharose was swollen in PBS. Swollen gel was mixed with PBS so that the settled gel occupied 50% of the volume. The gel was suspended and 100 μl was mixed with 100 μg

pure rh-EC-SOD for 2 hours at room temperature while gently agitating. The gel was then washed 4 times with PBS + 1% bovine serum albumin (BSA) and made up 100 µl with PBS + 1% BSA. 100 µl of rabbit anti-rh-EC-SOD at two times the concentration used for immunolabeling was then added and mixed for 2 hours with gentle agitation at room temperature. Non-immune rabbit IgG was then added to the supernatant in a concentration equivalent to the predicted concentration of anti-rh-EC-SOD IgG removed by the procedure. This supernatant was then used for subsequent immunolabeling.

*Light microscopic immunohistochemistry*: 4 µm serial sections of O.C.T. embedded tissue were cut on a cryostat at -20°C and put on poly-L-lysine-coated slides (3 sections/slide). Sections were stored at -70°C until labeling was done. Sections were then labeled for EC-SOD using an indirect immunoperoxidase method (Milde et al, J. Histochem. Cytochem. 37:1609 (1989); Randell et al, Am. J. Respir. Cell. Mol. Biol. 4:544 (1991)) with a biotinylated goat anti-rabbit IgG and streptavidin-horseradish peroxidase (Jackson, ImmunoResearch Laboratoreis (West Grove, Pennsylvania)) (Table V). To reduce background staining, the sections were incubated in 1% $H_2O_2$ in methanol to inactivate endogenous peroxidases, 10 mM borohydride to block aldehydes, and nonspecific binding was blocked by incubation with 5% normal goat serum (NGS), 5% milk, and 1% BSA in PBS. The optimal primary and secondary antibody dilutions were determined empirically and made in PBS with 1% milk plus 1% BSA (milk was not included in the streptavidin solution). The slides were developed using diaminobenzidine (10 mg diaminobenzidine, 50 ml 0.05 M Tris-Cl, pH 7.6, 100 µl 3% $H_2O_2$) and counterstained with 1% methyl green. As a control, serial sections were separately labeled with either rabbit anti-rh-EC-SOD (EC-SOD), non-immune rabbit IgG, or rabbit anti-rh-BC-SOD from which EC-SOD binding IgG had been absorbed out (EC-SOD absorbed; see above).

TABLE V

| Staining procedures for light microscopic immunohistochemistry. All incubations were performed in a humidified chamber at room temperature. | | |
|---|---|---|
| | Incubation | Time |
| Step 1 | 1% $H_2O_2$ in methanol | 30 minutes |
| Step 2 | 10 mM borohydride in PBS (gluteraldehyde fixed tissue only) | 30 minutes |
| Step 3 | 5% NGS, 5% milk, 1% BSA/PBS | 30 minutes |
| Step 4 | Primary antibody 1% milk, 1% BSA/PBS (various dilutions) | 1 hour |
| Step 5 | Biotin-labeled goat anti-rabbit IgG (1:6000 in 1% milk, 1% BSA/PBS) | 1 hour |
| Step 6 | Streptavidin-Horse radish peroxidase (1:2000 in 1% BSA/PBS) | 1 hour |
| Step 7 | Diaminobenzidine | 15 minutes |
| Step 8 | 1% Methyl Green in water | 15 minutes |

*Electron microscopic immunocytochemistry*: Ultrathin cryo sections (70 nm) of human lung tissue were immunolabeled with rabbit anti rh-EC-SOD and 10-nm protein A-gold as prevsiously described (Crapo et al, Proc. Natl. Acad. Sci. USA 89:10405 (1992)) (Table VI). Briefly, sections were first incubated three times for five minutes at room temperature in 0.15% glycine in PBS to block aldehyde groups. Nonspecific binding was further blocked by incubation in 1% BSA in PBS for 10 minutes. Primary and secondary antibody dilutions were determined empirically and made in PBS containing 1% BSA. Sections were stained with uranyl oxalate and uranyl acetate in methyl cellulose as previously described (Crapo et al, Proc. Natl. Acad. Sci. USA 89:10405 (1992)). Control groups were as described for light microscopy above.

TABLE VI

| Staining procedures for electron microscopic immunohistochemistry. All incubations were performed at room temperature. | | |
|---|---|---|
| | Incubation | Time |
| Step 1 | PBS + 0.15% glycine | 3 x 5 minutes |
| Step 2 | 1% BSA/PBS | 5 minutes |
| Step 3 | Primary antibody in 1% BSA/PBS | 45 minutes |
| Step 4 | Protein -A gold | 30 minutes |
| Step 5 | Uranyl oxalate | 5 minutes |
| Step 6 | Uranyl acetate/methyl cellulose | 10 minutes |

Results:

*Characteristic of EC-SOD antibody*: The antibody to rh-EC-SOD was characterized by Western blot analysis of rh-EC-SOD and a human lung homogenate. Figure 13 shows that the antibody reacted with both the EC-SOD type C (top band) and the type A (bottom band) subunits (Sandström et al, Biochem. J. 267:18205 (1992) in a human lung homogenate. The type A subunit does not exist in the interstium of tissues *in vivo* (Sandström et al, Biochem. J. 290: 623 (1993)). The antibody reacted with three bands in the lane containing purified type C rh-BC-SOD. The two lowest molecular weight species in Figure 13 are due to partial insufficient glycosylation of the rh-ECSOD in the heavily overproducing CHO-cells.

*Light microscopic immunohistochemistry*: Using an antibody to rh-EC-SOD, this protein was immunolocalized in human lungs. Light microscopic immunohistochemistry revealed with BC-SOD is mainly associated with the connective tissue matrix around vessels and airways in the lung (Figure 14a and b, Figure 15a, b, and c). EC-SOD was found in close proximity to vascular and airway smooth muscle (Figure 14a and b, and Figure 15a). EC-SOD was also seen in connective tissue of alveolar septal tips (Figure 15c) suggesting an affinity of BC-SOD for connective tissue matrix. No labeling was seen in association with vascular endothelial cells in large elastic arteries, medium-sized vessels, or capillaries, (Figure 14a and b). EC-SOD was notably absent from the epithelial cell surfaces of airways (Figure 15a and b) and was also not present in cartilage (Figure 15a).

The antibody to EC-SOD was an IgG polyclonal rabbit antibody which was affinity purified using rh-EC-SOD. To test the specificity of the labeling for EC-SOD, IgG specific for EC-SOD was absorbed out of the antisera using pure rh-EC-SOD bound to cyanogen bromide sepharose. Nonimmune rabbit IgG was then added to this absorbed antibody in a sufficient amount to replace the absorbed IgG. Labeling lung tissues with this preabsorbed antibody preparation resulted in the absence of labeling in all areas of the lung including the pulmonary vasculature (Figure 14c). Labeling lung tissue with nonimmune IgG alone also resulted in the absence of labeling in all areas of the lung. The controls indicate that the labeling observed with the primary antibody is specific for EC-SOD in the lung.

*Electron microscopic immunocytochemistry*: A summary of the labeling for EC-SOD in the lung found using electron microscopic immunocytochemistry is summarized in Table VII. EC-SOD was mainly associated with extracellular matrix proteins in all regions of the lung. In particular, a high degree of labeling was seen in areas rich in type I collagen (Figure 16) and in association with other unidentified proteoglycans in extracellular matrix (Figure 17). Notably, no labeling for EC-SOD was seen in elastin-rich areas (Figure 16). A high degree of labeling was observed near the surface of smooth muscle cells and in the connective tissue matrix surrounding smooth muscle cells in vessels (Figure 17) and airways. Labeling was notably absent from the surface of endothelial cells on small, medium and large vessels (Figures 18a and b). The lack of endothelial cell labeling found with the light microscopic immunohistochemistry support the electron microscopic findings. Labeling of EC-SOD was also seen in plasma within the lumen of blood vessels (Figure 18a). The localization of EC-SOD in plasma is expected as this protein was first discovered in plasma (Marklund, Acta Physiol. Scand., 5492:19 (1980)). Labeling for EC-SOD was observed in the intercellular junctions between bronchial epithelial cells (Figure 19), but was absent from the apical surface of these cells. Finally, EC-SOD labeling was absent from the surface of type I and type II cells. A moderate, but consistent amount of intracellular EC-SOD was found in type II epithelial cells and in bronchial epithelial cells (Figure 19).

Table VII

| Distribution of EC-SOD in human lung. (+) indicates presence of labeling for EC-SOD and (-) indicates no labeling for EC-SOD. (±) represents areas where low low amounts of labeling for EC-SOD were inconsistently observed. | |
|---|---|
| Location | EC-SOD |
| **Cell Surfaces** | |
| Endothelial | - |
| Type I cell | - |
| Type II cell | - |
| Smooth muscle cell | + |
| Fibroblast | ± |
| **Extracellular Matrix** | |
| Type I collagen | + |
| Elastin | - |
| Cartilage | - |
| Unidentified matrix elements | + |

Table VII (continued)

| Distribution of EC-SOD in human lung. (+) indicates presence of labeling for EC-SOD and (-) indicates no labeling for EC-SOD. (±) represents areas where low low amounts of labeling for EC-SOD were inconsistently observed. | |
|---|---|
| Location | EC-SOD |
| **Intracellular** | |
| Endothelial cell | ± |
| Type I cell | - |
| Type II cell | + |
| Bronchial epithelial cell | + |
| Smooth muscle cell | - |
| Fibroblast | ± |
| **Blood** | |
| Plasma | + |
| Red Blood Cell | - |

Controls done by absorbing EC-SOD specific antibody out of the primary antibody and replacing this absorbed antibody with nonimmune rabbit IgG resulted in the absence of labeling in all areas of the lung including areas rich in type I collagen as seen in Figure 16c. In addition, use of nonimmune rabbit IgG instead of the primary antisera also resulted in the absence of labeling in all areas of the lung. The lack of labeling with these controls indicates that the labeling observed with the primary antisera is specific for EC-SOD in the lung.

The localization of EC-SOD on the surface of smooth muscle cells and in the extracellular matrix around these cells in both blood vessels and airways indicates that EC-SOD may have an important function in this location. Superoxide is known to rapidly react with nitric oxide and inactivate its properties as a smooth muscle relaxant. Therefore, the presence of EC-SOD along the diffusion path of nitric oxide to smooth muscle cells should increase the half life of this short lived intercellular messenger in this particular region and thus increase its potency as a vasodilator. The high labeling for EC-SOD seen around vascular and airway smooth muscle cells indicates a function for EC-SOD as a mediator of nitric oxide activity in the maintenance of low pulmonary vascular pressures and airway resistence.

In addition to the labeling of EC-SOD in association with smooth muscle cells, EC-SOD also appears to strongly colocalize with type I collagen. Collagen has previously been demonstrated to be susceptible to attack by reactive oxygen species such as the superoxide anion. In addition, the superoxide anion may be capable of activating latent collagenases from polymorphonuclear leukocytes (PMN) which can lead to further collagen degradation. Because collagen fragments have been shown to chemoattract and activate PMN's, any increased produced of superoxide that results in collagen degradation may accelerate inflammatory reactions and tissue destruction through PMN recruitment and activation. Consequently, the association of EC-SOD with collagen may be important in preventing superoxide mediated degradation of collagen and therefore, represent a means of controlling inflammatory responses.

Example V

Human EC-SOD Gene

Protocols:

*Materials and radiochemicals*:

[$\alpha$-$^{35}$S]dATP (~1400 Ci/mmol), [$\gamma$-$^{32}$P]ATP (3000 Ci/mmol), and [$\alpha$-$^{35}$P]CTP (800 Ci/mmol), were purchased from New England Nuclear. Human genomic DNA, $T_7$, $T_3$, and SP6 RNA polymerase, RNasin, and the pGEM3Zf(+) plasmid were obtained from Promega Biotec. Sequenase sequencing kit (V 2.0) was from United States Biochemicals Corporation. Human poly A+ RNA was acquired from Clontech. SeaPlaque GTG agarose was from FMC BioProducts. Restriction enzymes were from New England Biolabs. All other reagents used were molecular biology grade. Oligonucleotides were synthesized using an Applied Biosystems 380B or 392 by the Duke University, Department of Botany DNA synthesis facility. Charged nylon membranes (GeneScreen Plus) were from DuPont.

*Human Northern blot or analysis*:

Two µg poly A+ RNA were purified from eight different human tissues. These mRNAs were electrophoresed on a denaturing formaldehyde 1.2% agarose gel and transferred to a charge-modified nylon membrane followed by UV irradiation fixation. The membrane was prehybridized in 50% formamide, 0.25 M $NaPO_4$ (pH 7.2), 0.25 M NaCl, 1 mM EDTA, 7% SDS, and 5% polyethylene glycol (molecular weight 8000). The blot was hybridized in the same buffer overnight at 60°C with 1 X $10^6$ cpm/ml of [$^{32}$P]-labeled human EC-SOD RNA generated by transcription of the full-length cDNA using $T_3$ RNA polymerase in the presence of [$\alpha$-$^{32}$P]CTP. The blot was washed in 0.25 M $NaPO_4$ (pH 7.2), 1% SDS, and 1mM EDTA at 75°C followed by a second wash using 0.04 M $NaPO_4$ (pH 7.2), 1% SDS, and 1 mM EDTA at 75°C for 30 minutes. This was followed by exposure to XAR-5 film using a Lightening Plus intensifier screen at -70°C. The autoradiogram was scanned using an LKB Ultrascan XL laser densitomer, and the peaks were quantitated by integration using the internal digital integrator of the densitometer or by cutting out the peak from a printer tracing and weighing.

*5' Rapid amplifciation of cDNA ends*:

0.5 µg of poly A+ mRNA from human heart was reversed transcribed using 2 pmoles of EC7, a 5' EC-SOD gene specific anti-sense oligonucleotide (5'-ATGACCTCCTGCCAGATCTCC-3'), following a protocol by GIBCO BRL (5' RACE System). The RNA template was degraded by the addition of RNase H at 55°C for 10 minutes. The resulting cDNA was isolated using a GlassMAX DNA (GIBCO BRL) isolation spin cartridge. The purified cDNA was dC-tailed using terminal deoxynucleotidyl transferase (TdT, 0.5 units/ul). 200 µM dCTP, 10 mM Tris-HCl (pH 8.4), 25 mM KCl, 1.25 mM $MgCl_2$ and 50 µg/ml bovine serum albumin for 10 minutes at 37°C. The TdT was heat inactivated for 10 minutes at 70°C.

Products of this reaction were next PCR amplified using the "anchor" primer (GIBCO BRL), which hybridizes to the homopolymeric tail, and EC4 (a nested internal 5' EC-SOD gene specific anti-sense oligonucleotide, 5'-AGGCAGGAACACAGTAGC-3'). Alternatively, the dC-tailed products were PCR amplified using BC7 and HECl (a sense-strand EC-SOD gene specific primer, 5'-TGGGTGCAGCTCTCTTTTCAGG-3'). The final composition of the reaction included 20 mM Tris-HCl (pH 8.4), 50 mM KCl, 2.5 mM $MgCl_2$, 100 µg/ml bovine serum albumin, 400 nM Anchor primer, 200 nM gene-specific primer, and 200 µM each of dATP, dCTP, dGTP, dTTP. After incubating the PCR reaction for 5 minutes at 94°C, amplitaq (Perkin Elmer Cetus) was added at a final concentration of 0.04 units/µl. PCR cycling was performed on a Perkin Elmer 9600 for 35 cycles with melting at 94°C for 45 seconds and annealing at 53°C for 15 seconds and extension at 72°C for 90 seconds. The full-length EC-SOD cDNA (6 ng) was used as a positive control in the PCR reaction. The PCR products were electrophoresed in a 2% SeaPlaque GTG agarose gel, transferred to charged nylon membranes by the method of Southern (Southern, J. Mol. Biol. 98:503 (1975)) using the alkaline transfer protocol (Reed et al, Nuc. Acids Res. 13:7207 (1985)). The DNA was fixed to the membrane by baking at 80°C in a vacuum oven for 2 hours. The subsequent blot was hybridized to [$^{32}$P] end-labeled HBC2 (an internal, nested EC-SOD specific primer, 5'-TCCAGCTCCTCCAAGAGAGC-3') overnight at 37°C. The blot was washed at increasing stringencies until background hybridization was removed. This was followed by exposure to XAR-5 film using a Lightening Plus intensifier screen at -70°C.

*Human genomic Southern blot analysis:*

Ten µg human genomic DNA were digested BamH I, EcoR I, Kpn I, and Pst I restriction endonuclease enzymes until completion. The DNA was then electrophoresed on 1% agarose gel and transferred to a charged nylon membrane by the Southern technique (Southern, J. Mol. Biol. 98:503 (1975)), after alkaline denaturation (Reed et al, Nuc. Acids Res. 13:7207 (1985)). The DNA was fixed to the membrane by heating to 80°C in a vacuum oven for 2 hours. [$^{32}$P] CTP-labeled human EC-SOD antisense strand cRNA was synthesized using the EC-SOD cDNA which had been linearized with Stu I. The blot was hybridized (500 X $10^3$ cpm/ml) in 50% formamide, 0.25 M $NaPO_4$ (pH 7.2), 0.25 M NaCl, 1 mM EDTA, 7% SDS, and 5% polyethylene glycol (molecular weight 8000), at 50°C. Following overnight hybridization, they were washed in 0.25 M $NaPO_4$ (pH 7.2), 2% SDS, and 1 mM EDTA followed by 0.04 M $NaPO_4$ (pH 7.2), 1% SDS, and 1 mM EDTA at increasing stringencies until background hybridization was minimized. The blot was exposed to XAR-5 film using a Lightening Plus intensifier screen at -70°C.

*Isolation of the human gene for EC-SOD*:

A human adult female leukocyte genomic library constructed in the EMBL-3 vector was obtained from Clontech. Approximately 1 X $10^6$ pfu were screened at a density of ~50,000 pfu/plate using [$^{32}$P]CTP-labeled human EC-SOD cRNA (1 X $10^6$ dpm/ml). The primary through tertiary screens identified approximately 7 unique putative

positive plaques. Individual plaques were isolated and lambda DNA purified using LambdaSorb phage adsorbent (Promega Biotec). The size of the insert DNA from each clone was assessed by Sal I restriction endonuclease digestion followed by electrophoresis in 0.7% agarose. Selected clones underwent extensive restriction endonuclease mapping. Based on the restriction mapping results and asymmetric hybridization using 5' and 3' annealing EC-SOD oligonucleotides, Clone #7 was selected for all subsequent DNA sequence analysis. Clone #7 contains an approximate 18-20 kb fragment.

*DNA sequencing of the human EC-SOD gene*:

The overall strategy used for sequencing clone #7 is illustrated in Figure 20. Various size restriction endonuclease DNA fragments from clone #7 were subcloned into the pGEM3Zf(+) vector DNA. The dideoxy sequencing method using double-stranded DNA (Ausubel et al, Current Protocols in Molecular Biology, Green Publishing Assoc. and Wiley Interscience, New York (1992)) as template and Sequenase enzyme (United States Biochemicals) were employed (Sanger et al, Proc. Natl. Acad. Sci. USA 74:5463 (1977)). Both the Universal and -40 M13 sequencing primers were used to initiate DNA sequencing for each subcloned fragment. Oligonucleotides derived from this initial sequencing data were synthesized approximately every 250 base pairs until the complete nucleotide sequence was obtained. Sequencing data were obtained from both strands as shown in Figure 20B except at the 3' portion of the gene where DNA sequence was obtained on one strand only.

*Computer-assisted sequence analysis and transcriptional database search*:

The IntelliGenetics Geneworks program (Version 2.2) was used for organizing the DNA sequence data. Homology searching was performed at the NCBI using the BLAST (Altschul et al, J. Mol. Biol. 215:403 (1990)) network service and the non-redundant nucleotide sequence database (GenBank(77.0) + EMBL(35.0) + EMBLUpdate + GBUdate). Transcriptional factor database searching was performed using both the SIGNAL SCAN 3.0 algorithm (Prestridge et al, CABIOS 9:113 (1993)) as well as the FINDPATTERNS program of the GCG Package (V 7.2) using Release 6.3 of the Transcription Factor Database (Gosh, Nuc. Acids Res. 18:1749 (1990)). For a prediction of the site of signal peptide cleavage, the programs SIGSEQ1 (Folz et al, J. Biol. Chem. 261:14752 (1986)) and SIGSEQ2 were employed (Folz et al, Biochem. Biophys. Res. Commun. 146:870 (1987)).

Results:

*Tissue specific expression of human EC-SOD*:

To investigate the expression of human EC-SOD, poly A(+) mRNA from eight different human tissues was fractionated on a denaturing agarose gel and transferred to a charged nylon membrane. Because a previous paper reported long exposures times in order to identify EC-SOD specific bands during genomic Southern analysis (Hendrickson et al, Genomics 8:736 (1990)), a radiolabeled antisense cRNA probe derived from full-length human BC-SOD cDNA was used (Oury et al, Proc. Nat. Acad. Sci. USA 89:9715 (1992)). A discrete band of approximately 1.4 kb can be seen in all eight human tissues analyzed (Figure 21A). In addition, skeletal muscle contains an approximate 4.2 kb message, not detected in the other tissues. By densitometric scanning of the 4.2 and 1.4 kb bands, it can be calculated that larger message to make up about 32% of the total skeletal muscle EC-SOD message. In brain, a very faint band of 2.2 kb can be seen. This band was too weak for quantitation by laser densitometer. Quantitation of these bands was performed by laser densitometry and integration of peaks of autoradiograms obtained in the linear range of exposure (Figure 21B). After normalizing to the brain, the heart showed the most expression with 10.1 times brain. This was followed by the placenta, pancreas, and lung which gave 13.6, 10.2, and 7.5, respectively. Expression in skeletal muscle was 4.7 for the 1.4 kb band or 6.9 for both 1.4 kb and 4.2 kb message, while the kidney and liver gave 6.3 and 4.1 time expression over brain. These patterns of expression have been reproduced based on probing an additional independent multiple tissue Northern blot. The bands are specific based on the relatively high stringencies of washing and from data using a sense strand EC-SOD cRNA as a probe which showed no hybridization under the conditions given.

*Mapping the site transcription initiation*:

Initially, mapping of the site of transcription initiation was attempted using the primer extension method. Using several different end-labeled 5' oligonucleotides and both human lung and human heart poly A+ mRNA as well as total RNA isolated from human foreskin fibroblasts, a positive signal was not obtained even after long exposure times. This did not seem to be due to technique since it was not possible to get positive signals using RNA generated by *in vitro*

transcription of the EC-SOD cDNA. Whether lack of success using this technique was due to very low abundance of mRNA encoding EC-SOD or some other problem(s) is unclear. Working under the assumption of low abundance mRNA, the technique of rapid amplification of cDNA ends in order to PCR amplify this signal was attempted. The EC-SOD gene specific primer EC7 was used for hybridization and reverse transcription of human heart poly A+ mRNA as shown in Figure 22. Half of this reaction was 3' dC tailed using terminal deoxynucleotidyl transferase and the remaining half was not. These templates were then subjected to PCR amplification using the gene specific primers HEC1 + EC7 as well as the anchor primer + EC4. The products of these reactions were fractioned by agarose electrophoresis, transfered to nylon membranes, and probed with the interal nested gene specific primer HEC2. An autoradiogram of this experiment is shown in Figure 22A. Using EC-SOD cDNA as a control template and HEC1 + EC7, a band of 217 bp is expected (lane 3 of Figure 22A). Since the primers HEC1 and EC7 are expected to amplify independant of dC tailing, bands of equal intensity in lanes 4 and 5, which are also of the same size as the EC-SOD control, are seen. Using the anchor primer (which hybridizes to the dC tail) and EC4, only one band of ~190 bp was seen (lane 1). Since the template was not poly C tailed, lane 2 shows no signal as expected. By subtracting 48 bp (the size of the anchor primer), the size of the reverse transcribed DNA would correspond to ~136 bp 5' of the EC4 primer. This analysis would predict that there is approximately 6 base pairs of additional 5' sequence on the cDNA clone and that transcription initiation starts about 6 bp upstream of the first intron (indicated by a dashed box). Although eukaryotic transcription initiation usually begins at an adenosine residue, it is expected that it will begin at a G (Breathnach et al, Ann. Rev. Biochem. 50:349 (1981)).

_Genomic Southern blot analysis_:

To begin to charcterize the human EC-SOD gene, 10 µg of the total human genomic DNA was restriction digested and the reaction products electrophoresed on an agarose gel followed by transfer to a nylon membrane. The blot was probed with a [$^{32}$P]-labeled partial EC-SOD cRNA. An autoradiogram of this blot is shown in Figure 23. As can be seen for each lane, there are unique bands associated with each restriction digest. No shadow bands which might suggest pseudogenes were seen. When a full-length cRNA probe was used for Kpn I digested DNA, an additional band of ~4000 bp was seen which corresponds to the 3' end of the gene. In addition, the Kpn I lane shows a 0.5 kb band which was better seen on other blots. This banding pattern was similar to a restriction map of the human EC-SOD clone #7 (see Figure 20A).

_Isolation and characterization of the human EC-SOD by DNA sequencing_:

Multiple independent positive clones were identified from a human adult leukocyte genomic library constructed in BMBL-3. These clones underwent extensive restriction endonuclease mapping and were probed with EC-SOD specific 5' and 3' oligonucleotides in order to determine the relative orientation of the inserts. Based on these results, clone #7 was picked for further analysis. Clone #7 is about 18 to 20 kb and contains at least 5000 bp of 5' flanking DNA and at least 4000 bp of 3' flanking DNA. Restriction mapping of clone #7 is shown in Figure 20A. This map is similar to the results obtained with genomic Southern blot analysis data indicating that clone #7 contains the EC-SOD gene. The strategy for sublconing and sequencing clone #7 is shown in Figure 20B. Various size continguous and overlapping restriction fragments were subcloned into the plasmid vector pGEM32f(+) (Figure 20B). The DNA inserts were sequenced on both strands using a combination of primer walking and vector specific, universal sequencing primers. The 3' half of 7K36 insert was sequenced on one strand only. Published sequence data for the human EC-SOD cDNA (Rjalmarsson et al, Proc. Natl. Acad. Sci. USA 84:6340 (1987)) as well as DNA sequence information obtained from an independant cDNA clone which contained additional 5' untranslated data (Hendrickson et al, Genomics 8:736 (1990)) were used to determine the genomic intron/exon structure. Based on a comparison of these data with the genomic sequence information, the human EC-SOD gene was determined to contain three exons and two introns (Figure 20C). Exon 1 contains at least 5 base pairs and is probably larger (by about 6 base paris), since the exact start of transcription initiation was not determined (note below). Exon 2 contains 84 bp and is separated from exon 1 by a 572 bp intervening sequence marked as intron 1. Exon 3 is separated from exon 2 by intron 2, a 3849 bp segment. Exon 3 contains a total of 1336 bp and at 17 bp into this exon starts the beginning of the complete coding sequence for preEC-SOD (Figure 20D). This includes an 18 amino acid signal peptide that precedes the 222 amino acid mature protein sequence. There are no introns separating the various structural domains of EC-SOD. These domains are shown schematically in Figure 20D and include amino acids 1-95 which contain a glycosylated Asn-89 and show no sequence homology with other proteins. Resides 96-193 show strong homology with CuZn-SOD protein sequences with preservation of critical amino acids important in enzyme catalysis and structure. Amino acids 194-222 contain multiple charged resides which have been shown to be important in binding to sulfated proteoglycans. 558 bp of the 5'-flanking region containing putative regulatory elements and 3675 bp of the 3'-flanking region were also sequenced. The exonic DNA sequence data are in agreement with the published cDNA sequence (Hjalmarsson et al, Proc. Natl.

Acad. Sci. USA 84:6340 (1987)). The intron-exon boundries are shown in Table VIII and conform to the eukaryotic consensus splice sequence (Senapathy et al, Methods Enzymol. 183:252 (1990)). Both introns split sequences in the 5'-nontranslated region of the EC-SOD gene.

## Table VIII

### Sequences at intron/exon splice junctions

**The size of the introns and exons are shown in base pairs (bp). The uppercase letters indicate exon sequence while the lowercase letters indicate intron sequence. The splice junctions shown conform to previously published concensus sequences for splice junctions (Senapathy et al, Methods Enzymol. 183:252 (1990)).**

| Donor | Intron size (bp) | Acceptor | Exon |
|---|---|---|---|
| TGCGGG gt ggac | 572 | gccc ag GCTCCA | 84 |
| GGAAAG gt gggt | 3849 | ccgc ag GTGCCC | 1336 |

Figure 24 shows the entire sequence for the human EC-SOD gene. Exonic sequences are shown in boxed uppercase letters while intronic, 5'- and 3'-flanking sequence are shown in lowercase. Exon 3 contains the entire uninterrupted coding region for EC-SOD and the protein sequence is shown using the single letter amino acid code. The 18 amino acid signal peptide and 222 amino acid mature protein sequence are highlighted. The identification of the signal peptide cleavage site is consistent with computer algorithms which predict the site of eukaryotic signal peptide cleavage (Folz et al, Biochem. Biophys. Res. Comm. 146:870 (1987)); Von Heijne, Eur. J. Biochem. 133:17 (1983)).

Transcriptional factor database searching was used to putatively identify transcriptional regulatory elements. Although almost all eukaryotic promoters utilize a TATA box element to fix the position of transcription initiation, an obvious TATA box cannot be discerned for the EC-SOD gene. Two CAAT box elements were identified. One is in the reverse orientation and located about 20 bp upstream of the first exon, while the second can be found about 335 bp upstream. The putative signal for polyadenylation is shown and the site of poly A adenylation is indicated. Transcriptional factor database searching of the 5'-nontranslated region and first intron identified several potential regulatory elements. A cAMP responsive element (CREB) (TGACGT) which is similar to the adenovirus transcription virus (ATF) element can be found starting at 121 bp (Fink et al, Proc. Natl. Acad. Sci. USA 85:6662 (1988); Sassone-Corsi Proc. Natl. Acad. Sci. USA 85:7192 (1988)). A half site for the glucocorticoid response element (GRE) (TGTCCT) is located at 370 bp (Karin et al, Nature 308:513 (1984)). A skeletal muscle specific trans-activating factor response element (M-CAT) (CATTCCT) is found in the reverse orientation beginning at position 238 (Mar et al, Mol. Cell. Biol. 10:4271 (1990)). A xenobiotic responsive element (XRE) (CACGCW) is found within the first intron at position 1085 bp (Rushmore et al, J. Biol. Chem. 265:14648 (1990)). A metal regulatory element (MRE) (TGCRCYC) is found at position 89 (Culotta et al, Mol. Cell. Biol. 9:1376 (1989)). Two putative antioxidant response elements (ARE) (RGTGACNNNGC) are found at position 650 and 5022 (Rushmore et al, J. Biol. Chem. 266:11632 (1991)). A sis responsive element (SIF) (CCCGTC) important in the induction of the c-fos proto-oncogene is found in the reverse orientation at position 251 (Wagner et al, EMBO J. 9:4477 (1990)). There is an AP1 binding site or TPA responsive element (TRE) (TGACTCA) found at position 162 (Risse et al, EMBO J. 8:3825 (1989)). The SV40 enhancer region AP4 (CAGCTGTGG) can be found at position 171 (Jones et al, Genes Dev. 2:267 (1988)).

Example VI

Screening Patients for Gene Defects in EC-SOD

Preparation of leukocyte derived genomic DNA from patients: Normal healthy control patients and patients with asthma, primary pulmonary hypertension, and secondary pulmonary hypertension will be identified. Genomic DNA will be purified utilizing a Qiagen Blood PCR Kit. One ml of blood containing $^{-}10^7$ leukocytes/ml will be collected in sodium citrate from each patient or control subject. The blood is placed into a QIAGEN-spin column and the leukocytes

are entrapped in the resin by brief centrifugation, while erythrocytes and hemoglobin are washed through. The leukocytes are lysed by the addition of 0.7 ml of lysis buffer and incubated at room temperature for 30 minutes. DNA that is released, binds to the resin in the tube. Remaining cellular debris is washed away by multiple wash/spin cycles. The DNA is eluted by the addition of 1.2 M KCl, 50 mM MOPS, 15% ethanol, pH 8.3. This typically yields ~10 µg of genomic DNA (Reihsaus et al, Am. J. Respir. Cell. Mol. Biol. 8:334 (1993)).

Primer design and PCR amplification of EC-SOD exonic sequences: Sense and antisense oligonucleotide primers (or use primers already obtained from sequencing the genomic DNA) will be designed containing a 3'GC clamp (Sheffeld et al, Proc. Natl., Acad. Sci. USA 86:232 (1989)). These primers will encode the intronless coding region of the EC-SOD gene. A 172 bp region in the 3' untranslated region has been amplified using DNA sequencing primers and human genomic DNA. PCR conditions are as described (Reihause et al, Am. J. Respir. Cell. Mol. Biol. 8:334 (1993); Innis et al (eds) Academic Press San Diego pp. 1-12 (1990)) using Taq polymerase, with temperature cycling as follows: initial denaturation at 95°C for 5 min followed by 35 cycles of 94°C denaturing for 20 sec, 57°C annealing for 15 sec, and 72°C elongation for 45 sec. Because of the GC composition and actual primer sequence, it will be necessary to experimentally optimize conditions for PCR amplification using each set of primers. Three sets of primers will be used to encompass the entire coding region.

Identification of mutations with single-strand conformational polymorphism (SSCP) analysis: SSCP analysis has been used to detect single base pair mismatch (Orita et al, Genomics 5:874 (1989)). Temperature-gradient gel electrophoresis (TGGE) will be used to detect differences in mobility (Wartell et al, Nuc. Acids Res. 18:2699 (1990)). Samples for TGGE will be prepared by heat denaturing the PCR product at 98°C for 5 min, then renaturing at 50°C for 15 min with the corresponding wild-type DNA derived from PCR of the cloned gene. Electrophoresis will be carried out on a 5% acrylamide, 8 M urea gel over a temperature gradient. The temperature gradient will be optimized for each of the EC-SOD DNA segments. Typical gradients for the detection of $\beta_2$-adrenergic receptor mutations were between 35°C to 60°C, and required 4 to 6 hours of run time (Rosen, Nature 262:59 (1993)).

All PCR samples found to be positive for mutations by TGGE will be sequenced directly using the dideoxy technique (Sanger et al, Proc. Natl. Acad. Sci. USA 74:5463 (1977)).

Example VII

Inhibition of Xanthine Oxidase

In a first study, assays were performed in a 1 ml quartz cuvette containing 50 mM carbonate buffer, pH 10, 0.1 mM EDTA, 1 nM xanthine oxidase (Boehringer Mannheim) at 25°C. Xanthine oxidase activity was measured spectrophotometrically by following the loss of xanthine over time at 295 nm. Four concentrations of xanthine (25, 50, 250, 500 µM) and 2 concentrations of MnTBAP (5, 10 µM) were used. Two inhibition constants were then derived from the curve's intercepts (Kii=5.5 µM) and slopes (Kis=15 µM). The results presented in Figure 25 show that MnTBAP inhibits xanthine oxidase in a non-competitive manner.

In a second study, calf pulmonary artery endothelial cell cultures (CPA-47 (Tissue and Cell 10:535 (1978)) were grown to confluence in Ham's F-12K medium with 10% fetal bovine serum at pH 7.4 and 37°C. Cells were then trypsinized and seeded at equal densities in 24 well plates and grown to 90% confluence. Cells were washed and preincubated for 1 hour with 50 µM of MnTBAP in minimum essential medium (MEM) or MEM only. Varing amounts of xanthine oxidase (XO) plus 200 µM xanthine (X) were added and allowed to incubate for 24 hours. Cell injury was quantitiated by measuring the release of cellular lactate dehydrogenase (LDH) into the medium. The efficacy of MnTBAP is shown in Figure 26 by the decrease in XO/X-induced LDH release.

Example VIII

SOD Mimetic Affords Cellular Protection From Paraquat-Induced Injury

Rat pulmonary epithelial cell cultures (L2 (Kaighn and Douglas J. Cell Biol. 59:160a (1973)) were grown to confluence in Ham's F-12K medium with 10% fetal bovine serum at pH 7.4 and 37°C. Cells were then trypsinized and seeded at equal densities in 24 well plates and grown to 90% confluence. Cells were washed and pre-incubated for 1 hour with 100 µM of MnTBAP or MnTMPyP in MEM or MEM only. Paraquat (2.5 mM) was added and allowed to incubate for 48 hours. Cell injury was quantitiated by measuring the release of cellular lactate dehydrogenase (LDH) into the medium. Figure 27 shows that MnTPyP (hatched bars) and MnTBAP (grey bars) decrease paraquat-induced LDH release.

In a further study, calf pulmonary artery endothelial cell cultures (CPA-47 (Tissue and Cell 10:535 (1987)) were grown to confluence in Ham's F-12K medium with 10% fetal bovine serum at pH 7.4 and 37°C. Cells were then trypsinized and seeded at equal densities in 24 well plates and grown to 90% confluence. Cells were washed and pre-incubated for 1 hour with varing concentrations of MnTBAP in MEM or MEM only. Paraquat (2 mM) was added and allowed to incubate for 24 hours. Cell injury was quantitiated by measuring the release of cellular lactate dehydrogenase (LDH) into the medium. MnTBAP decreases paraquat-induced LDH release in a dose-dependent manner (see Figure 28).

In contrast to MnTBAP, ZnTBAP does not protect against paraquat-induced injury. Calf pulmonary artery endothelial cell cultures (CPA-47) were grown to confluence in Ham's F-12K medium with 10% fetal bovine serum at pH 7.4 and 37°C. Cells were then trypsinized and seeded at equal densities in 24 well plates and grown to 90% confluence. Cells were washed and pre-incubated for 1 hour with varing concentrations of ZnTBAP in MEM or MEM only. Paraquat (2 mM) was added and allowed to incubate for 24 hours. Cell injury was quantitiated by measuring the release of cellular lactate dehydrogenase (LDH) into the medium. The results presented in Figure 29 demonstrate that ZnTBAP does not possess SOD-like activity. ZnTBAP can be used as a negative control to show that the redox metal is important in the protection against paraquat toxicity.

Example IX

Protection by MnTBAP Against Paraquat-Induced Lung Injury

Mice were treated with either paraquat (PQ, 45 mg/kg, ip) or saline (10 ml/kg, ip) and exposed to MnTBAP (2.5 mg/ml, nebulized into a 2 L chamber at 2 L/min for 30 minutes twice daily for 2 days) or room air. Mice were killed 48 hours after start of treatment and lung injury was assessed by analysis of bronchoalveolar lavage fluid (BALF). BALF damage markers used were lactate dehydrogenase (LDH, as units/L), protein concentration (as mg/dl), and percent of polymorphonuclear leukocytes (PMN). MnTBAP treatment provided partial protection against paraquat-induced lung injury (see Figure 30).

All documents cited above are hereby incorporated in their entirety by reference.

One skilled in the art will appreciate from a reading of this disclosure that various changes in form and detail can be made without departing from the true scope of the invention.

## Claims

**1.** Use of a mimetic of superoxide dismutase of formula

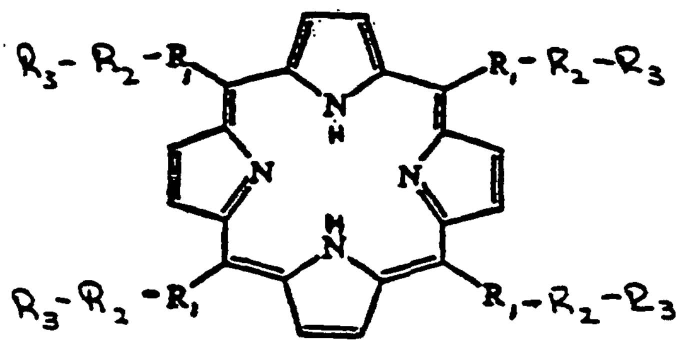

or a pharmaceutically acceptable salt thereof, wherein:

$R_1$ is a bond,

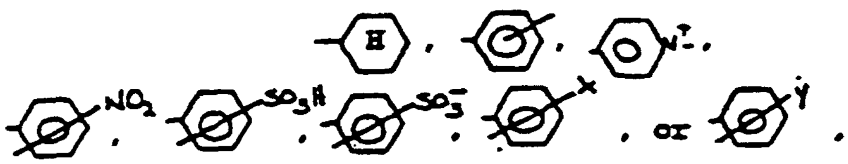

wherein X is a halogen and Y is an alkyl group and wherein

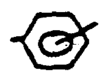

indicates bonding to $R_2$ at any position and

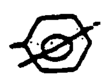

indicates bonding to $R_2$ and the substituent at any position; and

$R_2$ is a bond, $-(CY'_2)_n^-$, $-(CY'_2-CY'=CY')_n^-$, $-(CY'_2-CY'_2-CH=CH)_n^-$, $-(CY'=CY')_n^-$, or

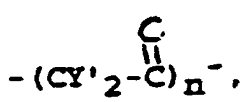

wherein Y' is hydrogen or an alkyl group and wherein n is 1 to 8; and

$R_3$ is $-Y''$, $-OH$, $-NH_2$, $-N^+(Y'')_3$, $-COOH$, $-COO-$, $-SO_3H$, $-SO_3-$, $-CH_2-PO_3H_2$ or $-CH_2-PO_3H-$, wherein Y'' is an alkyl group,

complexed with manganese;

in the preparation of a medicament for protecting cells from superoxide radical-induced toxicity in any one or more of the following applications

1. the protection against ischemic reperfusion injuries associated with myocardial infarction, stroke, acute head trauma, organ reperfusion following transplantation, bowel ischemia, pulmonary infarction, surgical occlusion of blood flow, or soft tissue injury;
2. the protection against skeletal reperfusion injuries;
3. the protection against damage to skin and eyes from sunlight;
4. the protection against damage to eyes from glaucoma or macular degeneration;
5. the protection against diseases of the bone;
6. the protection against connective tissue disorders associated with defects in collagen synthesis or degradation;
7. the treatment of diseases of the central nervous system;
8. the treatment of diseases of the musculature;
9. the treatment of neurologic disorders;
10. the treatment of arthritis, systemic hypertension, atherosclerosis, edema, septic shock, pulmonary hypertension, impotence, infertility, endometriosis, premature uterine contractions, memory disorders, microbial infections, and/or gout;
11. the treatment of inflammations; and
12. the treatment of chronic sinusitis, and/or autoimmune diseases.

**2.** Use according to claim 1 wherein said cells are mammalian cells.

**3.** Use according to claim 1 or claim 2 for the treatment of AIDS dementia; stroke; amyotrophic lateral sclerosis (ALS); Parkinson's diesease; Huntington's disease; diaphramic diseases, including:

respiratory fatigue in emphasemia, bronchitis, and cystic fibrosis; cardiac fatigue of congestive heart failure; muscle weakness syndromes associated with myopathies, ALS, or multiple sclerosis; Alzheimer's; asthma; ARDS; pneumonia, or rheumatoid arthntis.

**4.** A method of protecting plant cells from superoxide radical-induced toxicity comprising contacting said cells with a non-toxic amount, sufficient to effect said protection, of a mimetic of superoxide dismutase of formula

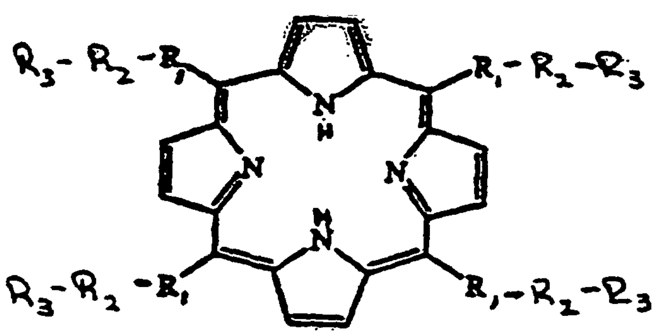

or a pharmaceutically acceptable salt thereof, wherein:

$R_1$ is a bond,

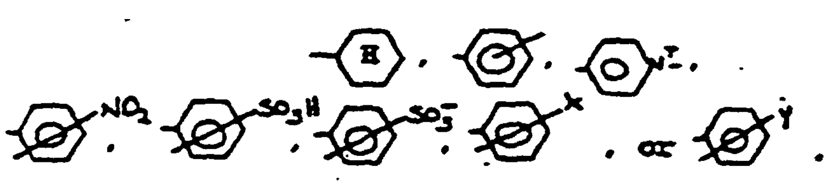

wherein X is a halogen and Y is an alkyl group and wherein

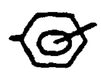

indicates bonding to $R_2$ at any position and

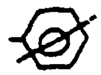

indicates bonding to $R_2$ and the substituent at any position; and
$R_2$ is a bond, $-(CY'_2)_n^-$, $-(CY'_2-CY'=CY')_n^-$, $-(CY'_2-CY'_2-CH=CH)_n^-$, $-(CY'=CY')_n^-$, or

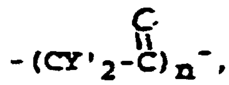

wherein Y' is hydrogen or an alkyl group and wherein n is 1 to 8; and
$R_3$ is -Y", -OH, $-NH_2$, $-N^+(Y")_3$, -COOH, -COO-, $-SO_3H$, $-SO_3-$, $-CH_2-PO_3H_2$ or $-CH_2-PO_3H-$, wherein Y" is an alkyl group,

complexed with manganese.

**5.** Use of a mimetic of superoxide dismutase of formula

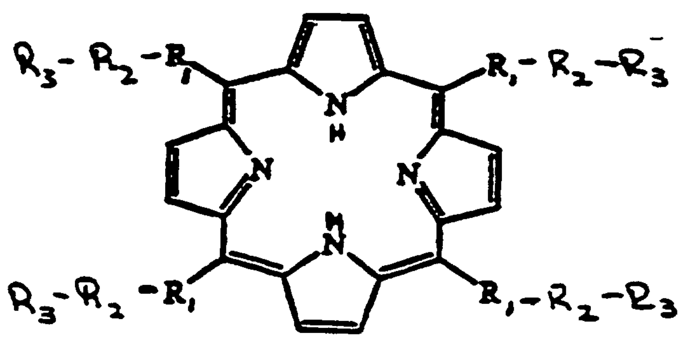

or a pharmaceutically acceptable salt thereof, wherein:

$R_1$ is a bond,

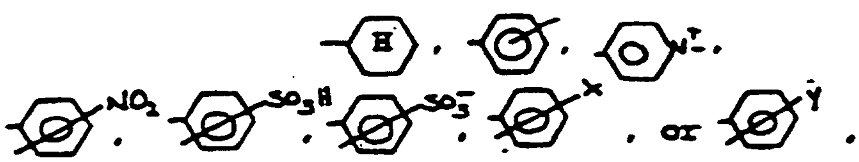

wherein X is a halogen and Y is an alkyl group and wherein

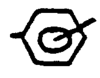

indicates bonding to $R_2$ at any position and

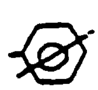

indicates bonding to $R_2$ and the substituent at any position; and
$R_2$ is a bond, $-(CY'_2)_n-$, $-(CY'_2-CY'=CY')_n-$, $-(CY'_2-CY'_2-CH=CH)_n-$, $-(CY'=CY')_n-$, or

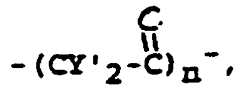

wherein Y' is hydrogen or an alkyl group and wherein n is 1 to 8; and
$R_3$ is -Y'', -OH, $-NH_2$, $-N^+(Y''')_3$, -COOH, -COO-, $-SO_3H$, $-SO_3-$, $-CH_2-PO_3H_2$ or $-CH_2-PO_3H-$, wherein Y'' is an alkyl group,

complexed with manganese;
in the preparation of a medicament for inhibiting damage due to oxidation of a substance with the subsequent formulation of $O_2^-$ in any one or more of the applications defined in claim 1.

**6.** Use of a mimetic of superoxide dismutase as defined in claim 1 to increase the storage viability of transplanted hearts, kidneys, skin and other organs and tissues.

**Patentansprüche**

**1.** Verwendung eines Nachahmers von Superoxiddismutase mit der Formel

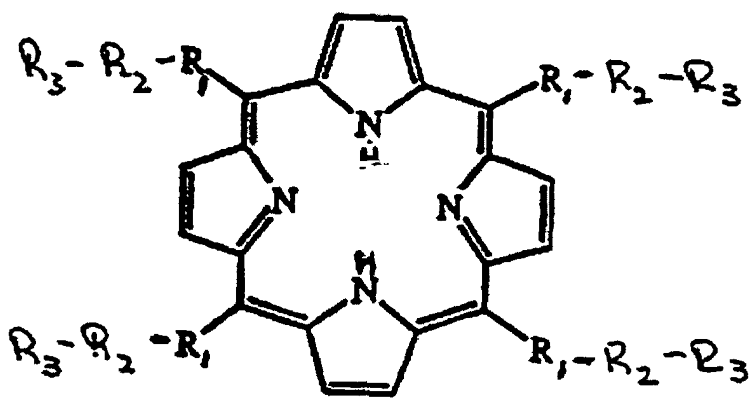

oder eines pharmazeutisch verträglichen Salzes davon, worin
$R_1$ eine Bindung,

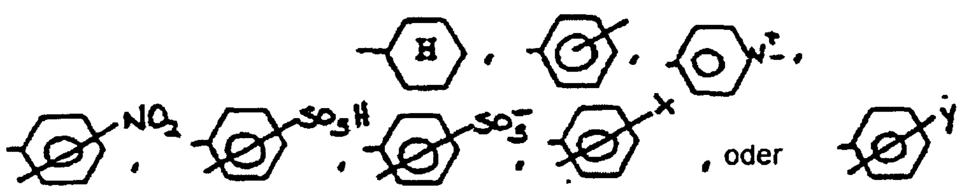

, , , , , , , oder

ist, wobei X ein Halogen ist und Y eine Alkylgruppe ist und wobei

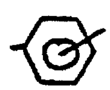

eine Bindung an $R_2$ an irgendeiner Position bezeichnet und

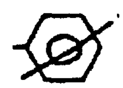

eine Bindung an $R_2$ und den Substituenten an irgendeiner Position bezeichnet und
$R_2$ eine Bindung, $-(CY'_2)_n^-$, $-(CY'_2-CY'=CY')_n^-$, $-(CY'_2-CY'_2-CH=CH)_n^-$, $-(CY'=CY')_n^-$, oder

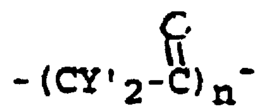

ist,
wobei Y' Wasserstoff oder eine Alkylgruppe ist und n 1 bis 8 ist und
$R_3$ -Y", -OH, $-NH_2$, $-N^+(Y")_3$, -COOH, $-COO^-$, $-SO_3H$, $-SO_3^-$, $-C-PO_3H_2$ oder $-C-PO_3H^-$ ist, wobei Y" eine Alkylgruppe ist, komplexiert mit Mangan,
bei der Herstellung eines Medikaments zum Schutz von Zellen vor durch Superoxidradikal induzierter Toxizität in einer oder mehreren der folgenden Anwendungen:

1. dem Schutz gegen ischämische Reperfusionsverletzungen, die mit Myokardinfarkt, Schlaganfall, akutem Kopftrauma, Organreperfusion nach Transplantation, Darmischämie, Lungeninfarzierung, chirurgischer Okklusion des Blutstroms oder Weichgewebeverletzung einhergehen,
2. dem Schutz gegen Skelettreperfusionsverletzungen,
3. dem Schutz gegen Schädigung von Haut und Augen durch Sonnenstrahlung,
4. dem Schutz gegen Schädigung der Augen durch Glaukom oder Makuladegeneration,
5. dem Schutz gegen Knochenerkrankung,
6. dem Schutz gegen Bindegewebsstörungen, die mit Störungen der Kollagensynthese oder Degradation einhergehen,
7. der Behandlung von Erkrankungen des zentralen Nervensystems,
8. der Behandlung von Erkrankungen der Muskulatur,
9. der Behandlung von neurologischen Störungen,
10. der Behandlung von Arthritis, systemischem Bluthochdruck, Arteriosklerose, Ödem, septischem Schock, pulmonalem Hochdruck, Impotenz, Unfruchtbarkeit, Endometriose, vorzeitigen Gebärmutterkontraktionen, Gedächtnisstörungen, Mikrobeninfektionen und/oder Gicht,
11. der Behandlung von Entzündungen und
12. der Behandlung von chronischer Sinusitis und/oder Autoimmunerkrankungen.

**2.** Verwendung nach Anspruch 1, wobei die Zellen Säugerzellen sind.

**3.** Verwendung nach Anspruch 1 oder Anspruch 2 für die Behandlung von AIDS, Demenz, Schlaganfall, amyotropher Lateralsklerose (ALS), Parkinson'scher Krankheit, Huntington'scher Krankheit, Zwerchfellerkrankungen, einschließlich Atemermüdung bei Emphysem, Bronchitis und zystischer Fibrose, Herzermüdung bei kongestiver Herzinsuffizienz, Muskelschwächesyndromen, die mit Myopathien einhergehen, ALS oder multipler Sklerose, Alzheimer'sche Krankheit, Asthma, ARDS, Pneumonie oder rheumatoider Arthritis.

**4.** Verfahren zum Schutz von Pflanzenzellen vor durch Superoxidradikal induzierter Toxizität, bei dem man die Zellen mit einer nicht toxischen Menge, die ausreicht, um den Schutz zu bewirken, eines Nachahmers von Superoxiddismutase in Kontakt bringt mit der Formel

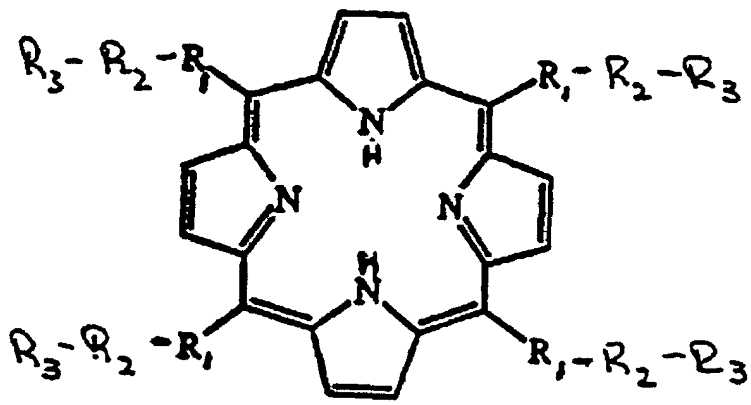

oder eines pharmazeutisch verträglichen Salzes davon,
worin
$R_1$ eine Bindung,

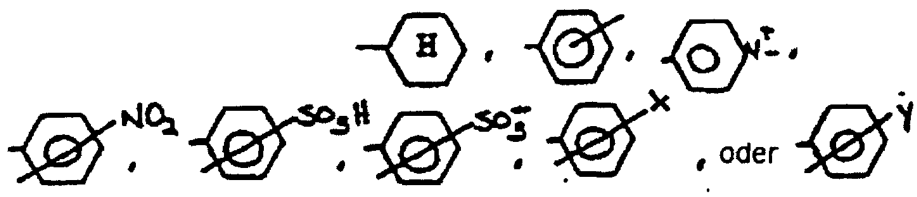

, , , , , , , oder

ist, wobei X ein Halogen ist und Y eine Alkylgruppe ist und wobei

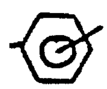

eine Bindung an $R_2$ an irgendeiner Position bezeichnet und

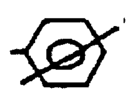

eine Bindung an $R_2$ und den Substituenten an irgendeiner Position bezeichnet und
$R_2$ eine eindung, $-(CY'_2)_n^-$, $-(CY'_2-CY'=CY')_n^-$, $-(CY'_2-CY'_2-CH=CH)_n^-$, $-(CY'=CY')_n^-$, oder

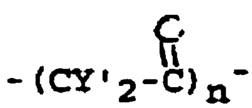

ist,
wobei Y' Wasserstoff oder eine Alkylgruppe ist und n 1 bis 8 ist und
$R_3$ -Y", -OH, $-NH_2$, $-N^+(Y")_3$, -COOH, $-COO^-$, $-SO_3H$, $-SO_3^-$, $-C-PO_3H_2$ oder $-C-PO_3H^-$ ist, wobei Y" eine Alkylgruppe ist, komplexiert mit Mangan.

**5.** Verwendung eines Nachahmers von Superoxiddismutase mit der Formel

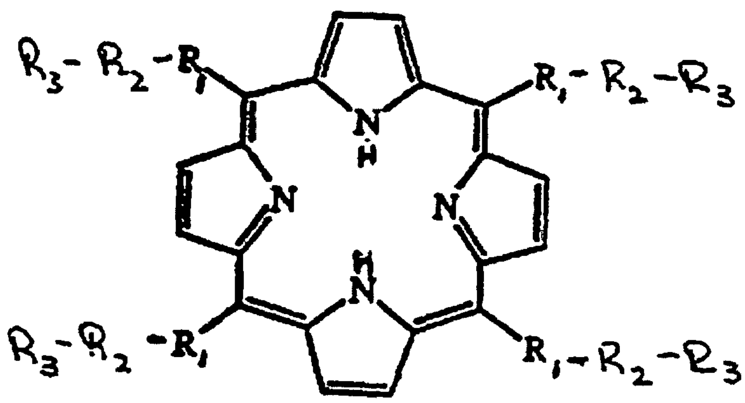

oder eines pharmazeutisch verträglichen Salzes davon,
worin

$R_1$ eine Bindung,

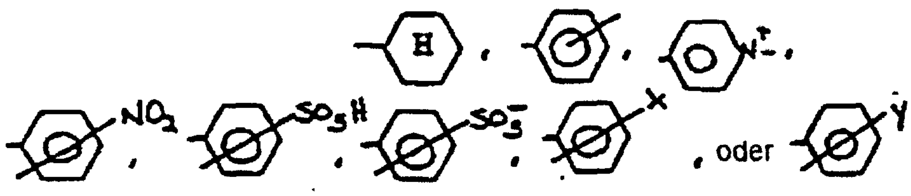

ist, wobei X ein Halogen ist und Y eine Alkylgruppe ist und wobei

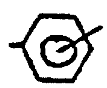

eine Bindung an $R_2$ an irgendeiner Position bezeichnet und

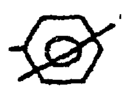

eine Bindung an $R_2$ und den Substituenten an irgendeiner Position bezeichnet und
$R_2$ eine Bindung, $-(CY'_2)_n-$, $-(CY'_2-CY'=CY')_n-$, $-(CY'_2-CY'_2-CH=CH)_n-$, $-(CY'=CY')_n-$, oder

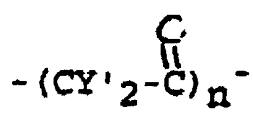

ist,
wobei Y' Wasserstoff oder eine Alkylgruppe ist und n 1 bis 8 ist und
$R_3$ -Y'', -OH, $-NH_2$, $-N^+(Y'')_3$, -COOH, $-COO^-$, $-SO_3H$, $-SO_3^-$, $-C-PO_3H_2$ oder $-C-PO_3H^-$ ist, wobei Y'' eine Alkylgruppe ist, komplexiert mit Mangan,
bei der Herstellung eines Medikaments zur Hemmung eines Schadens aufgrund von Oxidation einer Substanz mit der anschließenden Bildung von $O_2^-$ in einer oder mehreren der in Anspruch 1 definierten Anwendungen.

6. Verwendung eines Nachahmers von Superoxiddismutase, wie er in Anspruch 1 definiert ist, zur Erhöhung der

Lagerungsüberlebensfähigkeit von transplantierten Herzen, Nieren, Haut und anderen Organen und Geweben.

## Revendications

**1.** Utilisation d'un mimétique de superoxyde-dismutase de formule

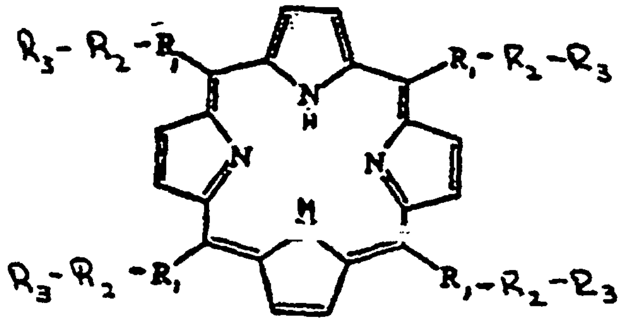

ou d'un sel pharmaceutiquement acceptable de celui-ci, dans laquelle :

$R_1$ est une liaison,

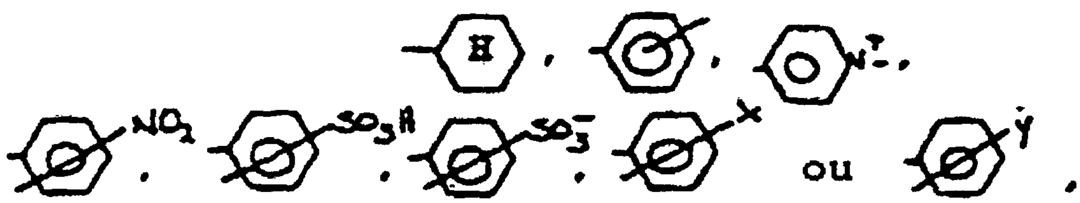

ou

où X est un halogène et Y est un groupe alkyle et où

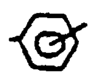

indique la liaison à $R_2$ à n'importe quelle position et

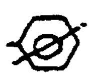

indique la liaison à $R_2$ et au substituant à n'importe quelles positions ; et

$R_2$ est une liaison, $-(CY'_2)_n-$, $-(CY'_2-CY'=CY')_n-$, $-(CY'_2-CY'_2-CH=CH)_n-$, $-(CY'=CY')_n-$ ou

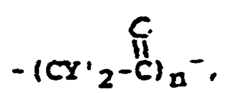

où Y' est l'hydrogène ou un groupe alkyle et où n est 1 à 8 ; et

$R_3$ est -Y"-, -OH, $-NH_2$, $-N^+(Y")_3$, -COOH, -COO-, $-SO_3H$, $-SO_3-$, $-CH_2-PO_3H_2$ ou $-CH_2-PO_3H-$, où Y" est un groupe alkyle,

complexé avec du manganèse ;

dans la préparation d'un médicament destiné à la protection de cellules contre la toxicité induite par les radicaux superoxyde dans l'une ou plusieurs quelconques des applications suivantes :

1. la protection contre des lésions ischémiques de reperfusion associées à un infarctus du myocarde, un ictus, un traumatisme crânien aigu, une reperfusion d'organe après transplantation, une ischémie intestinale, un infarctus pulmonaire, une occlusion chirurgicale du courant sanguin ou une lésion de tissus mous ;
2. la protection contre des lésions de reperfusion du squelette ;
3. la protection contre l'endommagement de la peau et des yeux par la lumière solaire ;
4. la protection contre l'endommagement des yeux par un glaucome ou une dégénérescence maculaire ;
5. la protection contre des maladies osseuses ;
6. la protection contre des affections du tissu conjonctif associées à des défauts de synthèse ou de dégradation du collagène ;
7. le traitement de maladies du système nerveux central ;
8. le traitement de maladies de la musculature ;
9. le traitement de troubles neurologiques ;
10. le traitement de l'arthrite, de l'hypertension systémique, de l'athérosclérose, de l'oedème, du choc septique, de l'hypertension pulmonaire, de l'impuissance, de la stérilité, de l'endométriose, des contractions utérines prématurées, de troubles de la mémoire, d'infections microbiennes et/ou de la goutte ;
11. le traitement d'inflammations ; et
12. le traitement de sinusite chronique et/ou de maladies auto-immunes.

**2.** Utilisation selon la revendication 1, dans laquelle lesdites cellules sont des cellules de mammifères.

**3.** Utilisation selon la revendication 1 ou la revendication 2 pour le traitement du SIDA ; de la démence ; d'un ictus ; de la sclérose latérale amyotrophique (SLA) ; de la maladie de Parkinson ; de la chorée de Huntington ; de maladies du diaphragme, comprenant :

la fatigue respiratoire dans l'emphysème, la bronchite et la fibrose kystique ; la fatigue cardiaque de l'insuffisance cardiaque ; les syndromes de faiblesse musculaire associés à la myopathie, la SLA ou la sclérose en plaques ; la maladie d'Alzheimer ; l'asthme ; le SDRA ; la pneumonie ou la polyarthrite rhumatoïde.

**4.** Procédé de protection de cellules végétales contre la toxicité induite par les radicaux superoxyde, comprenant la mise en contact desdites cellules avec une quantité non toxique, suffisante pour exercer ladite protection, d'un mimétique de superoxyde-dismutase de formule

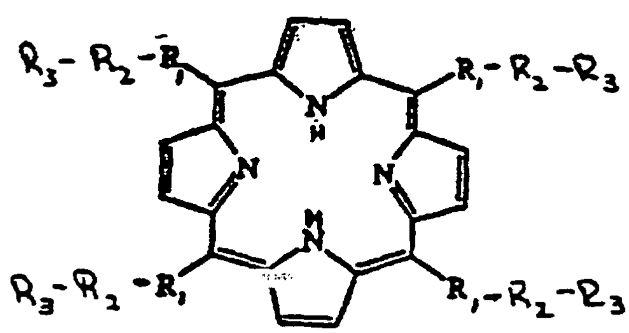

ou d'un sel pharmaceutiquement acceptable de celui-ci,
dans laquelle :

$R_1$ est une liaison,

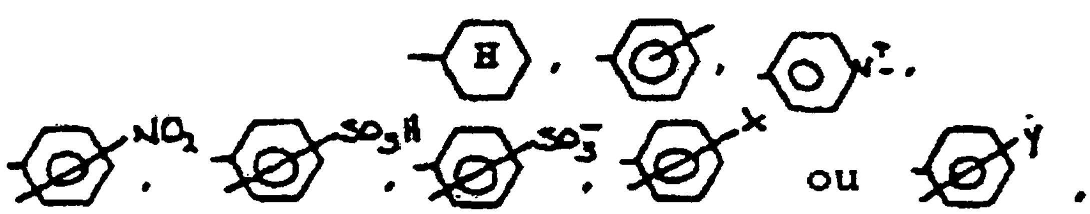

ou

où X est un halogène et Y est un groupe alkyle et où

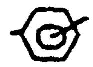

indique la liaison à $R_2$ à n'importe quelle position et

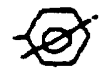

indique la liaison à $R_2$ et au substituant à n'importe quelles positions ; et
$R_2$ est une liaison, $-(CY'_2)_n-$, $-(CY'_2-CY'=CY')_n-$, $-(CY'_2-CY'_2-CH=CH)_n-$, $-(CY'=CY')_n-$ ou

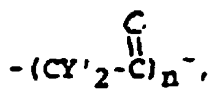

où Y' est l'hydrogène ou un groupe alkyle et où n est 1 à 8 ; et
$R_3$ est -Y"-, -OH, $-NH_2$, $-N^+(Y")_3$, -COOH, -COO-, $-SO_3H$, $-SO_3-$, $-CH_2-PO_3H_2$ ou $-CH_2-PO_3H-$, où Y" est un groupe alkyle,

complexé avec du manganèse.

**5.** Utilisation d'un mimétique de superoxyde-dismutase de formule

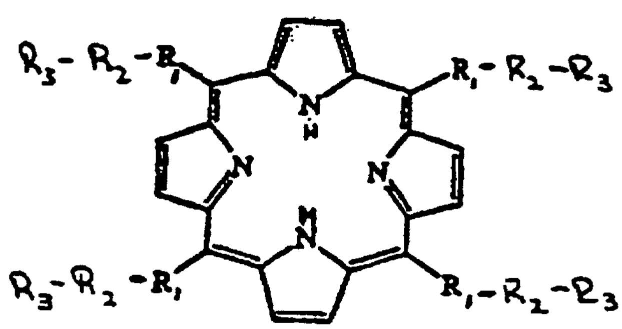

ou d'un sel pharmaceutiquement acceptable de celui-ci, dans laquelle :

$R_1$ est une liaison,

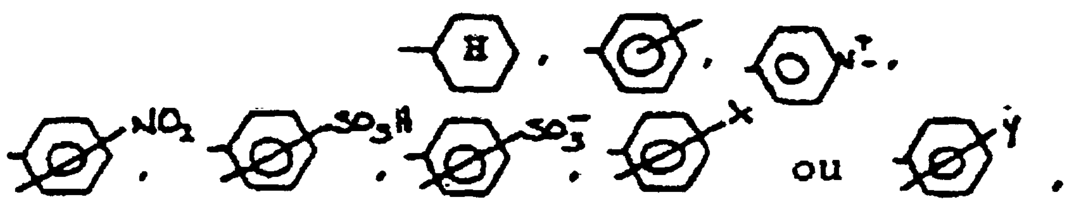

où X est un halogène et Y est un groupe alkyle et où

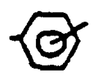

indique la liaison à $R_2$ à n'importe quelle position et

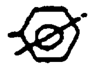

indique la liaison à $R_2$ et au substituant à n'importe quelles positions ; et

$R_2$ est une liaison, $-(CY'_2)_n-$, $-(CY'_2-CY'=CY')_n-$, $-(CY'_2-CY'_2-CH=CH)_n-$, $-(CY'=CY')_n-$ ou

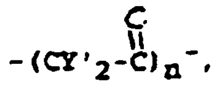

où Y' est l'hydrogène ou un groupe alkyle et où n est 1 à 8 ; et
$R_3$ est -Y"-, -OH, $-NH_2$, $-N^+(Y")_3$, -COOH, -COO-, $-SO_3H$, $-SO_3-$, $-CH_2-PO_3H_2$ ou $-CH_2-PO_3H-$, où Y" est un groupe alkyle,

complexé avec du manganèse ;
dans la préparation d'un médicament destiné à inhiber l'altération due à l'oxydation d'une substance avec la formulation subséquente de $O_2^-$ dans l'une ou plusieurs quelconques des applications définies dans la revendication 1.

**6.** Utilisation d'un mimétique de superoxyde-dismutase tel que défini dans la revendication 1, pour augmenter la viabilité à la conservation de coeurs, reins, peau et autres organes et tissus transplantés.

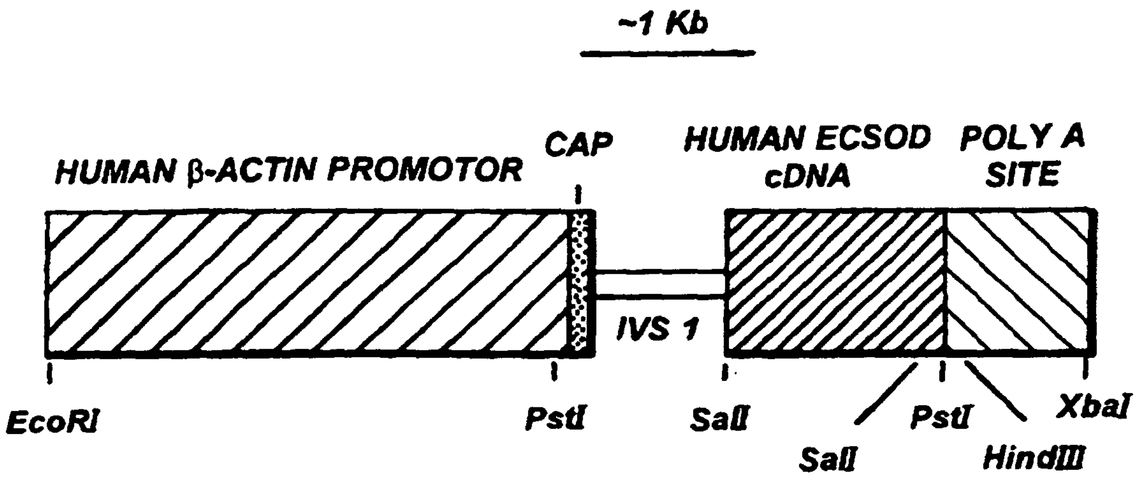
~1 Kb
HUMAN β-ACTIN PROMOTOR
CAP
HUMAN ECSOD cDNA
POLY A SITE
IVS 1
EcoRI
PstI
SalI
SalI
PstI
HindIII
XbaI

*FIG. 1*

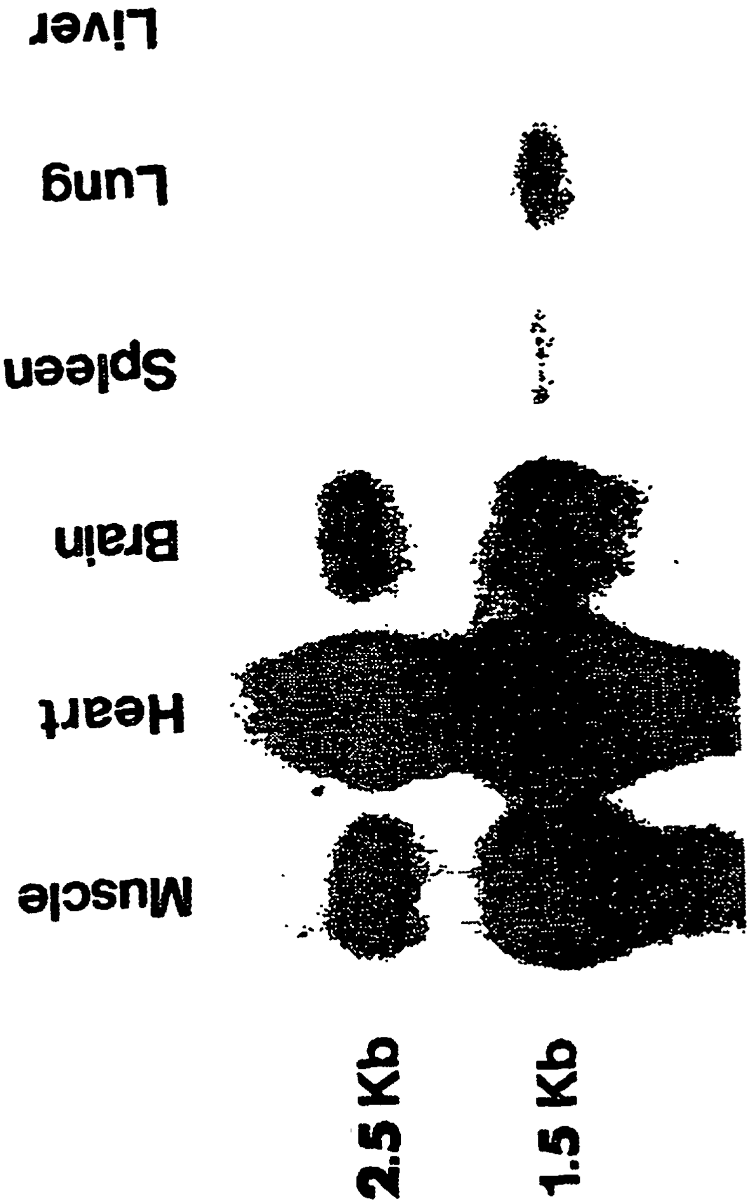


FIG. 2

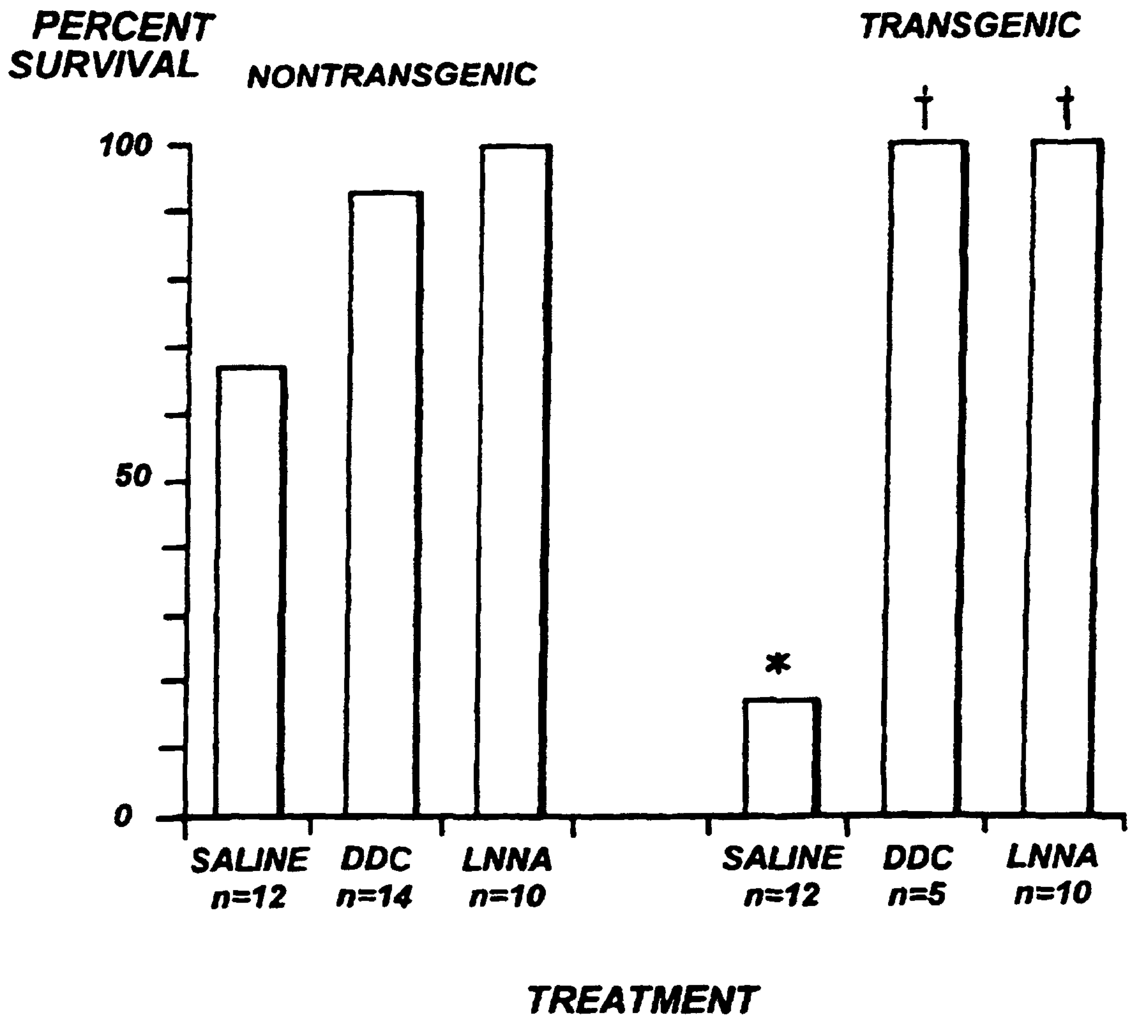
PERCENT SURVIVAL
NONTRANSGENIC
TRANSGENIC
†
†
100
50
0
*
SALINE n=12
DDC n=14
LNNA n=10
SALINE n=12
DDC n=5
LNNA n=10
TREATMENT

FIG. 3

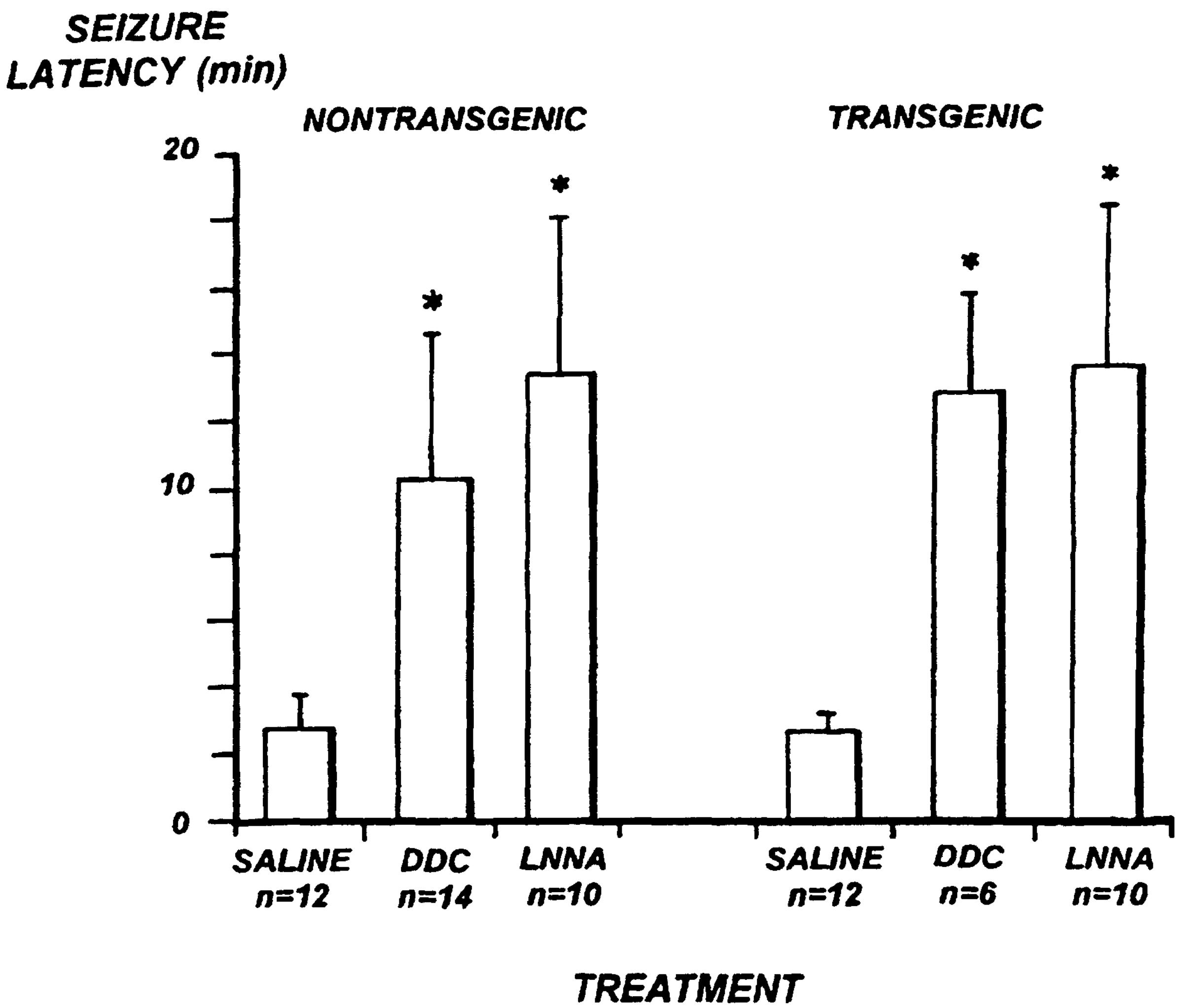
SEIZURE
LATENCY (min)
NONTRANSGENIC
TRANSGENIC
20
10
0
SALINE
n=12
DDC
n=14
LNNA
n=10
SALINE
n=12
DDC
n=6
LNNA
n=10
TREATMENT

FIG. 4

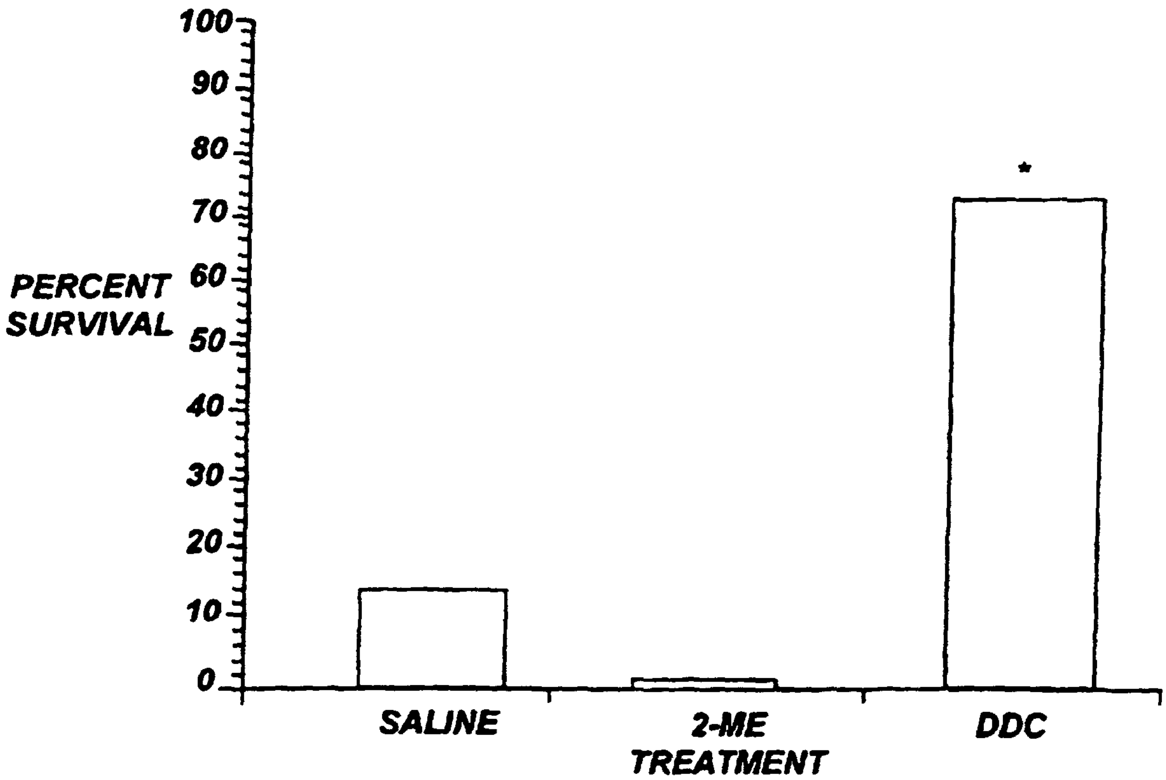
100
90
80
70
60
50
40
30
20
10
0
PERCENT
SURVIVAL
*
SALINE
2-ME
TREATMENT
DDC

FIG. 5

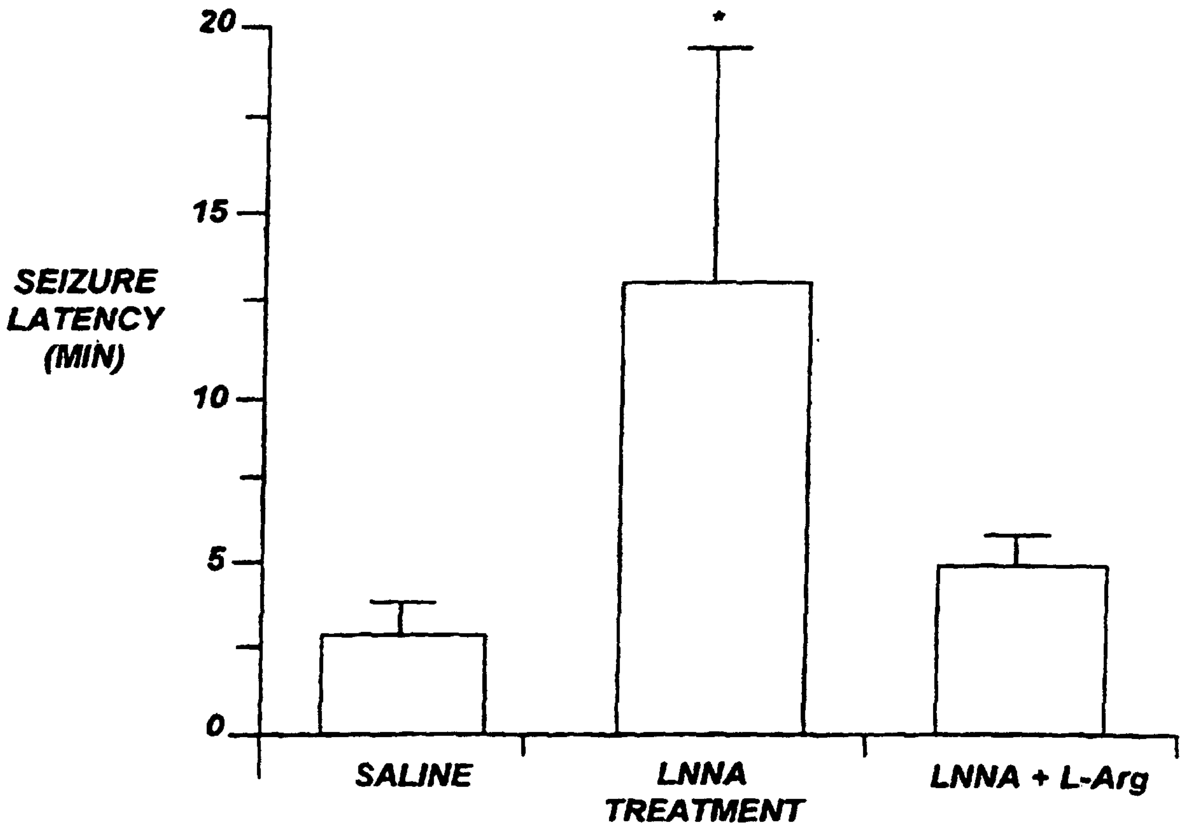
20
15
10
5
0
SEIZURE
LATENCY
(MIN)
*
SALINE
LNNA
TREATMENT
LNNA + L-Arg

FIG. 6

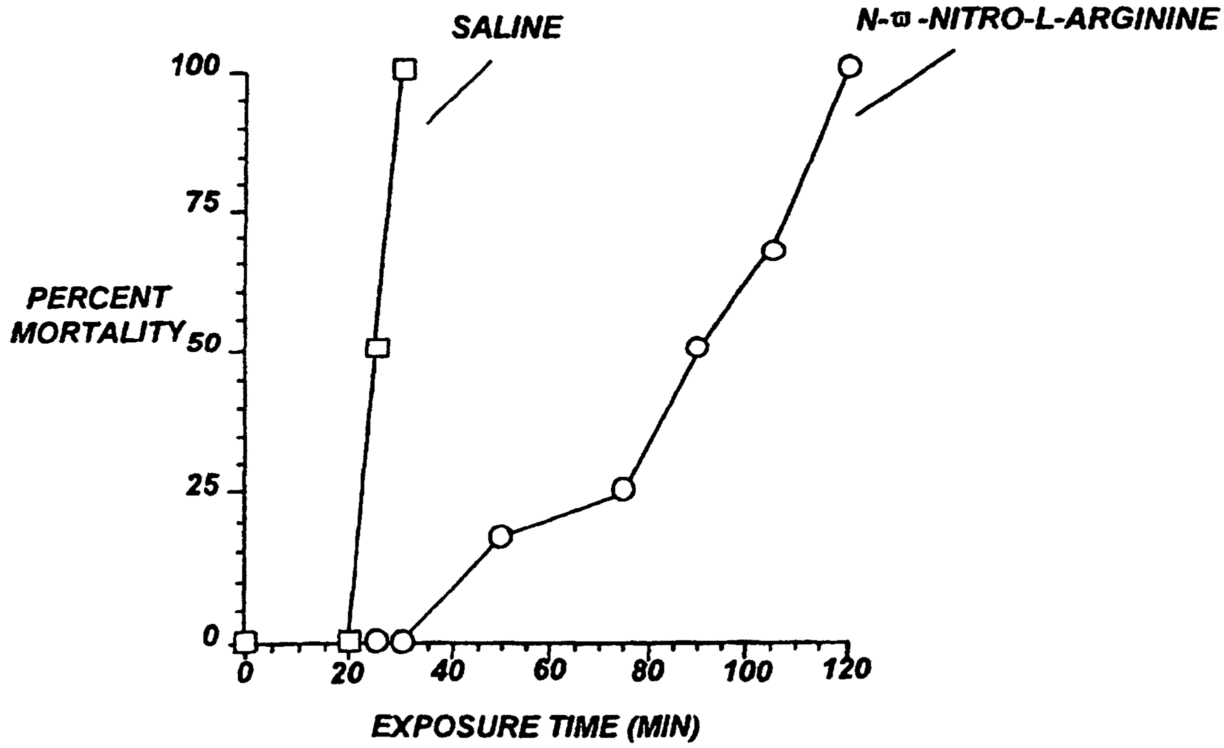
SALINE
N-ϖ-NITRO-L-ARGININE
100
75
PERCENT
MORTALITY
50
25
0
0
20
40
60
80
100
120
EXPOSURE TIME (MIN)

FIG. 7

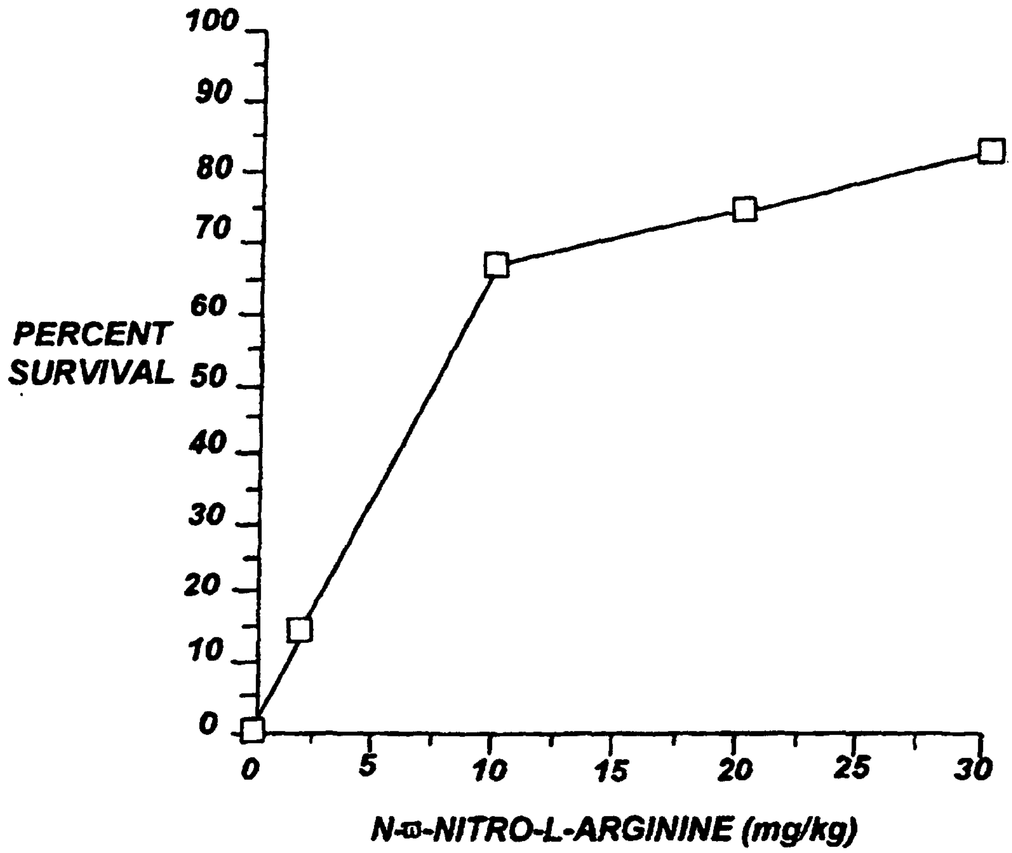
100
90
80
70
60
50
40
30
20
10
0
PERCENT SURVIVAL
0
5
10
15
20
25
30
N-ω-NITRO-L-ARGININE (mg/kg)

FIG. 8

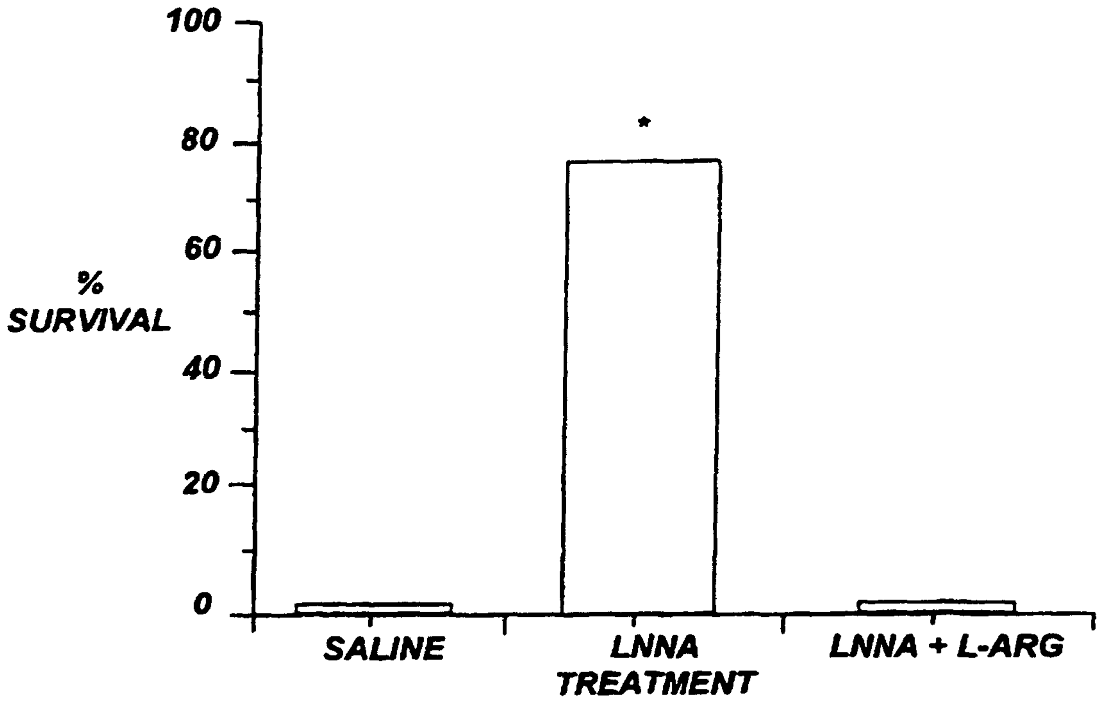


FIG. 9

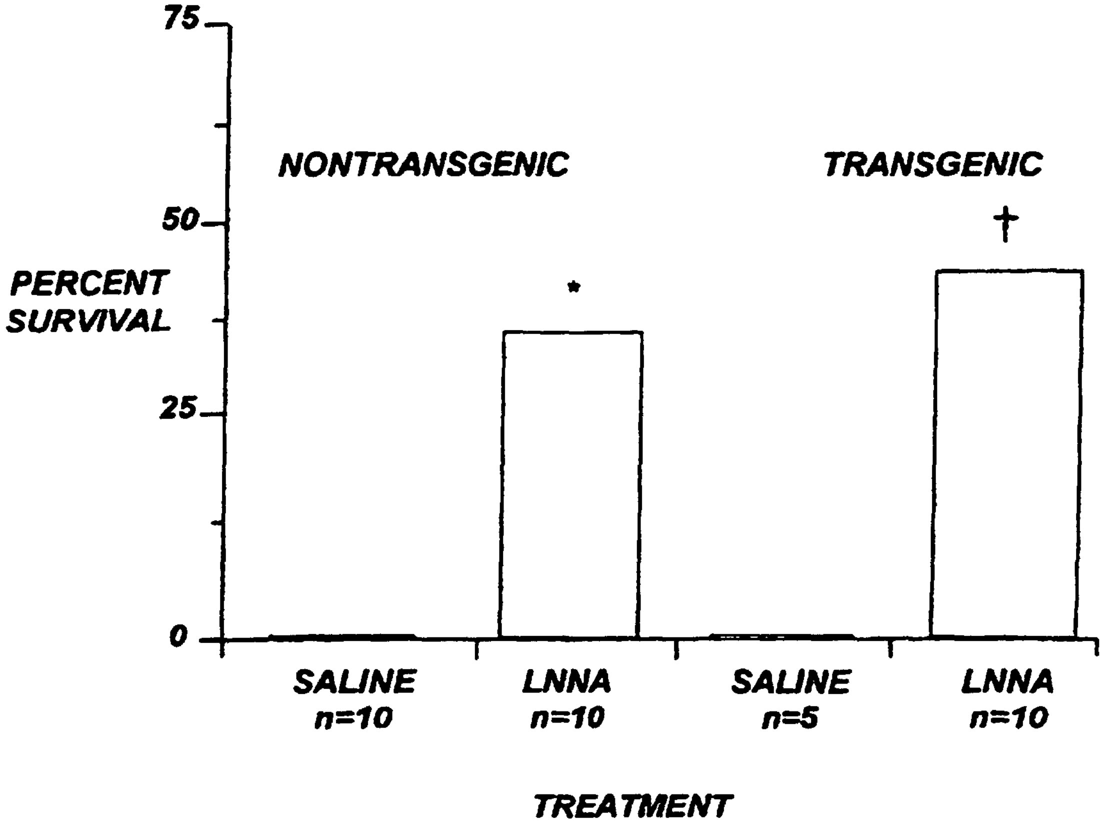
75
50
25
0
PERCENT SURVIVAL
NONTRANSGENIC
TRANSGENIC
*
†
SALINE n=10
LNNA n=10
SALINE n=5
LNNA n=10
TREATMENT

FIG. 10

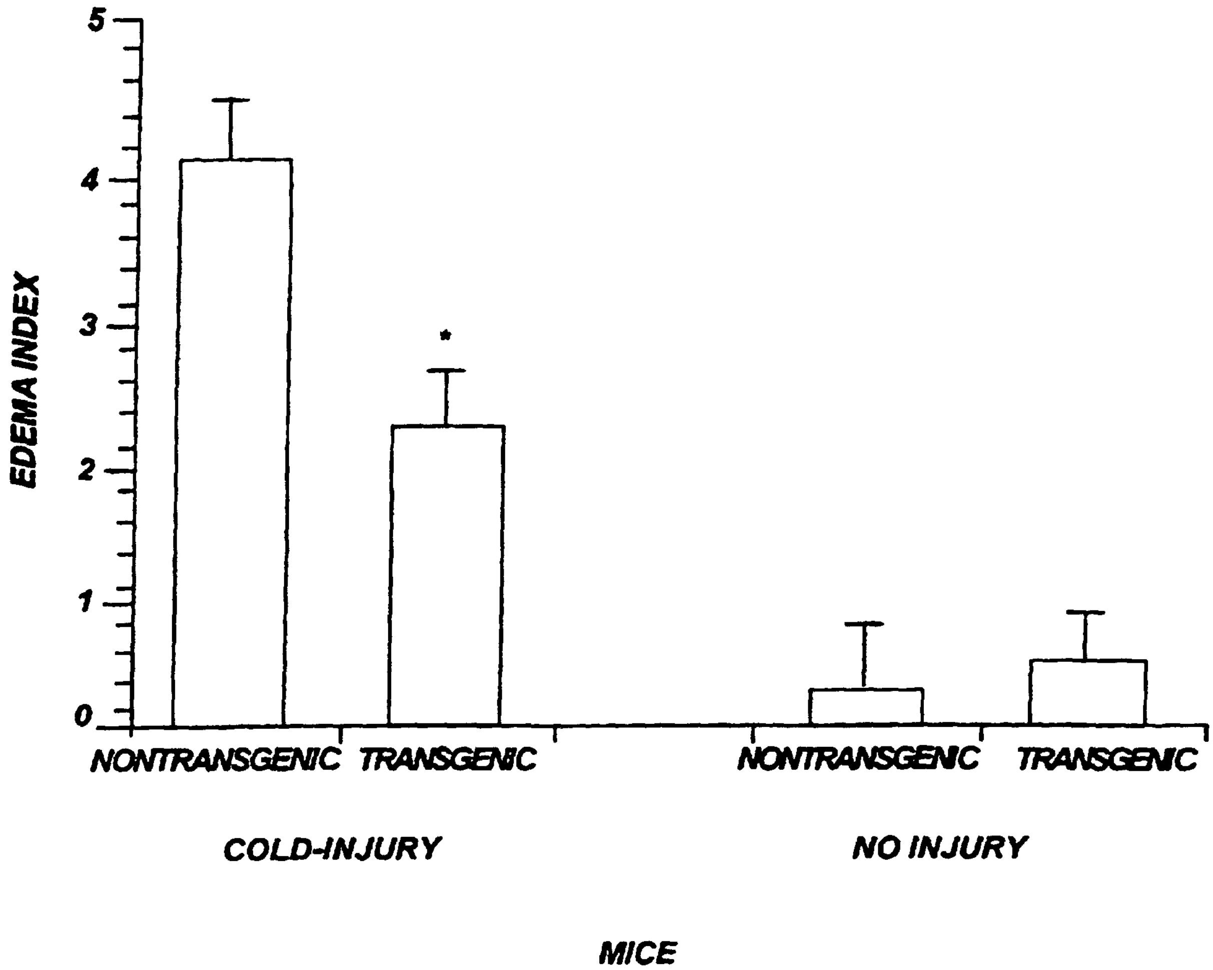
EDEMA INDEX
5
4
3
2
1
0
*
NONTRANSGENIC
TRANSGENIC
NONTRANSGENIC
TRANSGENIC
COLD-INJURY
NO INJURY
MICE

FIG. 11

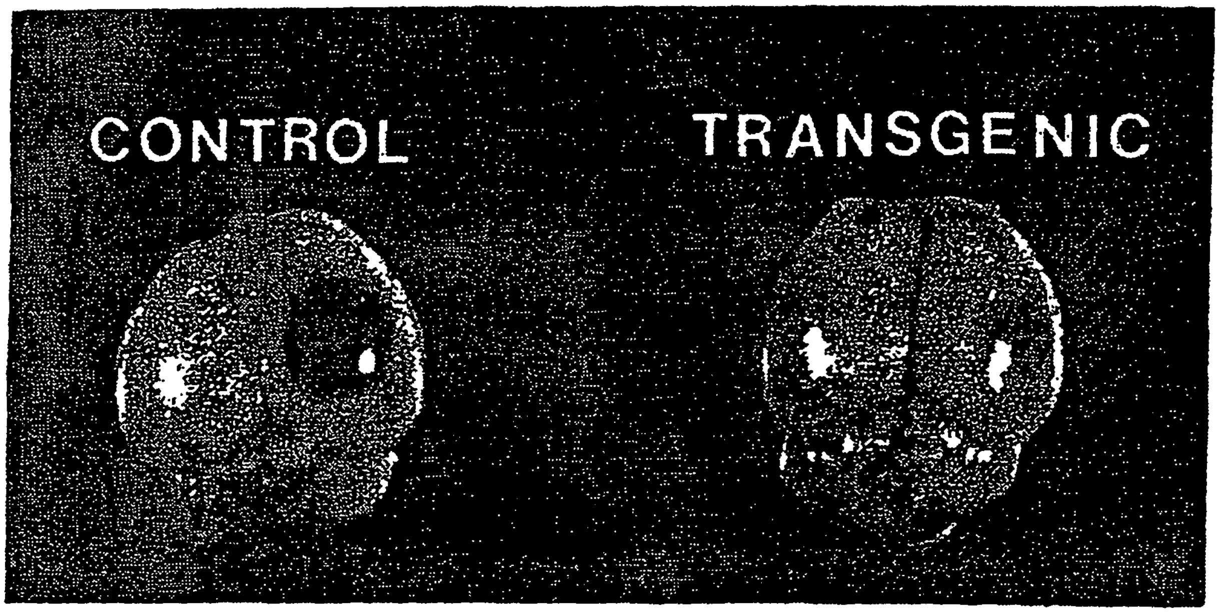


*FIG. 12*

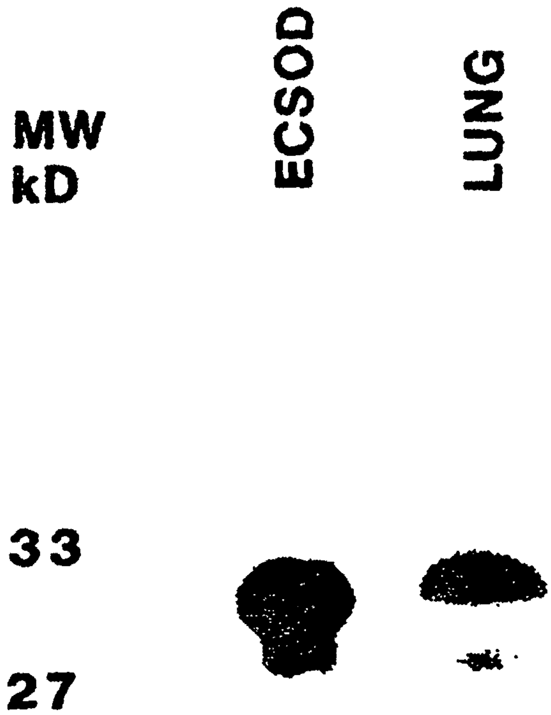


FIG. 13

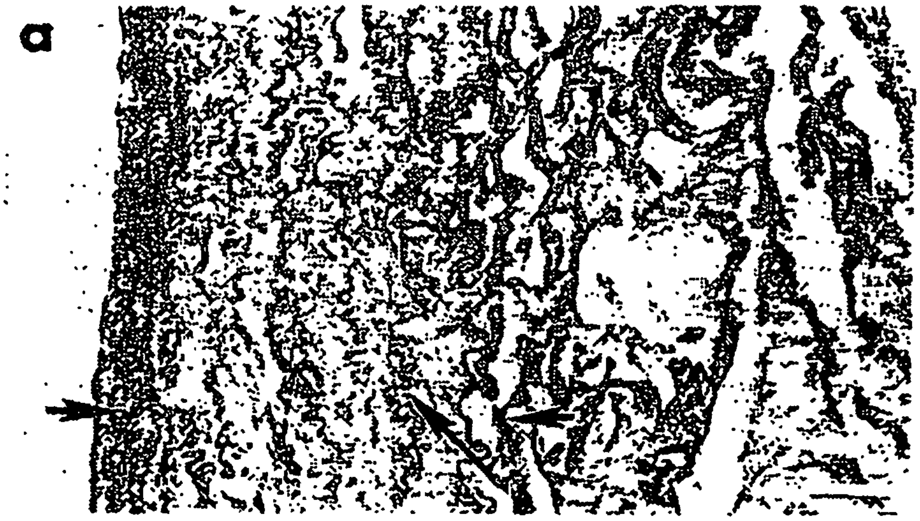


FIG. 14A

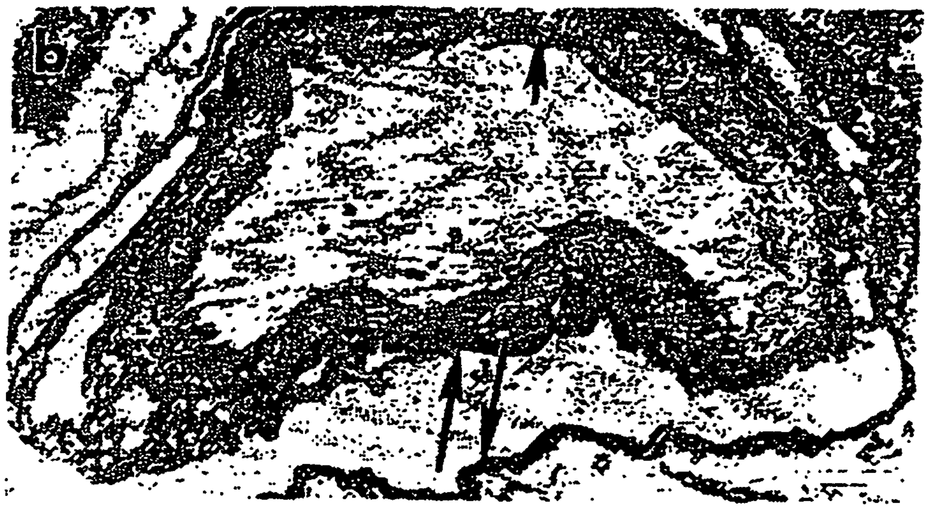


FIG. 14B

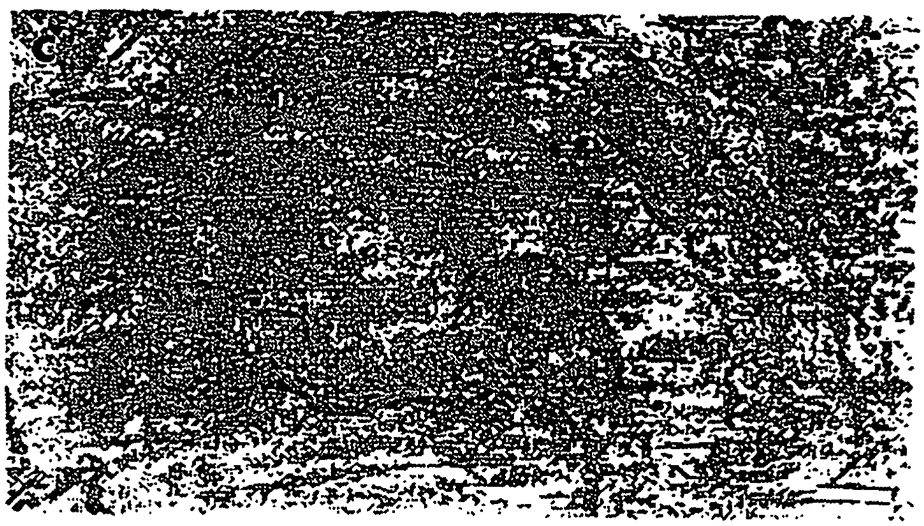


FIG. 14C

FIG. 15A

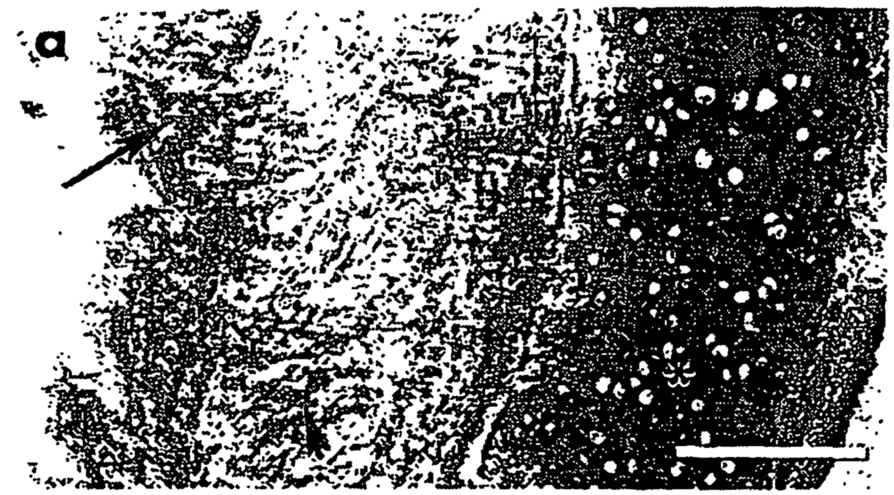


FIG. 15B

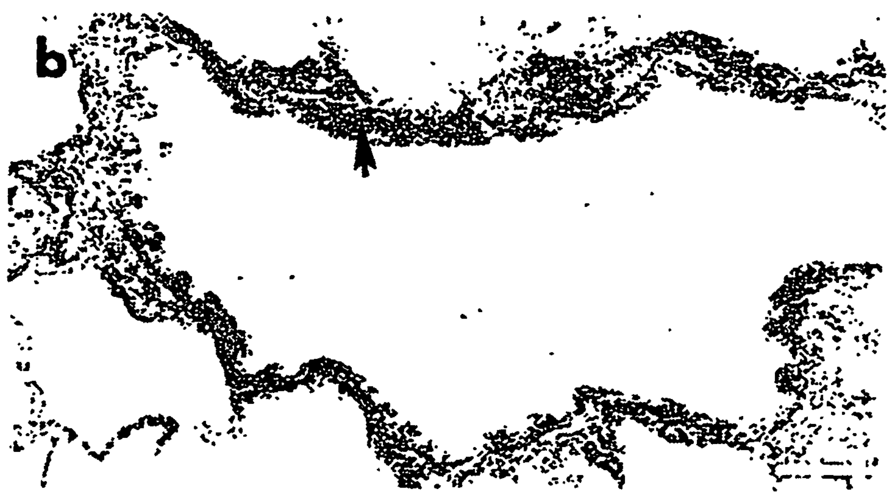


FIG. 15C

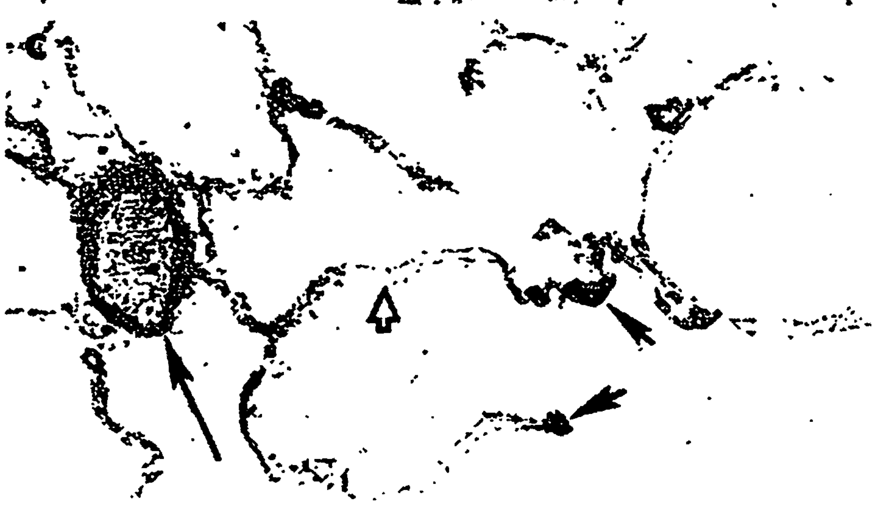

FIG. 16A

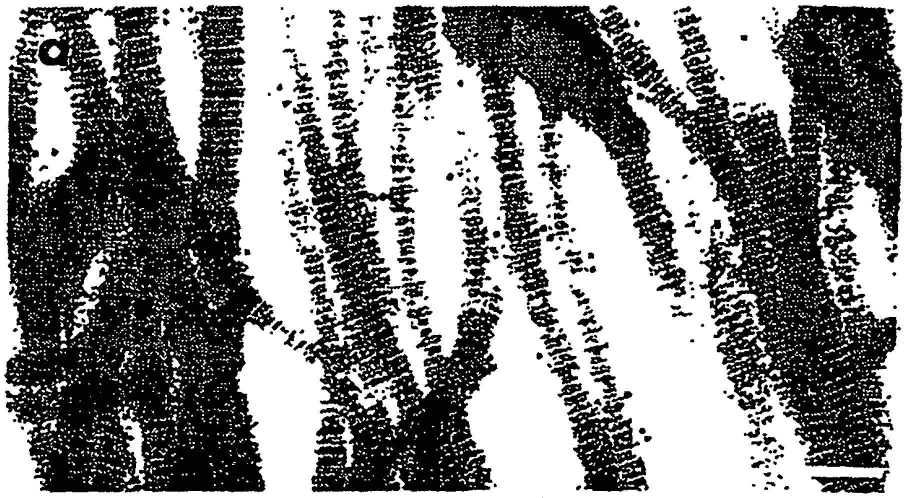


FIG. 16B

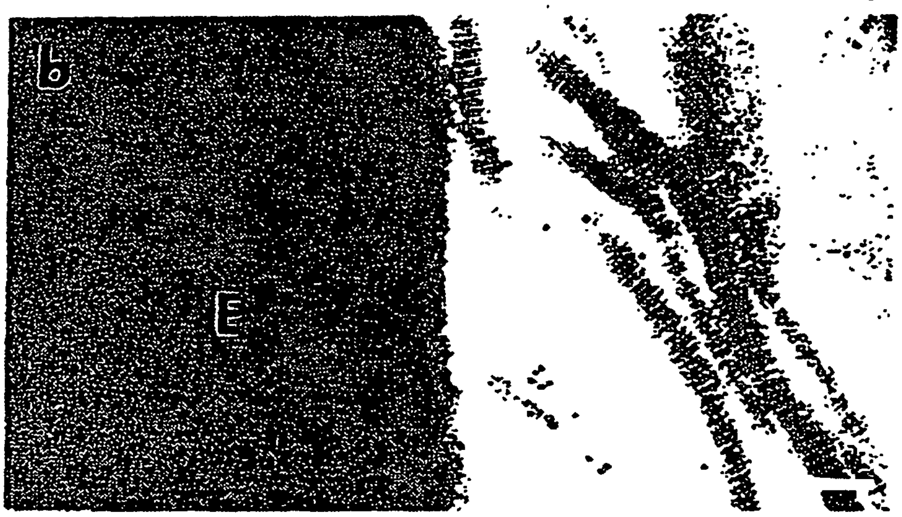


FIG. 16C

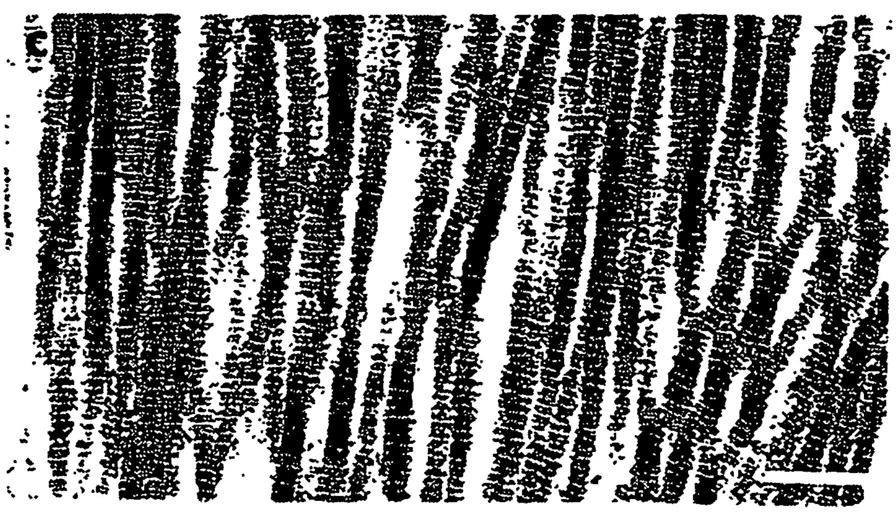

FIG. 17

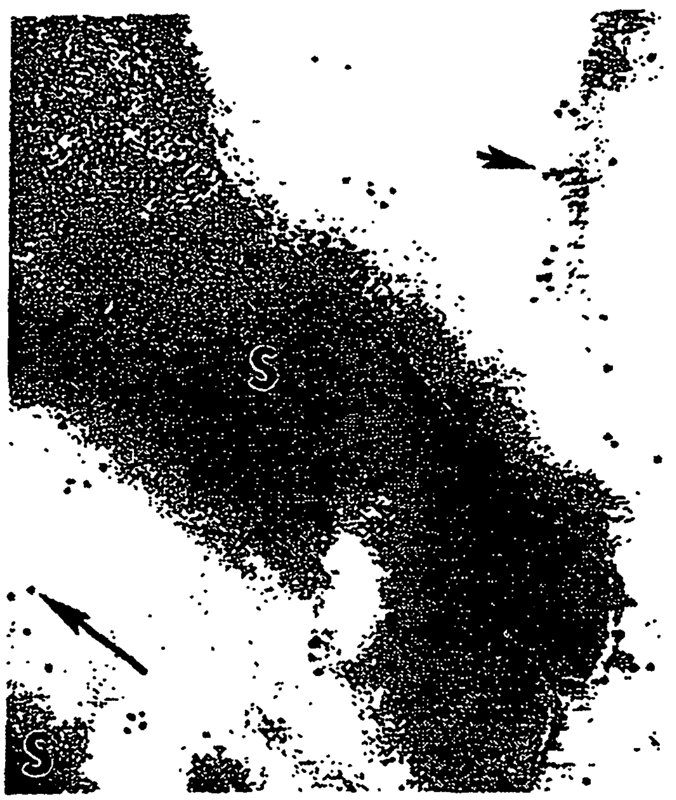

FIG. 18A

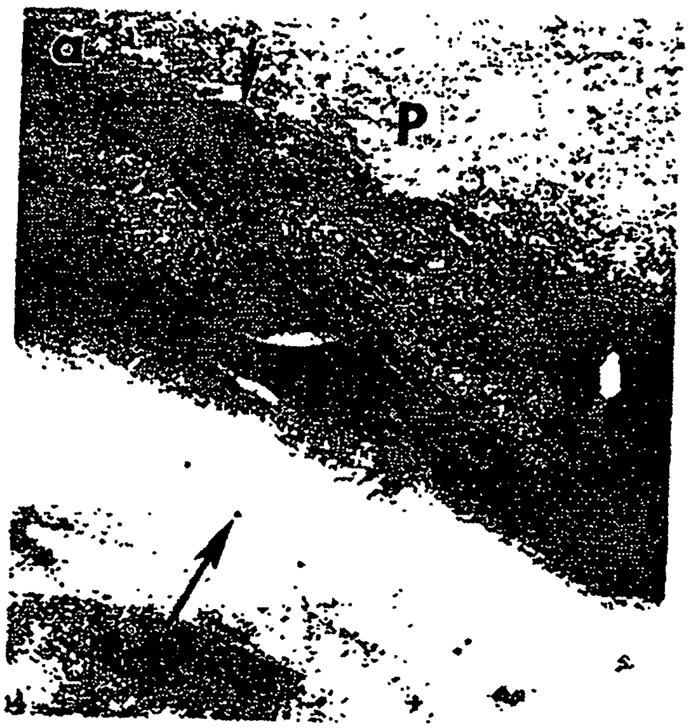


FIG. 18B

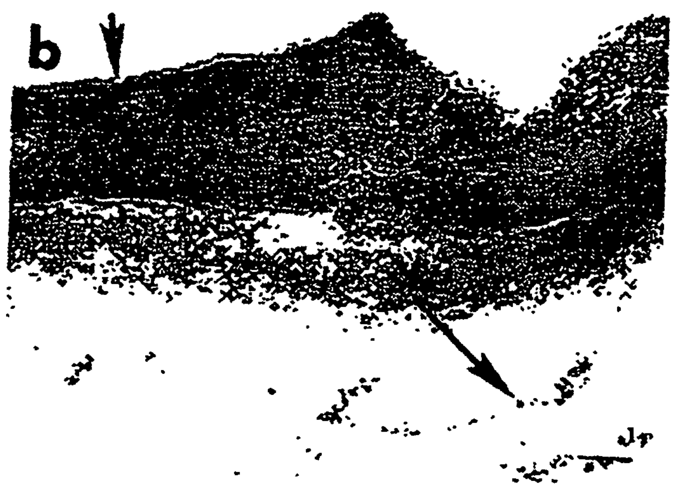

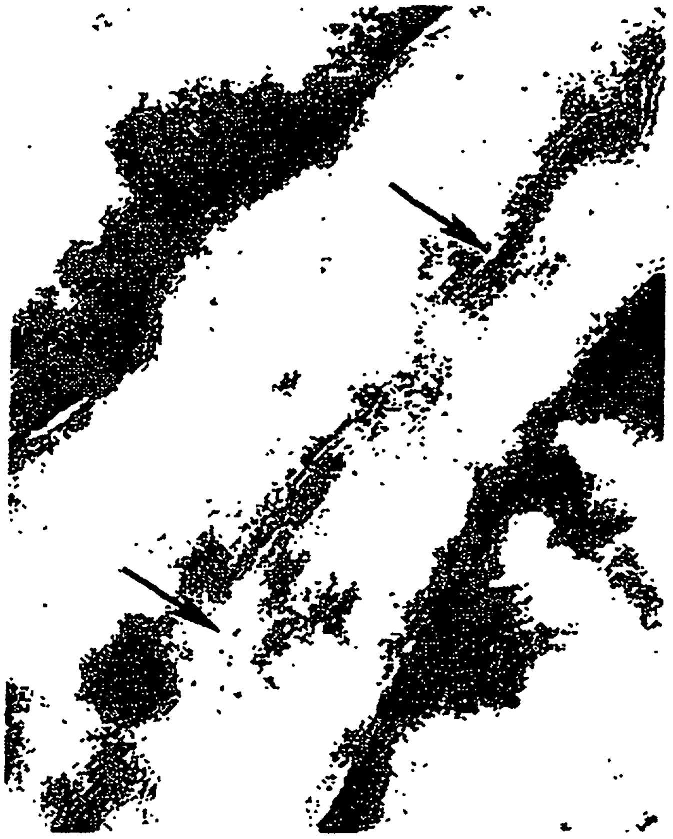

FIG. 19

## FIG. 20A

RESTRICTION MAP

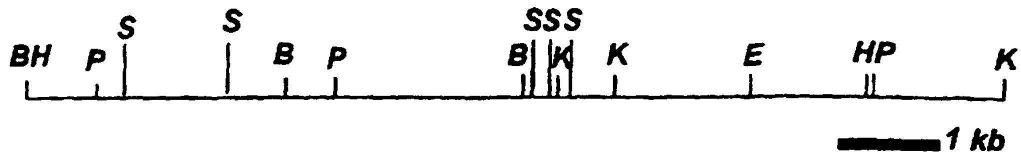


## FIG. 20B

SEQUENCING STRATEGY

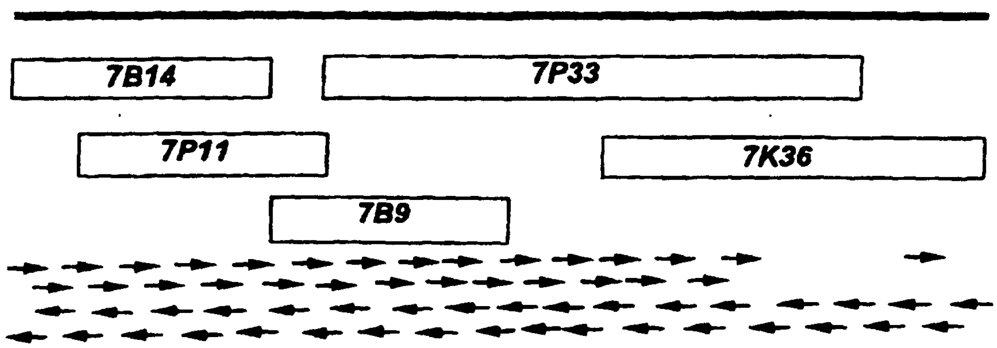


## FIG. 20C GENE STRUCTURE

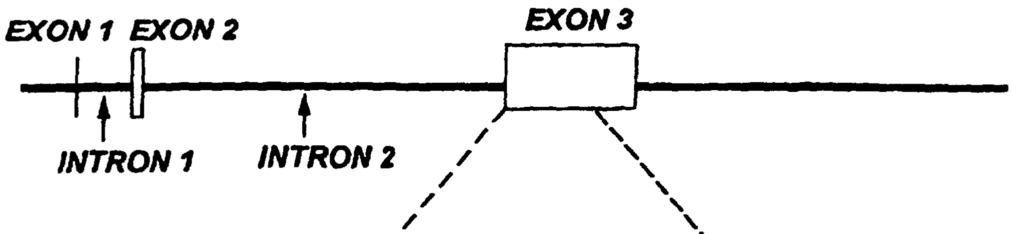


## FIG. 20D

PROTEIN STRUCTURE

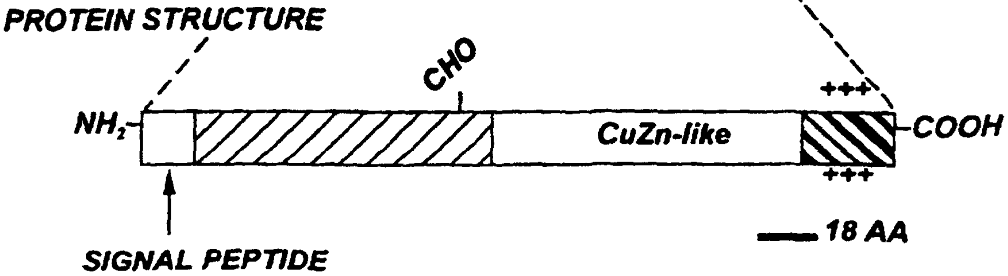

FIG. 21A

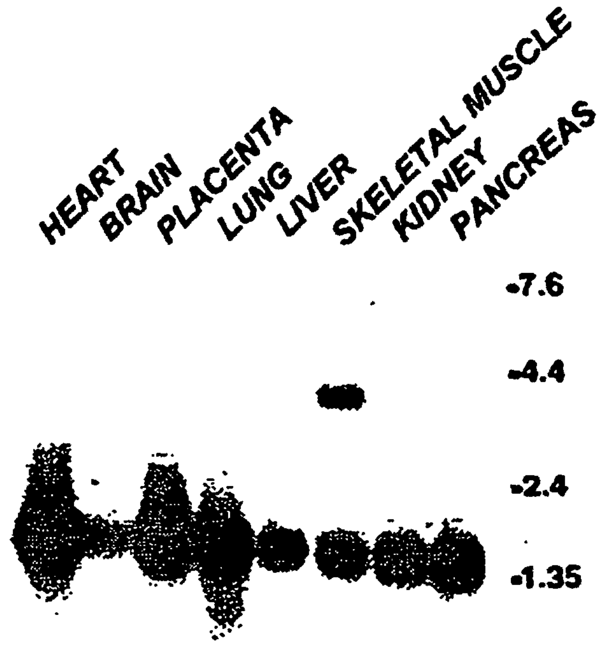


FIG. 21B

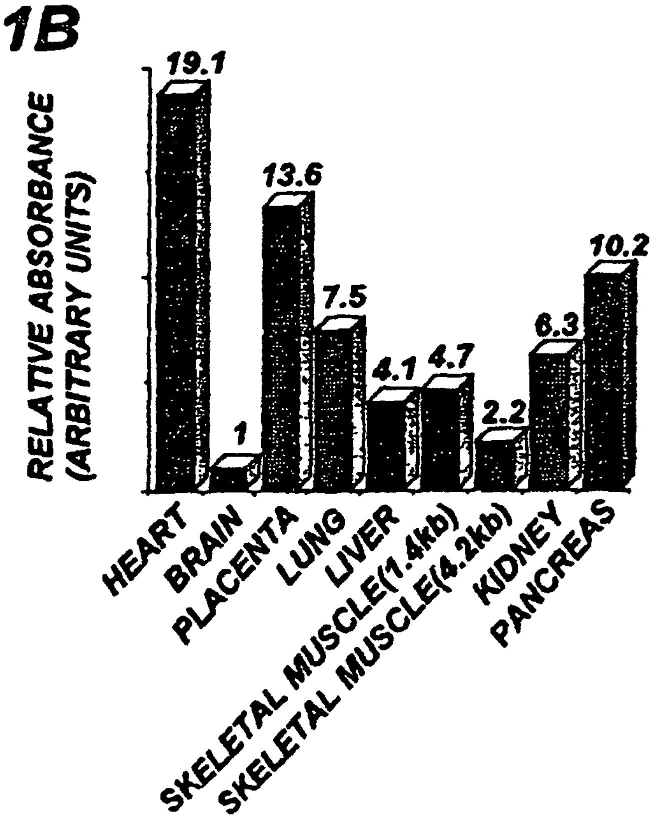

FIG. 22A

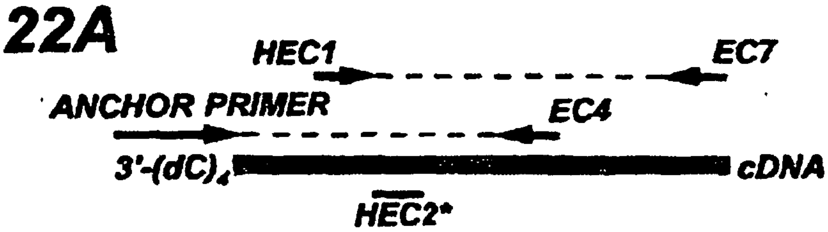


FIG. 22B

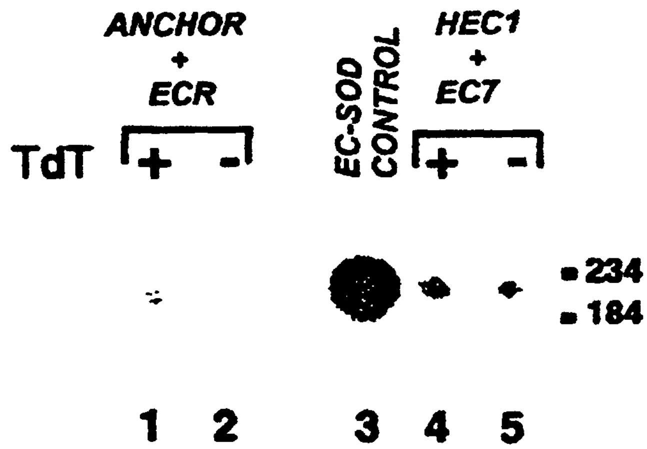

FIG. 23

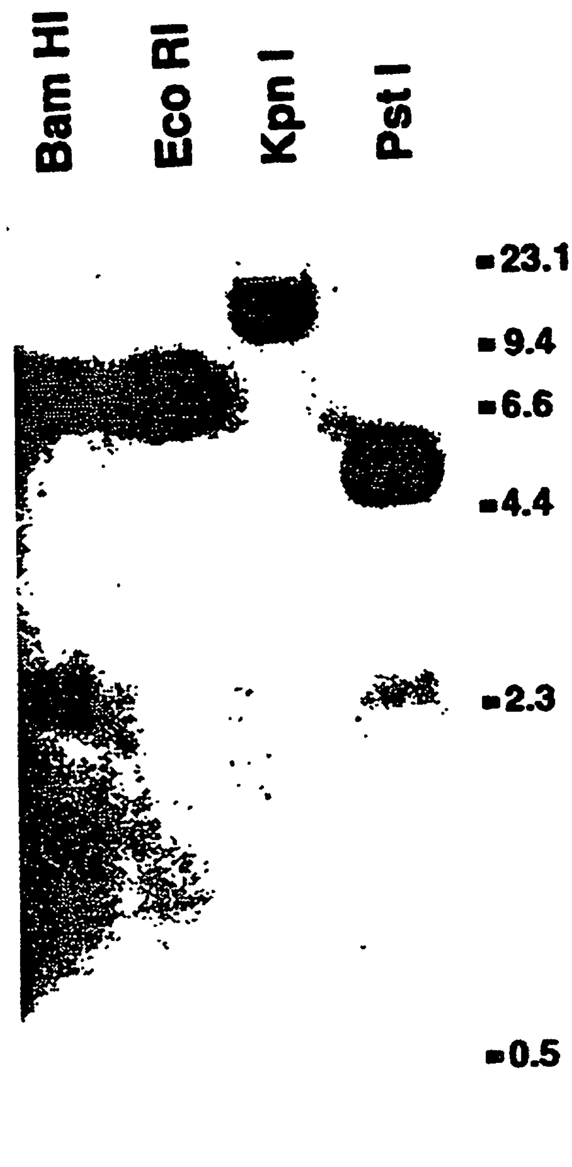

# FIG. 24

GGATCCAGAG ATTTAGATTT TTTATAAGCT TTCCTGCCAC CGAAACGGGT GTTTGGGACC 60
TCACGAGGCC CTGTTCATTC TTCGTCGCTG CGCTCCCCAC TCTGTACTGG ATGCATTTAC 120
TGACGTTGTT GTCTCCGTCC CCAGAGTATG AACCCCCAAG GTGACTCATG CAGCTGTGGG 180
TGCCCGGCAT ACAGCATGGT GACTGGAATG GATGAGCACC CAATAAACAT TTGTTGCAGG 240
AATGCAGGAG GACGGGCAGG CCAGCAAGCA GGCTGCCTGG TTTTTCCCAC ATGGGCTTTT 300
CTGGGAAAGA AGAGCTTCTA TTTTTGGAAA GGGCTGCTAT GATTGAGAAA AGTTCATGGC 360
AGCAAAAAAA GGACAGACGT CGGGAGGGAA ACACTCCTAG TTCTCCCAGA CAACACATTT 420
TTTAAAAAGA CTCCTTCATC TCTTTAATAA TAACGGTAAC GACAATGACA ATGATGATTA 480
CTTATGAGTG CGGCTAGTGC CAGCCACTGT GTTGTCACTG GGCGAGTAAT GATCTCATTG 540
GATCTTCACG GTGGGCGTGC GGGGTGGACA GCCTCACACC CCCATTTTAC AGATGATGAA 600
AAGGAGGTGC AGGGAGTGGT GCAGCTGCTT CAGGCGTACA CAGATAGGAA GTGACAAGGC 660
TGGGACTCTG CAGCCTGAGT GTGTCATCAC GACCCACCCG CTGCTCTGCT CTCATAGGTA 720
TGACAGCACA GCTCTGGAGC AAATGCCATG CACATTTGCA AGGTGCCCAT TTCCATGCAG 780
CAAAAATAAG TCAATAAGTT ATTGACTTAG AGAAAAGCAA AGGGCCTCTC AATAAAGAGG 840
TCATTGTACA CCTCTCCAAA CAGGCGATTT TCTTTCTCAT TTTTATTCCC CTGCTGTGTG 900
CTGAAGGTCA CTGGCTACAA GCCGGTGAAG TCGCGGAATG GAATCCTTGG CCCGAAAACC 960
CAAAAATGGG AGGGGCAGAG GAGGTGGGGA CAGAGCGGGA GGAGGTGGAG GCGAAGCAAT 1020
TCTACAACCC GGGGAGGTCT GGCCTGCTTT TCCTCCCTGA ACTGGCCCAA TGACTGGCTC 1080
CCTCACGCTG ACCACTCCTC TGGGCTGGCC TCCTGCACTC GCGCTAACAG CCCAGGCTCC 1140
AGGGACAGCC TGCGTTCCTG GGCTGGCTGG GTGCAGCTCT CTTTTCAGGA GAGAAAGCTC 1200
TCTTGGAGGA GCTGGAAAGG TGGGTGCTAA GTTGAGGTTC ATTTTGTTCT TCTCGGAGTG 1260
TGCTTATTGA GTCTGAAGCT GGGTTGGGGC AACGGGCCTC TTCTTGGGAA CAAATTGGAT 1320
CATCTTCTTG GGAAGGAAAT GTACTTTCCC TGGCTGCTCT GAGGGGTTAG TGGGGAGGTG 1380
GAGTGAGCGG GGAGGAAGGC AAGGAGGGGA GGAAGAAACC GTTCCTCCTG TGGATCTGCA 1440
AAGACCAGTC CAAGAGGATT TTAGTGTTAG GAAAAGGAAT CTGGAGTGAC GAGAAAGGGG 1500
GCCTTTCTAG ATGTTGCATG GCTTTGGTGT CGGGAGCCAC TTATGGGACA GCAGGTACTC 1560
TAAAAAGCCA CCTCCTTAGG AAAGCAGAGA GGCCCTGGCC AGCTCAGGCT CCCAGCAAGA 1620
GCTCCTTCTA GGAGACAGCT GAGGGATGAA ACACACCCAA GGCTCAAGAG GGGCAGGTTC 1680
TTCCCAGATA CAGACCCAGG AAGGAGATAA AGGCTTGGTG CCTCTATTTG GTTCAGGATA 1740
AGGGCCCCTG TCCTCTTTCT CTGATAACAC TGTCCTCTTT CTCTGATAAC ACCGTCCTCC 1800
CTTCCAGATC CACGTACAAA GGAGGCCCTT AAAAAGGCAC TTGGTCATTC ACAGCTCAAA 1860
CTGAGCAAGA GGCTGTGGGA GAAGAATCAA GTTGGTCCCG AGGGGAAGAG GTGTCAAAGG 1920
CTTAAGAAAC AAGAAGTCAG AGTTTACCTG GGTTTGAGGG AGAATTTTCT TTCCCCCTTT 1980

# FIG. 24(cont.)

TCCTCCTCCT CCTCCTTCTT CTCTTTTTTT TTTTTTTTTT TTTTTTTTTT TTGAGACATG 2040
GTCTCATTCT GTCACCCAGC ACCCAGGCTG GAATGTAGTG GCACGATCAC TATCACGGCT 2100
CACTACAGCC TCTACCTCCC GGGCTCAAGT GATCCTCCTA CCTCAGCCTC CTGAGTAACT 2160
GGGACTACAG GCACATGCCA CCACACCCAG CTATTTTTTT TTTTGCTAGA GATGGGGGTC 2220
TCTACCAGGT TGGTCTCATA CTCTTGTACT CAAATGATTC TCCTGTCTCA TCCTCCCAAA 2280
GGGTGGGATT ACAGGCATAA GCCACCATGC CTGGCTCTTC TTTTGGTTTC AGAGAAAAAC 2340
ATCTCCTTAA AATGTTTATT TCCCAAGGAT TCTTGAAAAA GAAAGCTCAC TGACACACCC 2400
AAAACAATCT GGTTTTGCTC TGTGCTTTTA GGGAGAACTT TCTAAGCAGC AGAGCCCTTC 2460
TGAGTGGCAG GGCTGTCTTA GGAGGAAGGT GTCTTTTGAT GATGGGGAAC TTCATGTCCA 2520
GGTCTGGCAG GAGAGTTACC CCACTTTCCT GCCTACTCCC TGGGGCTTTG GGGTAGTAGT 2580
ACCACATTGG GCCATGTCAT TTAGGTGAGT CCTTCAACAT CACTTTCTCT GCTTCTCCCT 2640
CTTTCTGGAT CCTCCTTCTT GGAGCCTTTC AAGGGGACCT CCTCTCACAG TGTCCATAGC 2700
ATCTCTTAGC TAATGGTCCT TAAAATCTCT ACCAGCAGCT TCTCTCTGAT AGCTAAGAGC 2760
TGCCATTTAC TGGGAACTTT CTATGTACTG GGCTCTGTGC TAAGTGCCCT AGATGAGAGA 2820
TGTGCAGTGT GGTGCCTAAA CCTTGGGCTT GGAGCAGACA CACACTTTCA AATCCTGCCT 2880
TCAGCTCCTT AGTGAACATG TCACCTTGGG CGGGACACAC GCCTCTCTGT GCCTCAGTTT 2940
CCTACACTTT AGAATGGGGA TAACACTGAA TAATGTTCTT GTGAGGATGC AGGGAATTAA 3000
CCCACGCACA GTACTTATAA TAGTGTCTGG CGCCTGTGTT CGATAAGTTT TAGCAATTCT 3060
AATCATCTCT TTTAAGCCTC GCAGCAAGCC TCTAAGGTAA GTCTGTATTA GTATCCCTAT 3120
TTACAGATGA GAAAACTGAG GTTCACAGGG GATGAGACAG TGTACAGTCT GCAGTCCAGC 3180
AATTACTCTG CTACTCAGCA ATAAAAATAG TAACAGCTAA CCCTTAGACT AAGTGGCAGA 3240
GTCAGGCTTT AGATTCATGA GGTGAGTTCT GGAATCCATC CCTTTAATAA CCACACTAAA 3300
TTGCCTTTCT GAAATGGTTA TATAAAGCAT ATCTACCCAA TCTTGGAGTT TTTTAAATGG 3360
CACCTAGTTT GGTGCTGGAA ATGCAGTTGA CCTTCAAAGC AATTCTTTGG AGGCAGCATC 3420
AATCCCTCTG GAAATACCTC GGTGGCATGG CTGGCCTTAT TCTACAGGTA AGGAACTTGA 3480
AGCTAAGCAT CAGTAACCCC GTGAAGTCAC AGTTAGTATA GGTTGGAATT GGGATTCAAA 3540
TCTGTACCTG ACTTTATAAT TCCTAGCTGG GCCCCAGAAT CTTTGATAGA GGTGTCTTCT 3600
TTCTTTTCTT TTCTTTCTTT CCTCTTTCTT TCCCTTCCTT CCTCTCTCTC TGTCTTTCTT 3660
CTCTCCTTTC TTTCTCACAG AATCAAAATC TCTTGGGGTG GGGCCTGGGC ATCTGATTTT 3720
TAAAAACCAG ACATCTGATG TGCAGTCAAC ACTGAGAACC CCTGCCAGCT TCATCTCCTC 3780
TTCTAAGTGC CAGACCCAAG TTTCCAACTG TCTGCCCACC TGTCTCCCCA CCTGGGCACC 3840
CGCCAGCGTC TCACCCTCAG GAGACTCCAG CTGAACTAAT CCTCTCTCCC TGCTTTTCCA 3900
GAACAGGTCC CACCCTCCCT CCACTCAGTC TCTCCTGCTG GGAACCCTGG TCATCTGCAC 3960

# FIG. 24(cont.)

```
TGTGCCTTCA TCTTCCATCC TGCCAGTGCT GCCCGGTGTG TCTCTTAAAC CCATGCCTCC     4020
TCTGTGTGCA CCACCTGCAC TTTGGTAAAA GCCTTCATTT CCTGCTTGGG TTACTACAAC     4080
GCCCCCTAAC TCATCTCACT GTCTCTATTT CTGCTTCTCT GTCTCTCCCT AGGCTACTCC     4140
CATTCTTCCT CCCCTTTCCT CTTCATCCCA AAGTCCAACC CATATCCTTT TACCAGTAGG     4200
ACTTAAGGAA CTAAAGACTA TCTCATCACC CACTTTTCTT CTTAAAAACT TCCACTGCAC     4260
TGCCTGCTGA GATGGCCTTC CTACCCAACT TGGCTGGAAA ACTCCTACCC ATCTTGTGGA     4320
ACCCAGTTCA AAAGTCACCA CCTCTGAGAA GCCTTCCCTG AGGCTCCTAG GGAGATGGGT     4380
ACTGCCTCCT CTGTCCTTCT CCAGCACAGG CCCCATCTTC AATCACAGGA TTGTGCTGGA     4440
ATGATTGGAT GCCAAGTCTG TCCCTCACTG AACTCCTTAT GCAAAATCCA TATTATATGT     4500
TTCCTTTTGC CAGGTGTGGG CCCAGGTGCT GGGGATACCG ATGAATAAAA CTGAGTTTCT     4560
GTCTTCAAGA AGCTCCAAGT CTACTGAGTG TAGCAGAGAA CAGGGAGAAG GCACTTCAGG     4620
GAGAAGGGGT AGCACATGCA AAGCCCCAGA AGGCAGGGAC AGAAGCCTTA GGGATGTCTG     4680
TGGGGGAGGA TGGAGGAAGA GGGTAACAGG AGACCAGGTG GGGAGATGAG GGAGGTGGTC     4740
TGGAAGGGCC ATGAGACACC CCTCACGCTC CCTGAGACCC CCTCCACGCT ATAGAGATGG     4800
GACTGGAGAG GACGATGATC ATTTGTGACT CAGATCCCTG TGGGTTTCTT CAGATTGGGT     4860
CTCACCCATC TTTACAGCCA CAGCACCTAA CACAGTGCCC GGCACACAGC AGGCCCTAGA     4920
CAAACGTTTG CCACATGAAG TCATGCCACT GGCCAGGAAG CCCACTGGGG ACTGGGGGGT     4980
TGGTTCTGCG ATAATGGGGT CCCTGAGATT CTATGTTTCA CGTGACTAAG CCTCACTCTG     5040
CCCCCACCTC CGCGGGGGCG TCCCGCAGGT GCCCGACTCC AGCC ATG CTG GCG CTA      5096
                                                 Met Leu Ala Leu
                                                  1

CTG TGT TCC TGC CTG CTC CTG GCA GCC GGT GCC TCG GAC GCC TGG ACG       5144
Leu Cys Ser Cys Leu Leu Leu Ala Ala Gly Ala Ser Asp Ala Trp Thr
  5                  10                  15                  20

GGC GAG GAC TCG GCG GAG CCC AAC TCT GAC TCG GCG GAG TGG ATC CGA       5192
Gly Glu Asp Ser Ala Glu Pro Asn Ser Asp Ser Ala Glu Trp Ile Arg
                 25                  30                  35

GAC ATG TAC GCC AAG GTC ACG GAG ATC TGG CAG GAG GTC ATG CAG CGG       5240
Asp Met Tyr Ala Lys Val Thr Glu Ile Trp Gln Glu Val Met Gln Arg
             40                  45                  50

CGG GAC GAC GAC GGC ACG CTC CAC GCC GCC TGC CAG GTG CAG CCG TCG       5288
Arg Asp Asp Asp Gly Thr Leu His Ala Ala Cys Gln Val Gln Pro Ser
         55                  60                  65

GCC ACG CTG GAC GCC GCG CAG CCC CGG GTG ACC GGC GTC GTC CTC TTC       5336
Ala Thr Leu Asp Ala Ala Gln Pro Arg Val Thr Gly Val Val Leu Phe
     70                  75                  80

CGG CAG CTT GCG CCC CGC GCC AAG CTC GAC GCC TTC TTC GCC CTG GAG       5384
Arg Gln Leu Ala Pro Arg Ala Lys Leu Asp Ala Phe Phe Ala Leu Glu
 85                  90                  95                 100

GGC TTC CCG ACC GAG CCG AAC AGC TCC AGC CGC GCC ATC CAC GTG CAC       5432
```

# FIG. 24(cont.)

```
Gly Phe Pro Thr Glu Pro Asn Ser Ser Ser Arg Ala Ile His Val His
                105                 110                 115

CAG TTC GGG GAC CTG AGC CAG GGC TGC GAG TCC ACC GGG CCC CAC TAC      5480
Gln Phe Gly Asp Leu Ser Gln Gly Cys Glu Ser Thr Gly Pro His Tyr
            120                 125                 130

AAC CCG CTG GCC GTG CCG CAC CCG CAG CAC CCG GGC GAC TTC GGC AAC      5528
Asn Pro Leu Ala Val Pro His Pro Gln His Pro Gly Asp Phe Gly Asn
        135                 140                 145

TTC GCG GTC CGC GAC GGC AGC CTC TGG AGG TAC CGC GCC GGC CTG GCC      5576
Phe Ala Val Arg Asp Gly Ser Leu Trp Arg Tyr Arg Ala Gly Leu Ala
    150                 155                 160

GCC TCG CTC GCG GGC CCG CAC TCC ATC GTG GGC CGG GCC GTG GTC GTC      5624
Ala Ser Leu Ala Gly Pro His Ser Ile Val Gly Arg Ala Val Val Val
165                 170                 175                 180

CAC GCT GGC GAG GAC GAC CTG GGC CGC GGC GGC AAC CAG GCC AGC GTG      5672
His Ala Gly Glu Asp Asp Leu Gly Arg Gly Gly Asn Gln Ala Ser Val
                185                 190                 195

GAG AAC GGG AAC GCG GGC CGG CGG CTG GCC TGC TGC GTG GTG GGC GTG      5720
Glu Asn Gly Asn Ala Gly Arg Arg Leu Ala Cys Cys Val Val Gly Val
            200                 205                 210

TGC GGG CCC GGG CTC TGG GAG CGC CAG GCG CGG GAG CAC TCA GAG CGC      5768
Cys Gly Pro Gly Leu Trp Glu Arg Gln Ala Arg Glu His Ser Glu Arg
        215                 220                 225

AAG AAG CGG CGG CGC GAG AGC GAG TGC AAG GCC GCC T GAGCGCGGCC         5815
Lys Lys Arg Arg Arg Glu Ser Glu Cys Lys Ala Ala
    230                 235                 240

CCCACCCGGC GGCGGCCAGG GACCCCCGAG GCCCCCCTCT GCCTTTGAGC TTCTCCTCTG    5875
CTCCAACAGA CACCTTCCAC TCTGAGGTCT CACCTTCGCC TCTGCTGAAG TCTCCCCGCA    5935
GCCCTCTCCA CCCAGAGGTC TCCCTATACC GAGACCCACC ATCCTTCCAT CCTGAGGACC    5995
GCCCCAACCC TCGGAGCCCC CCACTCAGTA GGTCTGAAGG CCTCCATTTG TACCGAAACA    6055
CCCCGCTCAC GCTGACAGCC TCCTAGGCTC CCTGAGGTAC CTTTCCACCC AGACCCTCCT    6115
TCCCCACCCC ATAAGCCCTG AGACTCCCGC CTTTGACCTG ACGATCTTCC CCCTTCCCGC    6175
CTTCAGGTTC CTCCTAGGCG CTCAGAGGCC GCTCTGGGGG GTTGCCTCGA GTCCCCCCAC    6235
CCCTCCCCAC CCACCACCGC TCCCGCGGCA AGCCAGCCCG TGCAACGGAA GCCAGGCCAA    6295
CTGCCCCGCG TCTTCAGCTG TTTCGCATCC ACCGCCACCC CACTGAGAGC TGCTCCTTTG    6355
GGGGAATGTT TGGCAACCTT TGTGTTACAG ATTAAAAATT CAGCAATTCA GTACTGCGTC    6415
GAGGTCTTGG TTACTTTTTT GTTTGTTTGT TTTAGGCTTC TCTCCCAAGC TGAGCTTTTT    6475
TTTGTTTTGT TTTCGTTTTC CTTTTTTTTC TTTTTTTTGG GAGTGGCAAA CATGCTTCCC    6535
AAATCCCTAC AGGACTTCTC CTTATCCTCT GCCCCCACCT CCCTAACCCT GCTGGCAACA    6595
ACGTTCAGCC ACTGCTTGTC TTGCCCTTCA GTGTGGCTCC AAGAGGAAGA TCACCAGAAT    6655
CACTCAGGGA AGTTAAAAAA AAAAATACAG CTTCCTGGGC TACATCCCAG AGCTGTGGAA    6715
```

# FIG. 24(cont.)

TCCAAAGGGA GAAGAGAAAG TGAATTTGCG ACAAGCGTCG GGATGATTCT GGCACTGGAC 6775
CCTCTGGCCT GAGAGGGGAA GAGGCCTTCC ATCTCACCTG GGCTGGTAGC TTGTCACATC 6835
TGCCTCCGAG TACAGCCTTA GGTCCATTTC CCAGATATCA GAGACAGTGC CAGGGAAGCC 6895
AGGTGACTGC ATCTTGCCTA GGCACAGAAG AGTAGGGTTG GAATGTGACG TTGTTAGCAT 6955
TTGGCAGGAC CAAAACCAGA GGCAAACGGA GGCAGTGGGA TGGAAAGGCA GTTGATTTTG 7015
ATGAAGGCTT GTTGGGAGTT CAGCTTTCTT TTGAAACTTA TAATCTATAC CCAGGCTAGA 7075
ACAGTCTTGT GTATACACCT TCATTCATGG AATAAACGTA CTTGCAATAA CTTTTTAGCC 7135
TCCCAGGGTA GCCTCACTTC CTAGCTGTGA CTTTTCCACC CTGGTTACTG GGAGGCAGCT 7195
TCCATTTCTC CCAGACTAGC TAGGCAGTGC GTCCAACTGA ACCGCAGCCA GAAACCTGTC 7255
TCCAGGGGTT ATTTTTACCT CTAACTAGGA CTAACTTATT TTAAAATCTT TCCTTGAGCC 7315
CAAGTGACAA CTGAAGAGAA AGGCTATTGC CTGGTGATTT TGCTCCACCA GTTGGTTCTC 7375
ACTGGTTTGA ATACTAACTT GAACTGTACT CATCGACACT GAAAGGGGAT GAGCAAACAG 7435
TGTCTCTAAA TCTCCTGATC CTGATCTCAA ATATCCCCCT AATTACAAGT TGCAACAAGG 7495
CAGCTATTAC ACGGGGACAC AGGATGGAGA GGATGGGTGC CAAACACCCA TCGTCTACTC 7555
TGCTGCCTCG GTTATGGTGA ATTCAGGACC ATCAAGGGAG GTGTGGACCT TTTTTTTCAG 7615
AAGGAGGCTG ACACTTCTTG TCAATTGCAT TGTGTTCTTA GTTTTGCTCT TCACAACCCT 7675
TGACCCCGTA GATGGGGGCT GAAGAGGCAC CCTGGCCGAC TCACTCTATT TCTGTTTTGG 7735
GAATGGGATG GATAAACTAT CCCATGGCCT CCAGAGCCAA AAAACCAAAA CGAAACAAAA 7795
CAAAAAACCC CAAAACAAAA AAGCAAAAAG CAAACAAGAA AAAAAAAAAA AGAGGAAATA 7855
ATAGGCAGAC AATTTACAGT TCATTGTAAG GGCAAAGATA TGCATATAGC ATGATGGTTA 7915
ACAGGTCAGG CTCAGGTAGA AAGGCCCATT TGAACCCCAG CTCTGCCACA CTCAGAAACT 7975
GTGTGACCCG AACAAGTCAC TTAACCTCTC TGAGCATAGG TAAAATAAGA TCATCATACC 8035
AGATTGTTTT GAAGATTAAA TCAAGTGTTA TTCACGAGAG GTGCACAGCA TAGCATGCAC 8095
AACAAATAAG GACCTGGTAA GTATCTAATT AATAACAATG GCTAAGATCC AAAAAACAGC 8155
TACCTACTAA TAAATAGATG GGGCTGCCTT GTAAGGCAGT GAGCATCATG CAACCAGGAT 8215
TCAAATGAAG GACAGTTGCT ACCTCTGAGG TTCCCGAGAA GGATTTCTCG ATCCATTGAG 8275
AGACTGAATG ACATGAACTC TGCGATCCCA TCTCTTGTGG GGAGGGAACC TAGAATGAAG 8335
GGAAGATTGT GGGCCATAAA GGCAGACATC TGGTTCCTGG GCACAGAACC ATATGTGTGC 8395
CACCAAAGCC ACCCACCGGA CCCCACTTGG CCCCTGGAGT CTATTTTTAC TCCTCTCATC 8455
TTACAAGATC TATTTTGTTA ATCTCCTTAT ATTTGCTGTT TTGACTTCCC AGCCAGCTTG 8515
CTAATCAGTT TGCCTATTTG ACTCACAGGG TTTGCATTTG TCACGGGGAC TGAAACACAC 8575
GCTTGTTTTG ATTTCTTTTT GTAAATTAGA AGCGTTGATG TAATGACTCT ACCTAGACAC 8635
AGCTGGTAAA GTGAGAATAA TGCTCAAGTT TGCACAGTTT AAACACAATG TAGACAATAA 8695

# FIG. 24(cont.)

TTAGAAATGC TATCTTTAGA TGTTTAGGAT AAGCTTTTCT CAGAATTGCA CTGATTTTTT 8755
TTTTCTGAGT GGGGCTTTTT AGTGCATATA TACAGAAATA CTAAAAACGT AAGAAAATAG 8815
AGCAAATCAG TGAGTGCTTT GGTCAACTTG AAAGACTGCA GGAAATAAAC CAACTGATTT 8875
TAGATCTGCC TTTTTTTGAC TGAATGCATA AAATCTTTAC ATTCTCCATA TTTTTCATGA 8935
CTACCATATG ATCAAATAGT TTTAGGTGAC AGATTGCAAC TGATAAGTTG CTGCAATATG 8995
GCAGAAGTCA TGCTCAGCCT CCGCTTGCCC GGTGGTGAGG GTGGAATATG AAGCAAACAA 9055
TAAAGATAAT TCATCATCTC TATCAGGAAA ATTGCCACAT GTTTATTTCA GGTAACAAAA 9115
AAGATATAGT TATGATATAC AATGACCATA GAATCCAATA AAGCAACTTC TGCAAATGAA 9175
TAGAAGGTAC TTTTTCTTTA AATGAAACTA CAAAATAGCA GCTGGTTTTA AAAACAAAGC 9235
CAATTGTTTT AGATTTAATA GGCTACCACT GGCCTCTGCT AAGATCCCCA AATATATTCC 9295
TGAGCTCACA TAGATTCCAG AAAGTCAAAC TTTTCAATAT TATGCAAACT TTCCCTATGC 9355
ATCCAAAAAA TTCTCATTTA GTAAAGAGGT GATATGAAAT GTAAGGCAGC ATGTCCATAT 9415
CTATCATTTT AAATTGCCTT CATGCTGTAT CAACTGGTTT TGTTTTGGGA AGCAACCATA 9475
ATATTGAGAG ACGGGTCTTT CCTATTTTTT CTGCTACTCA TTTCTAACTA GATTCACTAC 9535
GGAGCTCCCA ATTGCATCTC TCTGATCTAC AAATTTTTCT CTCTTCAGGA AGACACCTGG 9595
AAAGAAGGGA CTACATTAAA GGAGTGTGTT GGGGGCAATG CTTTGGCCTT TTGACATCCT 9655
ATCTAGTCTG AAGGGACCCT CACTATTGCT AAGGAGGAGG AGTGTTTTAA ATGGAGGCTT 9715
CAGAATGAAA GCAGAGGAAG AAGGTACTCT CTTTTTCAAA AAGAAGGAGG GTACAGGCCG 9775
GGCGCAGCTG TCACGCCTGC AATCCCAGCA CTTTGGGAGG CCGAGGAAGG CAGATCACGA 9835
GGTTGGGAGT TTGAGCCAGC CTGGTCAACA TAGTGAAACC CCGTCTCTAC TAAAAATACA 9895
AAAATTAGCC AGCATGGTGG TGCATGCCTG TAGTCCCAGT TACTCGGGAG GCTGAGGCAG 9955
GAGAATCGCT TGAACTCGGG AAGTGGAGGT TGCAGTGAGC CGAGATCATG CCACTGCACT 10015
CCACCCTGGG TGACAGAGTG AGACTCTCAA AAAAAAAAAA AAAAAAAAAA AGAAGTAGGG 10075
TACC 10079

FIG. 25

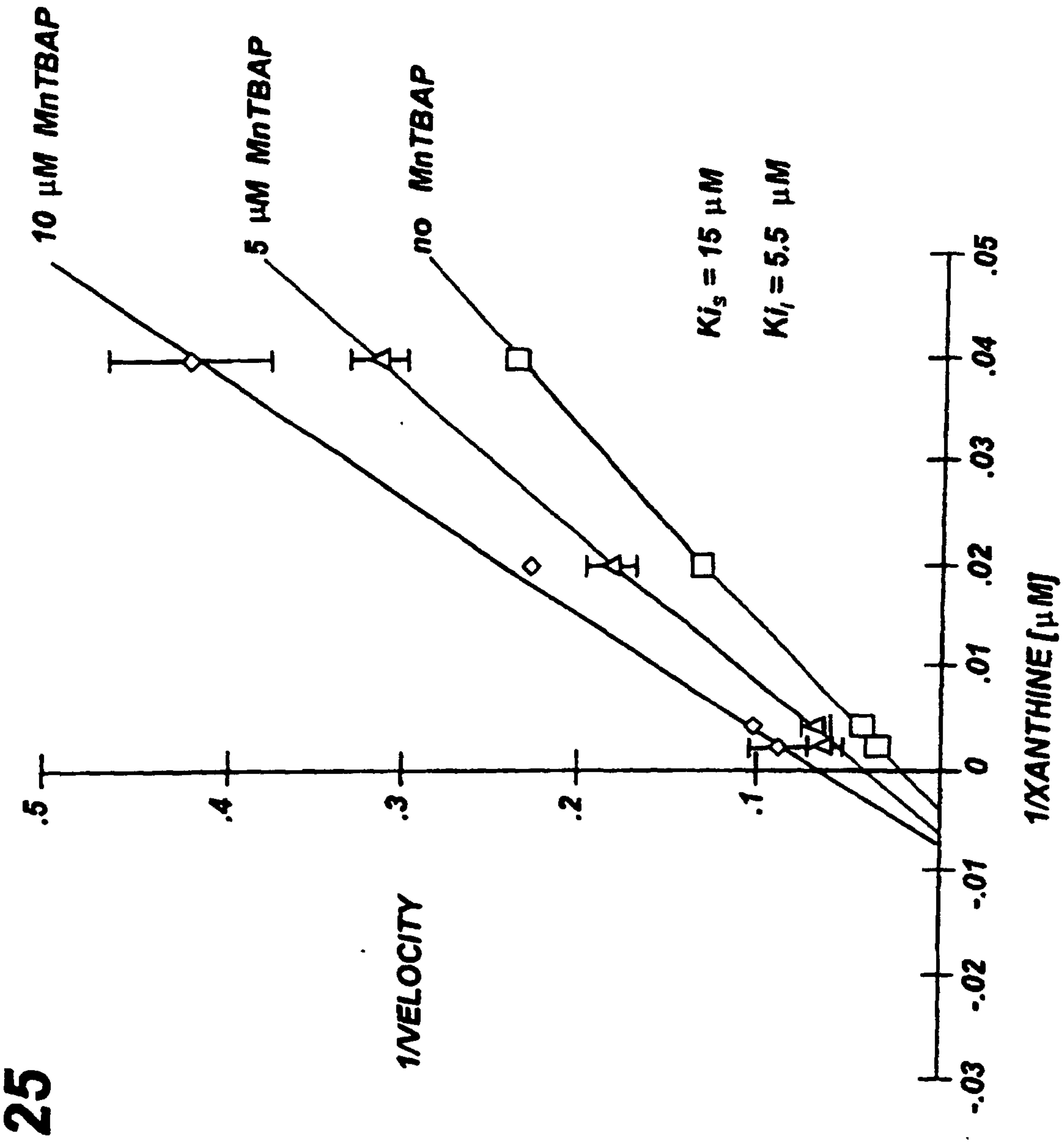
10 μM MnTBAP
5 μM MnTBAP
no MnTBAP
$Ki_s$ = 15 μM
$Ki_i$ = 5.5 μM
1/VELOCITY
1/XANTHINE [μM]
.5
.4
.3
.2
.1
0
-.03
-.02
-.01
.01
.02
.03
.04
.05

FIG. 26

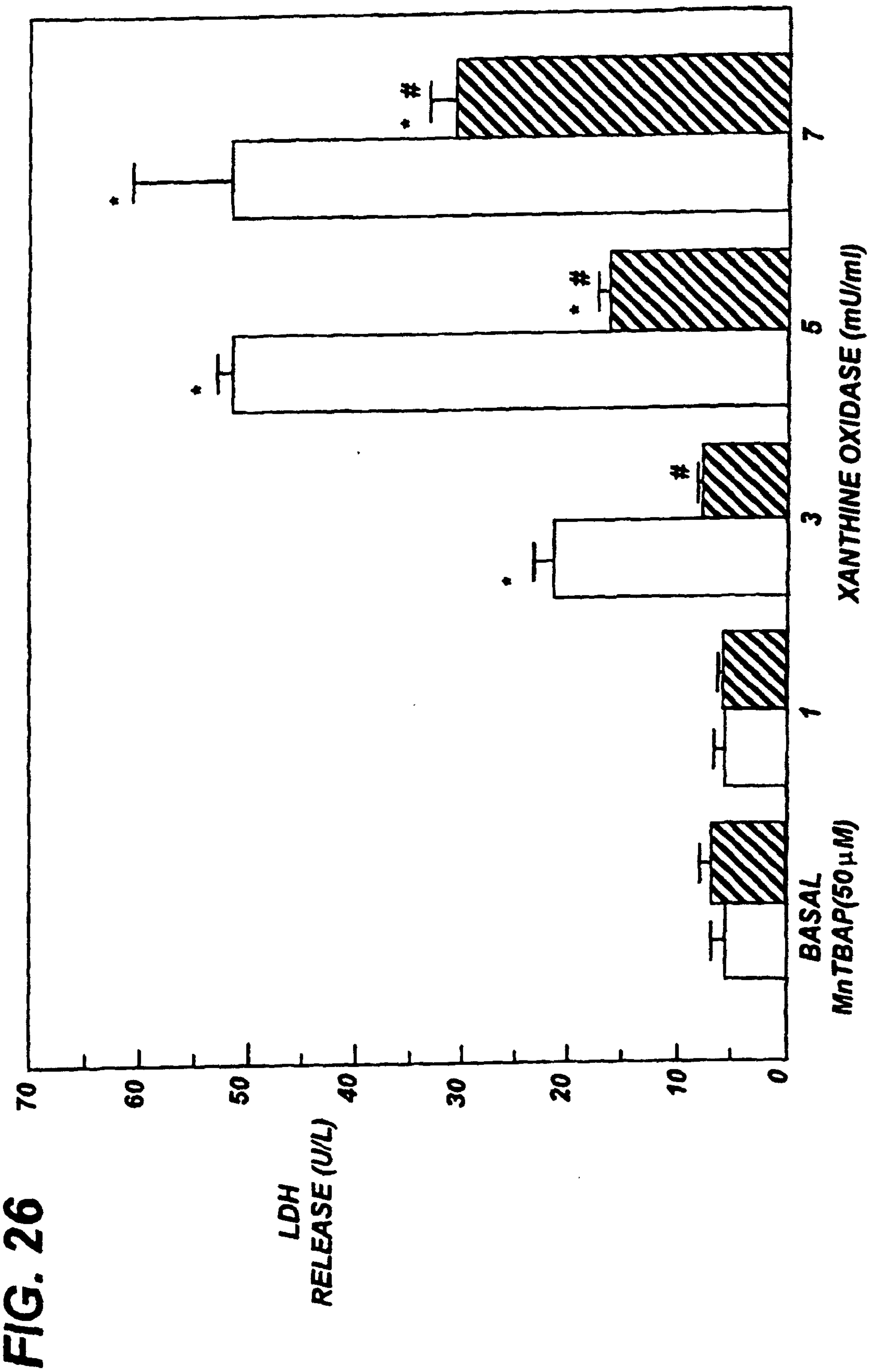
LDH
RELEASE (U/L)
0
10
20
30
40
50
60
70
BASAL
MnTBAP(50 μM)
1
3
5
7
XANTHINE OXIDASE (mU/ml)
*
#

FIG. 27

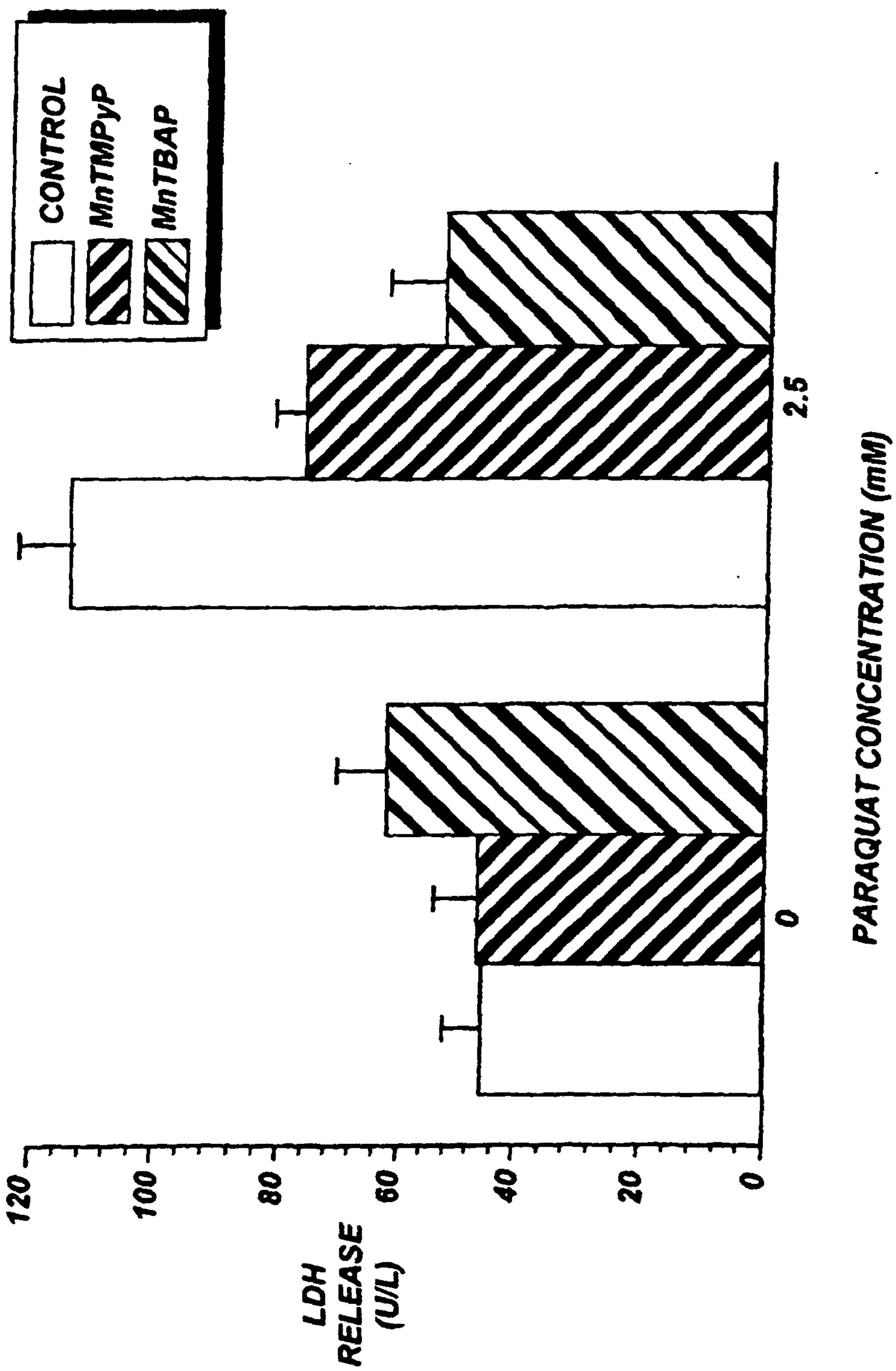
LDH
RELEASE
(U/L)
120
100
80
60
40
20
0
CONTROL
MnTMPyP
MnTBAP
0
2.5
PARAQUAT CONCENTRATION (mM)

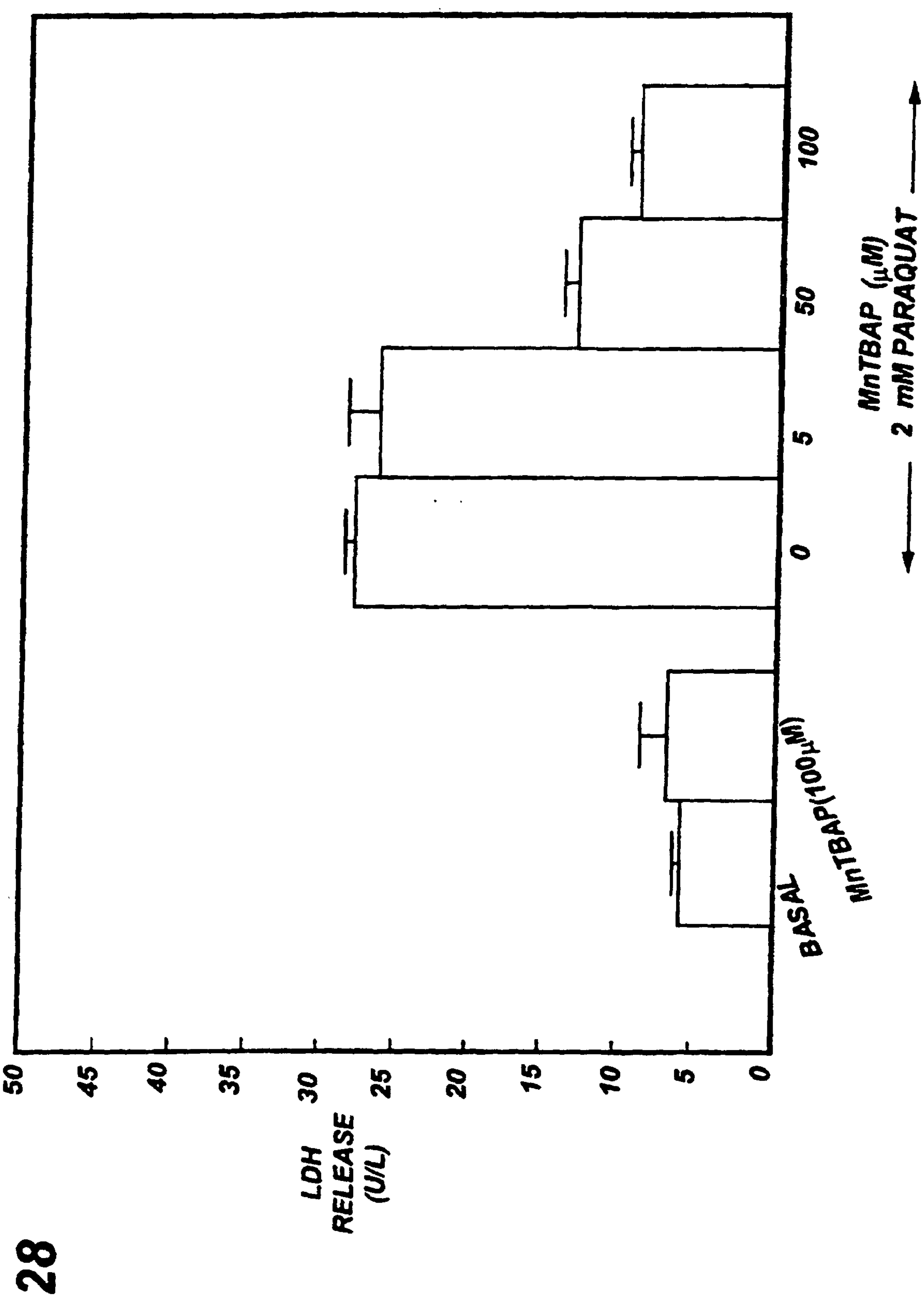
LDH RELEASE (U/L)
0
5
10
15
20
25
30
35
40
45
50
BASAL
MnTBAP(100μM)
0
5
50
100
MnTBAP (μM)
2 mM PARAQUAT

FIG. 28

FIG. 29

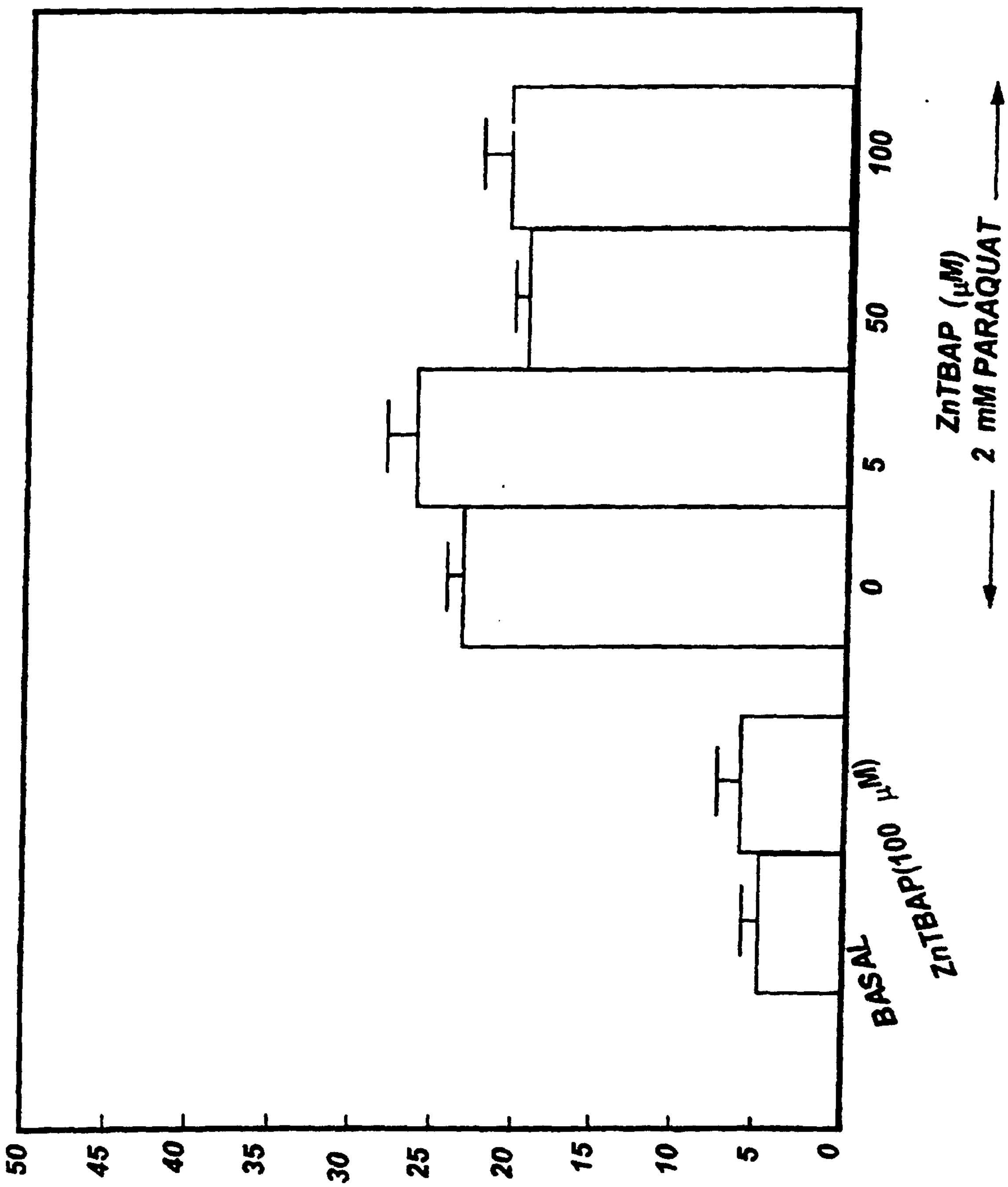
LDH RELEASE (U/L)
0
5
10
15
20
25
30
35
40
45
50
BASAL
ZnTBAP(100 μM)
0
5
50
100
ZnTBAP (μM)
2 mM PARAQUAT

FIG. 30

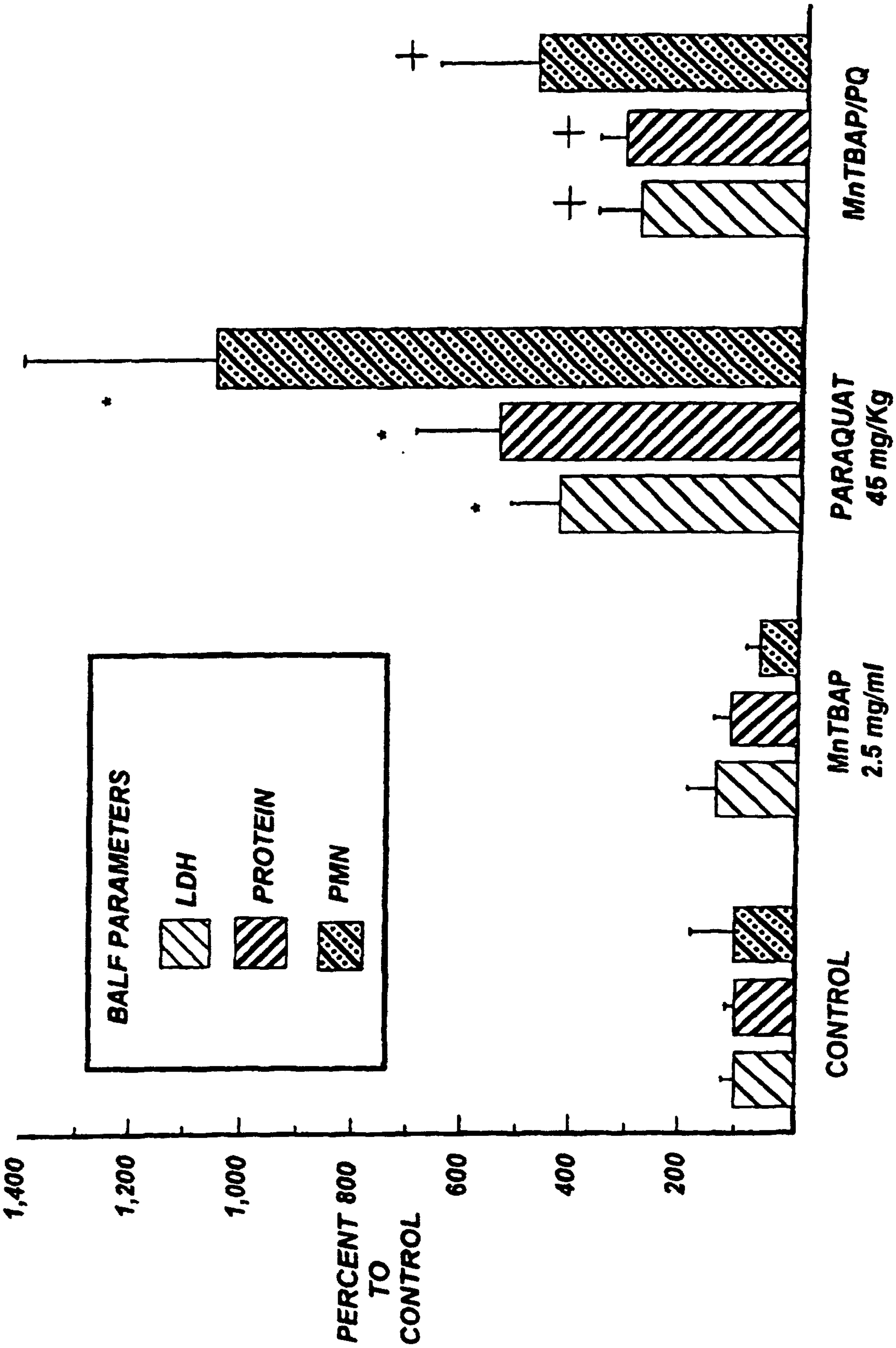
BALF PARAMETERS
LDH
PROTEIN
PMN
PERCENT TO CONTROL
1,400
1,200
1,000
800
600
400
200
CONTROL
MnTBAP 2.5 mg/ml
PARAQUAT 45 mg/Kg
MnTBAP/PQ

SEQUENCE LISTING

(1) GENERAL INFORMATION:

(i) APPLICANT:

(A) NAME: DUKE UNIVERSITY
(B) STREET: OFFICE OF SCIENCE AND TECHNOLOGY, P.O. BOX 90083, 001E ALLEN BUILDING
(C) CITY: DURHAM
(D) STATE: NC
(E) COUNTRY: USA
(F) POSTAL CODE (ZIP): 27708-0083

APPLICANT:

(A) NAME: UNIVERSITY OF ALABAMA AT BIRMINGHAM RESEARCH FOUNDATION
(B) STREET: 701 20th STREET SOUTH
(C) CITY: BIRMINGHAM
(D) STATE: AL
(E) COUNTRY: USA
(F) POSTAL CODE (ZIP): 35294-0111

(ii) TITLE OF INVENTION: SUPEROXIDE DISMUTASE AND MIMETICS THEREOF

(iii) NUMBER OF SEQUENCES:. 22

(iv) COMPUTER READABLE FORM:

(A) MEDIUM TYPE: Floppy disk

(B) COMPUTER: IBM PC compatible
(C) OPERATING SYSTEM: PC-DOS/MS-DOS
(D) SOFTWARE: PatentIn Release #1.0, Version #1.25

(v) CURRENT APPLICATION DATA:

(A) APPLICATION NUMBER: US 08/322,766
(B) FILING DATE: 13-OCT-1994
(C) CLASSIFICATION:

(vi) PRIOR APPLICATION DATA:

(A) APPLICATION NUMBER: US 08/136,207
(B) FILING DATE: 15-OCT-1993
(C) CLASSIFICATION:

(2) INFORMATION FOR SEQ ID NO:1:

(i) SEQUENCE CHARACTERISTICS:

(A) LENGTH: 15 amino acids
(B) TYPE: amino acid
(D) TOPOLOGY: linear

(ii) MOLECULE TYPE: protein

(xi) SEQUENCE DESCRIPTION: SEQ ID NO:1:

```
His Arg His His Pro Arg Glu Met Lys Lys Arg Val Glu Asp Leu
1               5                   10                  15
```

(2) INFORMATION FOR SEQ ID NO:2:

(i) SEQUENCE CHARACTERISTICS:

(A) LENGTH: 18 amino acids
(B) TYPE: amino acid
(D) TOPOLOGY: linear

(ii) MOLECULE TYPE: peptide

(xi) SEQUENCE DESCRIPTION: SEQ ID NO:2:

```
Arg Glu His Ser Glu Arg Lys Lys Arg Arg Arg Glu Ser Glu Cys Lys
1               5                   10                  15

Ala Ala
```

(2) INFORMATION FOR SEQ ID NO:3:

(i) SEQUENCE CHARACTERISTICS:

(A) LENGTH: 12 amino acids
(B) TYPE: amino acid
(D) TOPOLOGY: linear

(ii) MOLECULE TYPE: protein

(xi) SEQUENCE DESCRIPTION: SEQ ID NO:3:

```
Arg Glu His Ser Glu Arg Lys Lys Arg Arg Arg Glu
1               5                   10
```

(2) INFORMATION FOR SEQ ID NO:4:

(i) SEQUENCE CHARACTERISTICS:

(A) LENGTH: 12 amino acids
(B) TYPE: amino acid
(D) TOPOLOGY: linear

(ii) MOLECULE TYPE: protein

(xi) SEQUENCE DESCRIPTION: SEQ ID NO:4:

```
Arg Glu His Ser Glu Arg Lys Lys Arg Arg Arg Ala
1               5                   10
```

(2) INFORMATION FOR SEQ ID NO:5:

(i) SEQUENCE CHARACTERISTICS:

(A) LENGTH: 18 amino acids
(B) TYPE: amino acid
(D) TOPOLOGY: linear

(ii) MOLECULE TYPE: protein

(xi) SEQUENCE DESCRIPTION: SEQ ID NO:5:

```
Arg Glu His Ser Glu Arg Lys Lys Arg Arg Arg Ala Ser Glu Cys Lys
1               5                   10                  15

Ala Ala
```

(2) INFORMATION FOR SEQ ID NO:6:

(i) SEQUENCE CHARACTERISTICS:

(A) LENGTH: 18 amino acids
(B) TYPE: amino acid
(D) TOPOLOGY: linear

(ii) MOLECULE TYPE: protein

(xi) SEQUENCE DESCRIPTION: SEQ ID NO:6:

```
Arg Glu His Ser Glu Arg Lys Lys Arg Arg Arg Glu Ser Glu Ala Lys
1               5                   10                  15

Ala Ala
```

(2) INFORMATION FOR SEQ ID NO:7:

(i) SEQUENCE CHARACTERISTICS:

(A) LENGTH: 18 amino acids
(B) TYPE: amino acid
(D) TOPOLOGY: linear

(ii) MOLECULE TYPE: protein

(xi) SEQUENCE DESCRIPTION: SEQ ID NO:7:

```
Arg Glu His Ser Glu Arg Lys Lys Arg Arg Arg Glu Ser Glu Cys Ala
1               5                   10                  15

Ala Ala
```

(2) INFORMATION FOR SEQ ID NO:8:

(i) SEQUENCE CHARACTERISTICS:

(A) LENGTH: 18 amino acids
(B) TYPE: amino acid
(D) TOPOLOGY: linear

(ii) MOLECULE TYPE: protein

(xi) SEQUENCE DESCRIPTION: SEQ ID NO:8:

```
Arg Glu His Ser Glu Arg Lys Lys Arg Arg Arg Ala Ser Ala Cys Lys
1               5                   10                  15

Ala Ala
```

(2) INFORMATION FOR SEQ ID NO:9:

(i) SEQUENCE CHARACTERISTICS:

(A) LENGTH: 18 amino acids
(B) TYPE: amino acid
(D) TOPOLOGY: linear

(ii) MOLECULE TYPE: protein

(xi) SEQUENCE DESCRIPTION: SEQ ID NO:9:

```
Arg Glu His Ser Glu Arg Lys Lys Arg Arg Arg Ala Ser Glu Cys Ala
1               5                   10                  15

Ala Ala
```

(2) INFORMATION FOR SEQ ID NO:10:

(i) SEQUENCE CHARACTERISTICS:

(A) LENGTH: 21 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:10:

```
ATGACCTCCT GCCAGATCTC C                                          21
```

(2) INFORMATION FOR SEQ ID NO:11:

(i) SEQUENCE CHARACTERISTICS:

(A) LENGTH: 18 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:11:

```
AGGCAGGAAC ACAGTAGC                                              18
```

(2) INFORMATION FOR SEQ ID NO:12:

(i) SEQUENCE CHARACTERISTICS:

(A) LENGTH: 22 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:12:

```
TGGGTGCAGC TCTCTTTTCA GG                                         22
```

(2) INFORMATION FOR SEQ ID NO:13:

(i) SEQUENCE CHARACTERISTICS:

(A) LENGTH: 20 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:13:

TCCAGCTCCT CCAAGAGAGC 20

(2) INFORMATION FOR SEQ ID NO:14:

(i) SEQUENCE CHARACTERISTICS:

(A) LENGTH: 12 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:14:

TGCGGGGTGG AC 12

(2) INFORMATION FOR SEQ ID NO:15:

(i) SEQUENCE CHARACTERISTICS:

(A) LENGTH: 12 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:15:

GGAAAGGTGG GT 12

(2) INFORMATION FOR SEQ ID NO:16:

(i) SEQUENCE CHARACTERISTICS:

(A) LENGTH: 12 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:16:

GCCCAGGCTC CA 12

(2) INFORMATION FOR SEQ ID NO:17:

(i) SEQUENCE CHARACTERISTICS:

(A) LENGTH: 12 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:17:

CCGCAGGTGC CC 12

(2) INFORMATION FOR SEQ ID NO:18:

(i) SEQUENCE CHARACTERISTICS:

(A) LENGTH: 11 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:18:

RGTGACNNNG C 11

(2) INFORMATION FOR SEQ ID NO:19:

(i) SEQUENCE CHARACTERISTICS:

(A) LENGTH: 9 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:19:

CAGCTGTGG 9

(2) INFORMATION FOR SEQ ID NO:20:

(i) SEQUENCE CHARACTERISTICS:

(A) LENGTH: 10079 base pairs
(B) TYPE: nucleic acid

(C) STRANDEDNESS: single
(D) TOPOLOGY: linear

(ii) MOLECULE TYPE: DNA (genomic)

(v) FRAGMENT TYPE: linear

(ix) FEATURE:

(A) NAME/KEY: CDS
(B) LOCATION: 5085..5804

(xi) SEQUENCE DESCRIPTION: SEQ ID NO:20:

```
GGATCCAGAG ATTTAGATTT TTTATAAGCT TTCCTGCCAC CGAAACGGGT GTTTGGGACC      60

TCACGAGGCC CTGTTCATTC TTCGTCGCTG CGCTCCCCAC TCTGTACTGG ATGCATTTAC     120

TGACGTTGTT GTCTCCGTCC CCAGAGTATG AACCCCCAAG GTGACTCATG CAGCTGTGGG     180

TGCCCGGCAT ACAGCATGGT GACTGGAATG GATGAGCACC CAATAAACAT TTGTTGCAGG     240
```

AATGCAGGAG GACGGGCAGG CCAGCAAGCA GGCTGCCTGG TTTTTCCCAC ATGGGCTTTT 300
CTGGGAAAGA AGAGCTTCTA TTTTTGGAAA GGGCTGCTAT GATTGAGAAA AGTTCATGGC 360
AGCAAAAAAA GGACAGACGT CGGGAGGGAA ACACTCCTAG TTCTCCCAGA CAACACATTT 420
TTTAAAAAGA CTCCTTCATC TCTTTAATAA TAACGGTAAC GACAATGACA ATGATGATTA 480
CTTATGAGTG CGGCTAGTGC CAGCCACTGT GTTGTCACTG GGCGAGTAAT GATCTCATTG 540
GATCTTCACG GTGGGCGTGC GGGGTGGACA GCCTCACACC CCCATTTTAC AGATGATGAA 600
AAGGAGGTGC AGGGAGTGGT GCAGCTGCTT CAGGCGTACA CAGATAGGAA GTGACAAGGC 660
TGGGACTCTG CAGCCTGAGT GTGTCATCAC GACCCACCCG CTGCTCTGCT CTCATAGGTA 720
TGACAGCACA GCTCTGGAGC AAATGCCATG CACATTTGCA AGGTGCCCAT TTCCATGCAG 780
CAAAAATAAG TCAATAAGTT ATTGACTTAG AGAAAAGCAA AGGGCCTCTC AATAAAGAGG 840
TCATTGTACA CCTCTCCAAA CAGGCGATTT TCTTTCTCAT TTTTATTCCC CTGCTGTGTG 900
CTGAAGGTCA CTGGCTACAA GCCGGTGAAG TCGCGGAATG GAATCCTTGG CCCGAAAACC 960
CAAAAATGGG AGGGGCAGAG GAGGTGGGGA CAGAGCGGGA GGAGGTGGAG GCGAAGCAAT 1020
TCTACAACCC GGGGAGGTCT GGCCTGCTTT TCCTCCCTGA ACTGGCCCAA TGACTGGCTC 1080
CCTCACGCTG ACCACTCCTC TGGGCTGGCC TCCTGCACTC GCGCTAACAG CCCAGGCTCC 1140
AGGGACAGCC TGCGTTCCTG GGCTGGCTGG GTGCAGCTCT CTTTTCAGGA GAGAAAGCTC 1200
TCTTGGAGGA GCTGGAAAGG TGGGTGCTAA GTTGAGGTTC ATTTTGTTCT TCTCGGAGTG 1260
TGCTTATTGA GTCTGAAGCT GGGTTGGGGC AACGGGCCTC TTCTTGGGAA CAAATTGGAT 1320
CATCTTCTTG GGAAGGAAAT GTACTTTCCC TGGCTGCTCT GAGGGGTTAG TGGGGAGGTG 1380
GAGTGAGCGG GGAGGAAGGC AAGGAGGGGA GGAAGAAACC GTTCCTCCTG TGGATCTGCA 1440
AAGACCAGTC CAAGAGGATT TTAGTGTTAG GAAAAGGAAT CTGGAGTGAC GAGAAAGGGG 1500
GCCTTTCTAG ATGTTGCATG GCTTTGGTGT CGGGAGCCAC TTATGGGACA GCAGGTACTC 1560
TAAAAAGCCA CCTCCTTAGG AAAGCAGAGA GGCCCTGGCC AGCTCAGGCT CCCAGCAAGA 1620
GCTCCTTCTA GGAGACAGCT GAGGGATGAA ACACACCCAA GGCTCAAGAG GGGCAGGTTC 1680
TTCCCAGATA CAGACCCAGG AAGGAGATAA AGGCTTGGTG CCTCTATTTG GTTCAGGATA 1740
AGGGCCCCTG TCCTCTTTCT CTGATAACAC TGTCCTCTTT CTCTGATAAC ACCGTCCTCC 1800
CTTCCAGATC CACGTACAAA GGAGGCCCTT AAAAAGGCAC TTGGTCATTC ACAGCTCAAA 1860
CTGAGCAAGA GGCTGTGGGA GAAGAATCAA GTTGGTCCCG AGGGGAAGAG GTGTCAAAGG 1920

CTTAAGAAAC AAGAAGTCAG AGTTTACCTG GGTTTGAGGG AGAATTTTCT TTCCCCCTTT 1980

TCCTCCTCCT CCTCCTTCTT CTCTTTTTTT TTTTTTTTTT TTTTTTTTTT TTGAGACATG 2040

GTCTCATTCT GTCACCCAGC ACCCAGGCTG GAATGTAGTG GCACGATCAC TATCACGGCT 2100

CACTACAGCC TCTACCTCCC GGGCTCAAGT GATCCTCCTA CCTCAGCCTC CTGAGTAACT 2160

GGGACTACAG GCACATGCCA CCACACCCAG CTATTTTTTT TTTTGCTAGA GATGGGGGTC 2220

TCTACCAGGT TGGTCTCATA CTCTTGTACT CAAATGATTC TCCTGTCTCA TCCTCCCAAA 2280

GGGTGGGATT ACAGGCATAA GCCACCATGC CTGGCTCTTC TTTTGGTTTC AGAGAAAAAC 2340

ATCTCCTTAA AATGTTTATT TCCCAAGGAT TCTTGAAAAA GAAAGCTCAC TGACACACCC 2400

AAAACAATCT GGTTTTGCTC TGTGCTTTTA GGGAGAACTT TCTAAGCAGC AGAGCCCTTC 2460

TGAGTGGCAG GGCTGTCTTA GGAGGAAGGT GTCTTTTGAT GATGGGGAAC TTCATGTCCA 2520

GGTCTGGCAG GAGAGTTACC CCACTTTCCT GCCTACTCCC TGGGGCTTTG GGGTAGTAGT 2580

ACCACATTGG GCCATGTCAT TTAGGTGAGT CCTTCAACAT CACTTTCTCT GCTTCTCCCT 2640

CTTTCTGGAT CCTCCTTCTT GGAGCCTTTC AAGGGGACCT CCTCTCACAG TGTCCATAGC 2700

ATCTCTTAGC TAATGGTCCT TAAAATCTCT ACCAGCAGCT TCTCTCTGAT AGCTAAGAGC 2760

TGCCATTTAC TGGGAACTTT CTATGTACTG GGCTCTGTGC TAAGTGCCCT AGATGAGAGA 2820

TGTGCAGTGT GGTGCCTAAA CCTTGGGCTT GGAGCAGACA CACACTTTCA AATCCTGCCT 2880

TCAGCTCCTT AGTGAACATG TCACCTTGGG CGGGACACAC GCCTCTCTGT GCCTCAGTTT 2940

CCTACACTTT AGAATGGGGA TAACACTGAA TAATGTTCTT GTGAGGATGC AGGGAATTAA 3000

CCCACGCACA GTACTTATAA TAGTGTCTGG CGCCTGTGTT CGATAAGTTT TAGCAATTCT 3060

AATCATCTCT TTTAAGCCTC GCAGCAAGCC TCTAAGGTAA GTCTGTATTA GTATCCCTAT 3120

TTACAGATGA GAAAACTGAG GTTCACAGGG GATGAGACAG TGTACAGTCT GCAGTCCAGC 3180

AATTACTCTG CTACTCAGCA ATAAAAATAG TAACAGCTAA CCCTTAGACT AAGTGGCAGA 3240

GTCAGGCTTT AGATTCATGA GGTGAGTTCT GGAATCCATC CCTTTAATAA CCACACTAAA 3300

TTGCCTTTCT GAAATGGTTA TATAAAGCAT ATCTACCCAA TCTTGGAGTT TTTTAAATGG 3360

CACCTAGTTT GGTGCTGGAA ATGCAGTTGA CCTTCAAAGC AATTCTTTGG AGGCAGCATC 3420

AATCCCTCTG GAAATACCTC GGTGGCATGG CTGGCCTTAT TCTACAGGTA AGGAACTTGA 3480

AGCTAAGCAT CAGTAACCCC GTGAAGTCAC AGTTAGTATA GGTTGGAATT GGGATTCAAA 3540

TCTGTACCTG ACTTTATAAT TCCTAGCTGG GCCCCAGAAT CTTTGATAGA GGTGTCTTCT 3600

```
TTCTTTTCTT TTCTTTCTTT CCTCTTTCTT TCCCTTCCTT CCTCTCTCTC TGTCTTTCTT    3660

CTCTCCTTTC TTTCTCACAG AATCAAAATC TCTTGGGGTG GGGCCTGGGC ATCTGATTTT    3720

TAAAAACCAG ACATCTGATG TGCAGTCAAC ACTGAGAACC CCTGCCAGCT TCATCTCCTC    3780

TTCTAAGTGC CAGACCCAAG TTTCCAACTG TCTGCCCACC TGTCTCCCCA CCTGGGCACC    3840

CGCCAGCGTC TCACCCTCAG GAGACTCCAG CTGAACTAAT CCTCTCTCCC TGCTTTTCCA    3900

GAACAGGTCC CACCCTCCCT CCACTCAGTC TCTCCTGCTG GGAACCCTGG TCATCTGCAC    3960

TGTGCCTTCA TCTTCCATCC TGCCAGTGCT GCCCGGTGTG TCTCTTAAAC CCATGCCTCC    4020

TCTGTGTGCA CCACCTGCAC TTTGGTAAAA GCCTTCATTT CCTGCTTGGG TTACTACAAC    4080

GCCCCCTAAC TCATCTCACT GTCTCTATTT CTGCTTCTCT GTCTCTCCCT AGGCTACTCC    4140

CATTCTTCCT CCCCTTTCCT CTTCATCCCA AAGTCCAACC CATATCCTTT TACCAGTAGG    4200

ACTTAAGGAA CTAAAGACTA TCTCATCACC CACTTTTCTT CTTAAAAACT TCCACTGCAC    4260

TGCCTGCTGA GATGGCCTTC CTACCCAACT TGGCTGGAAA ACTCCTACCC ATCTTGTGGA    4320

ACCCAGTTCA AAAGTCACCA CCTCTGAGAA GCCTTCCCTG AGGCTCCTAG GGAGATGGGT    4380

ACTGCCTCCT CTGTCCTTCT CCAGCACAGG CCCCATCTTC AATCACAGGA TTGTGCTGGA    4440

ATGATTGGAT GCCAAGTCTG TCCCTCACTG AACTCCTTAT GCAAAATCCA TATTATATGT    4500

TTCCTTTTGC CAGGTGTGGG CCCAGGTGCT GGGGATACCG ATGAATAAAA CTGAGTTTCT    4560

GTCTTCAAGA AGCTCCAAGT CTACTGAGTG TAGCAGAGAA CAGGGAGAAG GCACTTCAGG    4620

GAGAAGGGGT AGCACATGCA AAGCCCCAGA AGGCAGGGAC AGAAGCCTTA GGGATGTCTG    4680

TGGGGGAGGA TGGAGGAAGA GGGTAACAGG AGACCAGGTG GGGAGATGAG GGAGGTGGTC    4740

TGGAAGGGCC ATGAGACACC CCTCACGCTC CCTGAGACCC CCTCCACGCT ATAGAGATGG    4800

GACTGGAGAG GACGATGATC ATTTGTGACT CAGATCCCTG TGGGTTTCTT CAGATTGGGT    4860

CTCACCCATC TTTACAGCCA CAGCACCTAA CACAGTGCCC GGCACACAGC AGGCCCTAGA    4920

CAAACGTTTG CCACATGAAG TCATGCCACT GGCCAGGAAG CCCACTGGGG ACTGGGGGGT    4980

TGGTTCTGCG ATAATGGGGT CCCTGAGATT CTATGTTTCA CGTGACTAAG CCTCACTCTG    5040

CCCCCACCTC CGCGGGGGCG TCCCGCAGGT GCCCGACTCC AGCC ATG CTG GCG CTA     5096
                                                 Met Leu Ala Leu
                                                  1

CTG TGT TCC TGC CTG CTC CTG GCA GCC GGT GCC TCG GAC GCC TGG ACG      5144
Leu Cys Ser Cys Leu Leu Leu Ala Ala Gly Ala Ser Asp Ala Trp Thr
 5                  10                  15                  20
```

```
GGC GAG GAC TCG GCG GAG CCC AAC TCT GAC TCG GCG GAG TGG ATC CGA        5192
Gly Glu Asp Ser Ala Glu Pro Asn Ser Asp Ser Ala Glu Trp Ile Arg
                25                  30                  35

GAC ATG TAC GCC AAG GTC ACG GAG ATC TGG CAG GAG GTC ATG CAG CGG        5240
Asp Met Tyr Ala Lys Val Thr Glu Ile Trp Gln Glu Val Met Gln Arg
            40                  45                  50

CGG GAC GAC GAC GGC ACG CTC CAC GCC GCC TGC CAG GTG CAG CCG TCG        5288
Arg Asp Asp Asp Gly Thr Leu His Ala Ala Cys Gln Val Gln Pro Ser
        55                  60                  65

GCC ACG CTG GAC GCC GCG CAG CCC CGG GTG ACC GGC GTC GTC CTC TTC        5336
Ala Thr Leu Asp Ala Ala Gln Pro Arg Val Thr Gly Val Val Leu Phe
    70                  75                  80

CGG CAG CTT GCG CCC CGC GCC AAG CTC GAC GCC TTC TTC GCC CTG GAG        5384
Arg Gln Leu Ala Pro Arg Ala Lys Leu Asp Ala Phe Phe Ala Leu Glu
85                  90                  95                  100

GGC TTC CCG ACC GAG CCG AAC AGC TCC AGC CGC GCC ATC CAC GTG CAC        5432
Gly Phe Pro Thr Glu Pro Asn Ser Ser Ser Arg Ala Ile His Val His
                105                 110                 115

CAG TTC GGG GAC CTG AGC CAG GGC TGC GAG TCC ACC GGG CCC CAC TAC        5480
Gln Phe Gly Asp Leu Ser Gln Gly Cys Glu Ser Thr Gly Pro His Tyr
            120                 125                 130

AAC CCG CTG GCC GTG CCG CAC CCG CAG CAC CCG GGC GAC TTC GGC AAC        5528
Asn Pro Leu Ala Val Pro His Pro Gln His Pro Gly Asp Phe Gly Asn
        135                 140                 145

TTC GCG GTC CGC GAC GGC AGC CTC TGG AGG TAC CGC GCC GGC CTG GCC        5576
Phe Ala Val Arg Asp Gly Ser Leu Trp Arg Tyr Arg Ala Gly Leu Ala
    150                 155                 160

GCC TCG CTC GCG GGC CCG CAC TCC ATC GTG GGC CGG GCC GTG GTC GTC        5624
Ala Ser Leu Ala Gly Pro His Ser Ile Val Gly Arg Ala Val Val Val
165                 170                 175                 180

CAC GCT GGC GAG GAC GAC CTG GGC CGC GGC GGC AAC CAG GCC AGC GTG        5672
His Ala Gly Glu Asp Asp Leu Gly Arg Gly Gly Asn Gln Ala Ser Val
                185                 190                 195

GAG AAC GGG AAC GCG GGC CGG CGG CTG GCC TGC TGC GTG GTG GGC GTG        5720
Glu Asn Gly Asn Ala Gly Arg Arg Leu Ala Cys Cys Val Val Gly Val
            200                 205                 210

TGC GGG CCC GGG CTC TGG GAG CGC CAG GCG CGG GAG CAC TCA GAG CGC        5768
Cys Gly Pro Gly Leu Trp Glu Arg Gln Ala Arg Glu His Ser Glu Arg
        215                 220                 225

AAG AAG CGG CGG CGC GAG AGC GAG TGC AAG GCC GCC TGAGCGCGGC             5814
Lys Lys Arg Arg Arg Glu Ser Glu Cys Lys Ala Ala
    230                 235                 240
```

CCCCACCCGG CGGCGGCCAG GGACCCCCGA GGCCCCCCTC TGCCTTTGAG CTTCTCCTCT 5874
GCTCCAACAG ACACCTTCCA CTCTGAGGTC TCACCTTCGC CTCTGCTGAA GTCTCCCCGC 5934
AGCCCTCTCC ACCCAGAGGT CTCCCTATAC CGAGACCCAC CATCCTTCCA TCCTGAGGAC 5994
CGCCCCAACC CTCGGAGCCC CCCACTCAGT AGGTCTGAAG GCCTCCATTT GTACCGAAAC 6054
ACCCCGCTCA CGCTGACAGC CTCCTAGGCT CCCTGAGGTA CCTTTCCACC CAGACCCTCC 6114
TTCCCCACCC CATAAGCCCT GAGACTCCCG CCTTTGACCT GACGATCTTC CCCCTTCCCG 6174
CCTTCAGGTT CCTCCTAGGC GCTCAGAGGC CGCTCTGGGG GGTTGCCTCG AGTCCCCCCA 6234
CCCCTCCCCA CCCACCACCG CTCCCGCGGC AAGCCAGCCC GTGCAACGGA AGCCAGGCCA 6294
ACTGCCCCGC GTCTTCAGCT GTTTCGCATC CACCGCCACC CCACTGAGAG CTGCTCCTTT 6354
GGGGGAATGT TTGGCAACCT TTGTGTTACA GATTAAAAAT TCAGCAATTC AGTACTGCGT 6414
CGAGGTCTTG GTTACTTTTT TGTTTGTTTG TTTTAGGCTT CTCTCCCAAG CTGAGCTTTT 6474
TTTTGTTTTG TTTTCGTTTT CCTTTTTTTT CTTTTTTTTG GGAGTGGCAA ACATGCTTCC 6534
CAAATCCCTA CAGGACTTCT CCTTATCCTC TGCCCCCACC TCCCTAACCC TGCTGGCAAC 6594
AACGTTCAGC CACTGCTTGT CTTGCCCTTC AGTGTGGCTC CAAGAGGAAG ATCACCAGAA 6654
TCACTCAGGG AAGTTAAAAA AAAAAATACA GCTTCCTGGG CTACATCCCA GAGCTGTGGA 6714
ATCCAAAGGG AGAAGAGAAA GTGAATTTGC GACAAGCGTC GGGATGATTC TGGCACTGGA 6774
CCCTCTGGCC TGAGAGGGGA AGAGGCCTTC CATCTCACCT GGGCTGGTAG CTTGTCACAT 6834
CTGCCTCCGA GTACAGCCTT AGGTCCATTT CCCAGATATC AGAGACAGTG CCAGGGAAGC 6894
CAGGTGACTG CATCTTGCCT AGGCACAGAA GAGTAGGGTT GGAATGTGAC GTTGTTAGCA 6954
TTTGGCAGGA CCAAAACCAG AGGCAAACGG AGGCAGTGGG ATGGAAAGGC AGTTGATTTT 7014
GATGAAGGCT TGTTGGGAGT TCAGCTTTCT TTTGAAACTT ATAATCTATA CCCAGGCTAG 7074
AACAGTCTTG TGTATACACC TTCATTCATG GAATAAACGT ACTTGCAATA ACTTTTTAGC 7134
CTCCCAGGGT AGCCTCACTT CCTAGCTGTG ACTTTTCCAC CCTGGTTACT GGGAGGCAGC 7194
TTCCATTTCT CCCAGACTAG CTAGGCAGTG CGTCCAACTG AACCGCAGCC AGAAACCTGT 7254
CTCCAGGGGT TATTTTTACC TCTAACTAGG ACTAACTTAT TTTAAAATCT TTCCTTGAGC 7314
CCAAGTGACA ACTGAAGAGA AAGGCTATTG CCTGGTGATT TTGCTCCACC AGTTGGTTCT 7374
CACTGGTTTG AATACTAACT TGAACTGTAC TCATCGACAC TGAAAGGGGA TGAGCAAACA 7434
GTGTCTCTAA ATCTCCTGAT CCTGATCTCA AATATCCCCC TAATTACAAG TTGCAACAAG 7494

GCAGCTATTA CACGGGGACA CAGGATGGAG AGGATGGGTG CCAAACACCC ATCGTCTACT 7554

CTGCTGCCTC GGTTATGGTG AATTCAGGAC CATCAAGGGA GGTGTGGACC TTTTTTTTCA 7614

GAAGGAGGCT GACACTTCTT GTCAATTGCA TTGTGTTCTT AGTTTTGCTC TTCACAACCC 7674

TTGACCCCGT AGATGGGGGC TGAAGAGGCA CCCTGGCCGA CTCACTCTAT TTCTGTTTTG 7734

GGAATGGGAT GGATAAACTA TCCCATGGCC TCCAGAGCCA AAAAACCAAA ACGAAACAAA 7794

ACAAAAAACC CCAAAACAAA AAAGCAAAAA GCAAACAAGA AAAAAAAAAA AAGAGGAAAT 7854

AATAGGCAGA CAATTTACAG TTCATTGTAA GGGCAAAGAT ATGCATATAG CATGATGGTT 7914

AACAGGTCAG GCTCAGGTAG AAAGGCCCAT TTGAACCCCA GCTCTGCCAC ACTCAGAAAC 7974

TGTGTGACCC GAACAAGTCA CTTAACCTCT CTGAGCATAG GTAAAATAAG ATCATCATAC 8034

CAGATTGTTT TGAAGATTAA ATCAAGTGTT ATTCACGAGA GGTGCACAGC ATAGCATGCA 8094

CAACAAATAA GGACCTGGTA AGTATCTAAT TAATAACAAT GGCTAAGATC CAAAAAACAG 8154

CTACCTACTA ATAAATAGAT GGGGCTGCCT TGTAAGGCAG TGAGCATCAT GCAACCAGGA 8214

TTCAAATGAA GGACAGTTGC TACCTCTGAG GTTCCCGAGA AGGATTTCTC GATCCATTGA 8274

GAGACTGAAT GACATGAACT CTGCGATCCC ATCTCTTGTG GGGAGGGAAC CTAGAATGAA 8334

GGGAAGATTG TGGGCCATAA AGGCAGACAT CTGGTTCCTG GGCACAGAAC CATATGTGTG 8394

CCACCAAAGC CACCCACCGG ACCCCACTTG GCCCCTGGAG TCTATTTTTA CTCCTCTCAT 8454

CTTACAAGAT CTATTTTGTT AATCTCCTTA TATTTGCTGT TTTGACTTCC CAGCCAGCTT 8514

GCTAATCAGT TTGCCTATTT GACTCACAGG GTTTGCATTT GTCACGGGGA CTGAAACACA 8574

CGCTTGTTTT GATTTCTTTT TGTAAATTAG AAGCGTTGAT GTAATGACTC TACCTAGACA 8634

CAGCTGGTAA AGTGAGAATA ATGCTCAAGT TTGCACAGTT TAAACACAAT GTAGACAATA 8694

ATTAGAAATG CTATCTTTAG ATGTTTAGGA TAAGCTTTTC TCAGAATTGC ACTGATTTTT 8754

TTTTTCTGAG TGGGGCTTTT TAGTGCATAT ATACAGAAAT ACTAAAAACG TAAGAAAATA 8814

GAGCAAATCA GTGAGTGCTT TGGTCAACTT GAAAGACTGC AGGAAATAAA CCAACTGATT 8874

TTAGATCTGC CTTTTTTTGA CTGAATGCAT AAAATCTTTA CATTCTCCAT ATTTTTCATG 8934

ACTACCATAT GATCAAATAG TTTTAGGTGA CAGATTGCAA CTGATAAGTT GCTGCAATAT 8994

GGCAGAAGTC ATGCTCAGCC TCCGCTTGCC CGGTGGTGAG GGTGGAATAT GAAGCAAACA 9054

ATAAAGATAA TTCATCATCT CTATCAGGAA AATTGCCACA TGTTTATTTC AGGTAACAAA 9114

AAAGATATAG TTATGATATA CAATGACCAT AGAATCCAAT AAAGCAACTT CTGCAAATGA 9174

ATAGAAGGTA CTTTTTCTTT AAATGAAACT ACAAAATAGC AGCTGGTTTT AAAAACAAAG 9234
CCAATTGTTT TAGATTTAAT AGGCTACCAC TGGCCTCTGC TAAGATCCCC AAATATATTC 9294
CTGAGCTCAC ATAGATTCCA GAAAGTCAAA CTTTTCAATA TTATGCAAAC TTTCCCTATG 9354
CATCCAAAAA ATTCTCATTT AGTAAAGAGG TGATATGAAA TGTAAGGCAG CATGTCCATA 9414
TCTATCATTT TAAATTGCCT TCATGCTGTA TCAACTGGTT TTGTTTTGGG AAGCAACCAT 9474
AATATTGAGA GACGGGTCTT TCCTATTTTT TCTGCTACTC ATTTCTAACT AGATTCACTA 9534
CGGAGCTCCC AATTGCATCT CTCTGATCTA CAAATTTTTC TCTCTTCAGG AAGACACCTG 9594
GAAAGAAGGG ACTACATTAA AGGAGTGTGT TGGGGGCAAT GCTTTGGCCT TTTGACATCC 9654
TATCTAGTCT GAAGGGACCC TCACTATTGC TAAGGAGGAG GAGTGTTTTA AATGGAGGCT 9714
TCAGAATGAA AGCAGAGGAA GAAGGTACTC TCTTTTTCAA AAAGAAGGAG GGTACAGGCC 9774
GGGCGCAGCT GTCACGCCTG CAATCCCAGC ACTTTGGGAG GCCGAGGAAG GCAGATCACG 9834
AGGTTGGGAG TTTGAGCCAG CCTGGTCAAC ATAGTGAAAC CCCGTCTCTA CTAAAAATAC 9894
AAAAATTAGC CAGCATGGTG GTGCATGCCT GTAGTCCCAG TTACTCGGGA GGCTGAGGCA 9954
GGAGAATCGC TTGAACTCGG GAAGTGGAGG TTGCAGTGAG CCGAGATCAT GCCACTGCAC 10014
TCCACCCTGG GTGACAGAGT GAGACTCTCA AAAAAAAAAA AAAAAAAAAA AAGAAGTAGG 10074
GTACC 10079

(2) INFORMATION FOR SEQ ID NO:21:

(i) SEQUENCE CHARACTERISTICS:

(A) LENGTH: 240 amino acids
(B) TYPE: amino acid
(D) TOPOLOGY: linear

(ii) MOLECULE TYPE: protein

(xi) SEQUENCE DESCRIPTION: SEQ ID NO:21:

Met Leu Ala Leu Leu Cys Ser Cys Leu Leu Leu Ala Ala Gly Ala Ser
1 5 10 15

Asp Ala Trp Thr Gly Glu Asp Ser Ala Glu Pro Asn Ser Asp Ser Ala
20 25 30

Glu Trp Ile Arg Asp Met Tyr Ala Lys Val Thr Glu Ile Trp Gln Glu
35 40 45

Val Met Gln Arg Arg Asp Asp Asp Gly Thr Leu His Ala Ala Cys Gln
50 55 60

```
Val Gln Pro Ser Ala Thr Leu Asp Ala Ala Gln Pro Arg Val Thr Gly
 65                 70                  75                  80

Val Val Leu Phe Arg Gln Leu Ala Pro Arg Ala Lys Leu Asp Ala Phe
                85                  90                  95

Phe Ala Leu Glu Gly Phe Pro Thr Glu Pro Asn Ser Ser Ser Arg Ala
            100                 105                 110

Ile His Val His Gln Phe Gly Asp Leu Ser Gln Gly Cys Glu Ser Thr
        115                 120                 125

Gly Pro His Tyr Asn Pro Leu Ala Val Pro His Pro Gln His Pro Gly
    130                 135                 140

Asp Phe Gly Asn Phe Ala Val Arg Asp Gly Ser Leu Trp Arg Tyr Arg
145                 150                 155                 160

Ala Gly Leu Ala Ala Ser Leu Ala Gly Pro His Ser Ile Val Gly Arg
                165                 170                 175

Ala Val Val Val His Ala Gly Glu Asp Asp Leu Gly Arg Gly Gly Asn
            180                 185                 190

Gln Ala Ser Val Glu Asn Gly Asn Ala Gly Arg Arg Leu Ala Cys Cys
        195                 200                 205

Val Val Gly Val Cys Gly Pro Gly Leu Trp Glu Arg Gln Ala Arg Glu
    210                 215                 220

His Ser Glu Arg Lys Lys Arg Arg Arg Glu Ser Glu Cys Lys Ala Ala
225                 230                 235                 240
```

(2) INFORMATION FOR SEQ ID NO:22:

(i) SEQUENCE CHARACTERISTICS:

(A) LENGTH: 18 amino acids
(B) TYPE: amino acid
(D) TOPOLOGY: linear

(ii) MOLECULE TYPE: protein

(xi) SEQUENCE DESCRIPTION: SEQ ID NO:22:

```
Arg Glu His Ser Glu Arg Lys Lys Gly Arg Arg Ala Ser Glu Cys Ala
1               5                   10                  15

Ala Ala
```